# EUROPEAN PATENT APPLICATION

(11) **EP 4 736 872 A1**
(43) Date of publication of application: **06.05.2026**
(21) Application number: 24830897.5
(22) Date of filing: 27.06.2024
(51) Int. Cl.: A61K 39/395, C07K 16/32

(54) **TREATMENT METHOD AND USE OF PHARMACEUTICAL COMBINATION CONTAINING CONJUGATE**

(30) Priority: 30.06.2023 WO PCT/CN2023/105235; 24.06.2024 CN 202410821898
(71) Applicant: Merck Sharp & Dohme LLC, Rahway, New Jersey 07065 (US); Sichuan Kelun-Biotech Biopharmaceutical Co., Ltd., Chengdu, Sichuan 611138 (CN)
(72) Inventor: GE, Junyou, Chengdu, Sichuan 611138 (CN); JIN, Xiaoping, Chengdu, Sichuan 611138 (CN); DIAO, Yina, Chengdu, Sichuan 611138 (CN); YANG, Yalan, Chengdu, Sichuan 611138 (CN); FU, Yujie, Chengdu, Sichuan 611138 (CN); JING, Dian, Chengdu, Sichuan 611138 (CN); ZHONG, Hongbo, Chengdu, Sichuan 611138 (CN); AKALA, Omobolaji, Rahway New Jersey 07065 (US); CHARTASH, Elliot, Rahway New Jersey 07065 (US); PIENTANZA, Catherine, Rahway New Jersey 07065 (US); ZHAO, Bin, Rahway New Jersey 07065 (US)
(74) Representative: Ipsilon
(86) International application number: PCT/CN2024/102036
(87) International publication number: WO 2025/002264

(57) **Abstract**

The present disclosure relates to a method of treatment and use of a pharmaceutical combination comprising a conjugate. Specifically, the present disclosure relates to a pharmaceutical composition, kit and therapeutic combination, and use thereof for treating a cell proliferative disorder (e.g., cancer), and a method for treating a cell proliferative disorder (e.g., cancer).

## Description

The present application is based on and claims priority to the PCT application with the application number PCT/CN2023/105235 (filed on June 30, 2023) and the Chinese patent application with the application number CN 202410821898.2 (filed on June 24, 2024). The content of the aforementioned PCT application is hereby incorporated by reference in its entirety whole into the present application.

### TECHNICAL FIELD

The present disclosure relates to a treatment method and use of a pharmaceutical combination containing a conjugate. Specifically, the present disclosure relates to a pharmaceutical composition, kit, and pharmaceutical combination containing an immunoconjugate. The present disclosure also relates to a method for treating a cell proliferative disorder, particularly non-small cell lung cancer (NSCLC) in patients.

### BACKGROUND OF THE DISCLOSURE

PD-1 is recognized as an important player in immune regulation and the maintenance of peripheral tolerance. PD-1 is moderately expressed on naive T, B and NKT cells and up-regulated by T/B cell receptor signaling on lymphocytes, monocytes and myeloid cells.

Two known ligands for PD-1, PD-L1 (B7-H1) and PD-L2 (B7-DC), are expressed in human cancers arising in various tissues. In large sample sets of e.g., ovarian, renal, colorectal, pancreatic, liver cancers and melanoma, it was shown that PD-L1 expression correlated with poor prognosis and reduced overall survival irrespective of subsequent treatment.

Similarly, PD-1 expression on tumor infiltrating lymphocytes was found to mark dysfunctional T cells in breast cancer and melanoma, and to correlate with poor prognosis in renal cancer. Thus, it has been proposed that PD-L1 expressing tumor cells interact with PD-1 expressing T cells to attenuate T cell activation and evasion of immune surveillance, thereby contributing to an impaired immune response against the tumor.

Immune checkpoint therapies targeting the PD-1 axis have resulted in groundbreaking improvements in clinical response in multiple human cancers. Immune therapies targeting the PD-1 axis include monoclonal antibodies directed to the PD-1 receptor (KEYTRUDA^{™} (pembrolizumab), Merck and Co., Inc., Kenilworth, NJ, USA and OPDIVO^{™} (nivolumab), Bristol-Myers Squibb Company, Princeton, NJ, USA) and also those that bind to the PD-L1 ligand (MPDL3280A; TECENTRIQ^{™} (atezolizumab), Genentech, San Francisco, CA, USA). Both therapeutic approaches have demonstrated anti-tumor effects in numerous cancer types.

It has been proposed that the efficacy of such antibodies might be enhanced if administered in combination with other approved or experimental cancer therapies, e.g., radiation, surgery, chemotherapeutic agents, targeted therapies, agents that inhibit other signaling pathways that are disregulated in tumors, and other immune enhancing agents.

Trophoblast cell surface antigen 2 (Trop-2) is a transmembrane glycoprotein involved in calcium signal transduction, and is expressed in multiple tumor types. Trop-2 is expressed in normal trophoblasts and allows for trophoblast cell growth, migration, and proliferation. Trop-2 has been implicated in several cell signaling pathways, including intracellular calcium transduction, MAPK signaling pathway, RAF, NF-κB, and Cyclin D/E among others.

It has been shown that Trop-2 is upregulated in cancer cells when compared to normal cell counterparts. This increased expression has been seen in many different tumor types including breast cancer, colon cancer, non-small cell lung cancer (NSCLC), esophageal squamous cell cancer, thyroid cancer, and hepatobiliary cancers, raising the possibility of Trop-2 as a tumor agnostic biomarker. The reason for Trop-2 upregulation in cancer cells is unclear; however it is postulated that Trop-2 has critical regulatory effects on cellular proliferation and invasion, meaning that overexpression would lead to selective tumor progression. In fact, preclinical data suggests that Trop-2 overexpression stimulates tumor growth while Trop-2 knock-down inhibits tumor growth.

In breast cancer specifically, elevated Trop-2 expression has been associated with worse survival. Trop-2 gene expression has been detected in all breast cancer subtypes with higher levels noted in HR+/HER2-, and triple negative breast cancer (TNBC) compared to HER2+ disease. Genomic analyses of TNBC also identified Trop-2 as an attractive candidate for targeted therapy, and led to further investigation of Trop-2 as a new therapeutic target.

The success of targeting Trop-2 via antibody-drug conjugates (ADCs) in metastatic breast cancer (MBC), and urothelial cancer and ongoing trials in NSCLC have established Trop-2 targeting as a valid and fruitful strategy, and several Trop-2-targeted therapeutics have recently been developed for clinical use, such as anti-Trop-2 antibodies and Trop-2-targeted ADCs. Subsequently, multiple early-phase clinical trials have demonstrated good safety profiles, and clinical benefit associated with Trop-2-based ADCs across multiple tumor types. This includes clinical benefit, and tolerability in tumor types with limited treatment options, such as triple-negative breast cancer, platinum-resistant urothelial cancer, and small-cell lung cancer. An example of an immunoconjugate that is currently in early phase clinical trials is Immunoconjugate A: 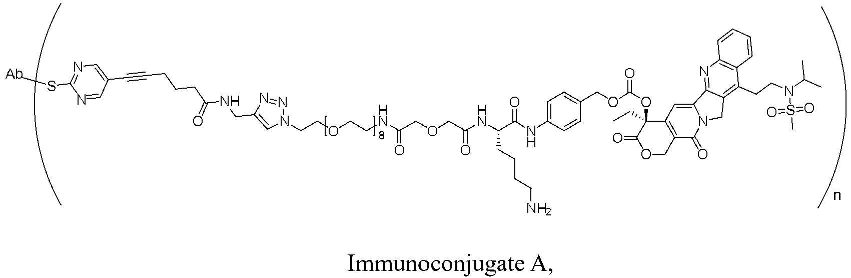 wherein Ab is an anti-Trop-2 antibody (sacituzumab), and which is described in US Patent Publication No. 20200347075.

Trop-2 may play a role in tumor progression given the involvement in several molecular pathways traditionally associated with cancer development. High Trop-2 expression correlates with poor prognosis in pancreatic, hilar cholangiocarcinoma, cervical cancer, gastric cancer, and others. In a meta-analysis including 2,569 patients, increased Trop-2 expression was associated with poor overall and disease-free survival outcomes across several solid tumors. Given Trop-2's expression pattern and associated poor prognostic outcomes, Trop-2 is a rational prognostic marker and therapeutic target.

Given the promising results shown by both anti-human PD-1 antibodies, and immunoconjugates as monotherapy for the treatment of cancer, the combination of these therapeutic agents with their differing mechanisms of action, and non-overlapping toxicities, has the potential to provide more significant and prolonged responses in numerous cancer types.

### SUMMARY OF THE PRESENT DISCLOSURE

In a first aspect, the present disclosure provides a method for treating a cell proliferative disorder such as cancer in a patient, comprising administering to the patient:
(a) an anti-human PD-1 antibody or antigen-binding fragment thereof, a platinum-containing chemotherapeutic agent, and/or an EGFR-tyrosine kinase inhibitor (EGFR-TKI);
(b) an immunoconjugate represented by Formula (I): 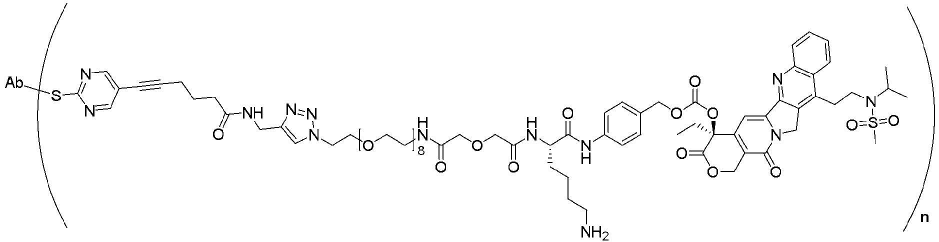 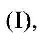 wherein,
   Ab represents an antibody that binds Trop-2; and
   n is an integer selected from 1 to 10;
   wherein, the amounts of (a) and (b) are effective for treating the cell proliferative disorder.

In some embodiments, the present disclosure provides a method of treating a cellular proliferative disorder, e.g., cancer, in a patient, comprising admadministering to the patient:
(a) an anti-human PD-1 antibody or antigen binding fragment thereof; and
(b) an Immunoconjugate of Formula (I): 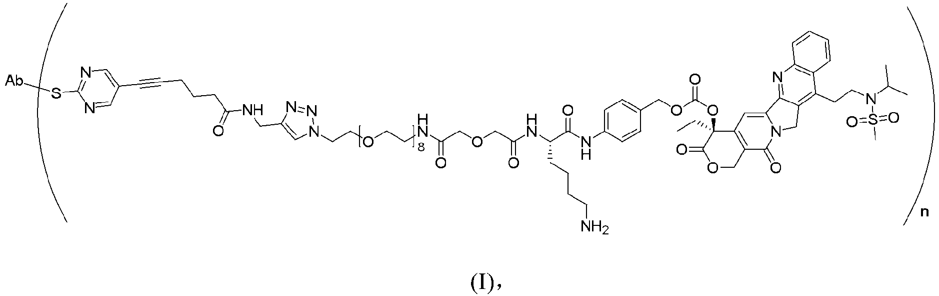 wherein:
   Ab is an antibody that binds to Trop-2; and
   n is an integer from 1 to 10,
   wherein the amounts of (a) and (b) administered are together effective to treat cancer.

In one embodiment of this method (embodiment no. 1), the cell proliferative disorder is cancer, wherein the cancer is colorectal cancer (CRC), prostate cancer, pancreatic cancer, melanoma, non-small cell lung cancer (NSCLC), head and neck cancer, urothelial cancer, breast cancer, gastrointestinal cancer, multiple myeloma, hepatocellular cancer, non-Hodgkin's lymphoma, renal cancer, Hodgkin's lymphoma, mesothelioma, ovarian cancer, small cell lung cancer, esophageal cancer, anal cancer, biliary tract cancer, colorectal cancer, cervical cancer, thyroid cancer, or salivary cancer. Also, in some embodiments above method, the cancer expresses an elevated level of PD-L1, such as cancers having tumors with a tumor proportion score (TPS) of ≥1%, 1% to 49% or ≥50%, or combined positive score (CPS) ≥1 or ≥10. For example, the level of PD-L1 TPS or CPS is determined by an FDA-approved test.

In embodiment no. 2 of this method, the cancer is non-small cell lung cancer. In a specific embodiment, the non-small cell lung cancer is advanced or metastatic non-small cell lung cancer. In another specific embodiment, the patient is in need of first line therapy, and the non-small cell lung cancer is without EGFR mutation, and ALK fusion genes. In another specific embodiment, the non-small cell lung cancer is non-small cell lung cancer with EGFR actionable mutation, and the patient has failed prior EGFR-TKI treatment. In another specific embodiment, the non-small cell lung cancer has a 19 del mutation and/or has a 21 L858R mutation. In another specific embodiment, the non-small cell lung cancer is non-small cell lung cancer with EGFR actionable mutation, and the patient has failed prior EGFR-TKI treatment, and failed prior treatment with PD-1/PD-L1 antibody and chemotherapy.

In embodiment no. 3 of this method, the cancer is cervical cancer. In specific embodiments, the cervical cancer is recurrent or metastatic cervical cancer.

In embodiment no. 4 of this method, the cancer is ovarian cancer. In specific embodiments, the ovarian cancer is recurrent or metastatic ovarian cancer. In another specific embodiment, the ovarian cancer is platinum-resistant ovarian cancer.

In embodiment no. 5 of this method, the cancer is urothelial cancer. In specific embodiments, the urothelial cancer is locally advanced or metastatic urothelial cancer.

In embodiment no. 6 of this method, the cancer is prostate cancer. In specific embodiments, the prostate cancer is locally advanced or metastatic urothelial cancer. In another specific embodiment, the prostate cancer is metastatic castration-resistant prostate cancer (mCRPC).

In embodiment no. 7 of this method, the Immunoconjugate of Formula (I) is Immunoconjugate A.

In embodiment no. 8 of this method, the patient is administered 200 mg of the anti-human PD-1 antibody or antigen binding fragment thereof, and 5 mg/kg of Immunoconjugate A, wherein the anti-human PD-1 antibody or antigen binding fragment thereof is administered once every three weeks and Immunoconjugate A is administered once every 3 weeks. For instance, the human patient is administered 200 mg of the anti-human PD-1 antibody or antigen binding fragment thereof on day 1 of a 3-week cycle, and 5 mg/kg of Immunoconjugate A on day 1 of a 3-week cycle.

In embodiment no. 9 of this method, the patient is administered 400 mg of the anti-human PD-1 antibody or antigen binding fragment thereof, and 5 mg/kg of Immunoconjugate A, wherein the anti-human PD-1 antibody or antigen binding fragment thereof is administered once every six weeks and Immunoconjugate A is administered once every 2 weeks. For instance, the human patient is administered 400 mg of the anti-human PD-1 antibody or antigen binding fragment thereof on day 1 of a 6-week cycle, and 5 mg/kg of Immunoconjugate A on days 1, 15, and 29, of a 6-week cycle.

In embodiment no. 10 of this method, the patient is administered 200 mg of the anti-human PD-1 antibody or antigen binding fragment thereof, 5 mg/kg of Immunoconjugate A, and a platinum-containing chemotherapy agent, wherein the anti-human PD-1 antibody or antigen binding fragment thereof is administered once every three weeks, Immunoconjugate A is administered once every 3 weeks, and the platinum-containing chemotherapeutic agent is administered once every 3 weeks. For instance, the human patient is administered 200 mg of the anti-human PD-1 antibody or antigen binding fragment thereof on day 1 of a 3-week cycle, 5 mg/kg of Immunoconjugate A on day 1 of a 3-week cycle, and the platinum-containing chemotherapeutic agent on day 1 of a 3-week cycle.

In embodiment no. 11 of this method, the patient is administered 400 mg of the anti-human PD-1 antibody or antigen binding fragment thereof, 5 mg/kg of Immunoconjugate A, and a platinum containing chemotherapeutic agent, wherein the anti-human PD-1 antibody or antigen binding fragment thereof is administered once every six weeks, Immunoconjugate A is administered once every 2 weeks, and the platinum-containing chemotherapeutic agent is administered once every 3 weeks. For instance, the human patient is administered 400 mg of the anti-human PD-1 antibody or antigen binding fragment thereof on day 1 of a 6-week cycle, 5 mg/kg of Immunoconjugate A on days 1, 15, and 29, of a 6-week cycle, and the platinum-containing chemotherapeutic agent on days 1 and 22 of a 6-week cycle.

In embodiment no. 12 of this method, the patient is administered 200 mg of the anti-human PD-1 antibody or antigen binding fragment thereof, 5 mg/kg of Immunoconjugate A, and either carboplatin or cisplatin, wherein the anti-human PD-1 antibody or antigen binding fragment thereof is administered once every three weeks, Immunoconjugate A is administered once every 3 weeks, and carboplatin or cisplatin is administered once every 3 weeks. For instance, the human patient is administered 200 mg of the anti-human PD-1 antibody or antigen binding fragment thereof on day 1 of a 3-week cycle, and 5 mg/kg of Immunoconjugate A on day 1 of a 3-week cycle, and carboplatin or cisplatin on day 1 of a 3-week cycle.

In a second aspect, the present disclosure provides a kit comprising:
(a) an Immunoconjugate of Formula (I); and
(b) an anti-human PD-1 antibody or antigen binding fragment thereof, a platinum-containing chemotherapeutic agent, and/or an EGFR-tyrosine kinase inhibitor (EGFR-TKI).

In some embodiments, the present disclosure provides a kit comprising:
(a) an Immunoconjugate of Formula (I); and
(b) an anti-human PD-1 antibody or antigen binding fragment thereof.

In embodiment no. 1 of this kit, the kit further comprises instructions for administering the Immunoconjugate of Formula (I), and the anti-human PD-1 antibody or antigen binding fragment thereof to a human patient.

In a third aspect, the present disclosure provides a combination therapy for treating a cell proliferative disorder such as cancer in a human patient, comprising:
(a) an immunoconjugate represented by Formula (I); and
(b) an anti-human PD-1 antibody or antigen-binding fragment thereof for treating cell proliferative disorder, a platinum-containing chemotherapeutic agent, and/or an EGFR-tyrosine kinase inhibitor (EGFR-TKI).

In some embodiments, the present disclosure provides a combination therapy for treating a cell proliferative disorder such as cancer in a human patient, comprising:
(a) an immunoconjugate represented by Formula (I); and
(b) an anti-human PD-1 antibody or antigen-binding fragment thereof.

In a fourth aspect, the present disclosure provides a pharmaceutical combination for use in treating a cell proliferative disorder such as cancer in a human patient, the pharmaceutical combination comprising:
(a) an immunoconjugate represetned by Formula (I); and
(b) an anti-human PD-1 antibody or antigen-binding fragment thereof, a platinum-containing chemotherapeutic agent, and/or an EGFR-tyrosine kinase inhibitor (EGFR-TKI).

In some embodiments, the present disclosure provides a pharmaceutical combination for use in treating a cell proliferative disorder such as cancer in a human patient, the pharmaceutical combination comprising:
(a) an immunoconjugate represetned by Formula (I); and
(b) an anti-human PD-1 antibody or an antigen-binding fragment thereof.

In a fifth aspect, the present disclosure provides a use of a pharmaceutical combination in the manufacture of a medicament for treating a cell proliferative disorder such as cancer in a human patient, wherein the pharmaceutical combination comprises:
(a) an immunoconjugate represented by Formula (I); and
(b) an anti-human PD-1 antibody or antigen-binding fragment thereof, a platinum-containing chemotherapeutic agent, and/or an EGFR-tyrosine kinase inhibitor (EGFR-TKI).

In some embodiments, the present disclosure provides a use of a pharmaceutical combination in the manufacture of a medicament for treating a cell proliferative disorder such as cancer in a human patient, wherein the pharmaceutical combination comprises:
(a) an immunoconjugate represented by Formula (I); and
(b) an anti-human PD-1 antibody or antigen-binding fragment thereof.

In certain embodiments of the the first, second, third, fourth or fifth aspect, the anti-human PD-1 antibody or antigen binding fragment thereof comprises three light chain CDRs of SEQ ID NO: 1, SEQ ID NO:2 and SEQ ID NO:3 and three heavy chain CDRs of SEQ ID NO:6, SEQ ID NO:7 and SEQ ID NO:8.

In certain embodiments of the the first, second, third, fourth or fifth aspect, the anti-human PD-1 antibody or antigen binding fragment thereof comprises a V_{L} region which comprises the amino acid sequence set forth in SEQ ID NO:4, and a V_{H} region which comprises the amino acid sequence set forth in SEQ ID NO:9.

In certain embodiments of the the first, second, third, fourth or fifth aspect, the anti-human PD-1 antibody or antigen binding fragment thereof comprises a light chain comprising or consisting of a sequence of amino acids as set forth in SEQ ID NO:5 and a heavy chain comprising or consisting of a sequence of amino acids as set forth in SEQ ID NO: 10.

In certain embodiments of the the first, second, third, fourth or fifth aspect, the anti-human PD-1 antibody or antigen binding fragment thereof is pembrolizumab.

In certain embodiments of the the first, second, third, fourth or fifth aspect, the anti-human PD-1 antibody or antigen binding fragment thereof is nivolumab.

In certain embodiments of the the first, second, third, fourth or fifth aspect, the anti-human PD-1 antibody or antigen binding fragment thereof is cemiplimab.

In certain embodiments of the the first, second, third, fourth or fifth aspect, the anti-human PD-1 antibody or antigen binding fragment thereof is dostarlimab.

### DETAILED DESCRIPTION OF THE PRESENT DISCLOSURE

### DEFINITIONS AND ABBREVIATIONS

Listed below are definitions of various terms used herein. These definitions apply to the terms as they are used throughout this specification and claims, unless otherwise limited in specific instances, either individually or as part of a larger group.

Unless defined otherwise, all technical and scientific terms used herein have the same meaning as commonly understood by one of ordinary skill in the art. Generally, the nomenclature used herein and the laboratory procedures in cell culture, molecular genetics, organic chemistry, and peptide chemistry are those well-known and commonly employed in the art.

As used herein, the articles "a" and "an" refer to one or to more than one (i.e., to at least one) of the grammatical object of the article. By way of example, "an element" means one element or more than one element. Furthermore, use of the term "including" as well as other forms, such as "include," "includes," and "included," is not limiting.

As used herein, the term "about" in quantitative terms refers to plus or minus 10% of the value it modifies (rounded up to the nearest whole number if the value is not sub-dividable, such as a number of molecules or nucleotides).

An "antibody-drug conjugate," or "ADC," or "immunoconjugate," refers to an antibody molecule or an antigen-binding fragment thereof that is covalently or non-covalently bonded, with or without a linker, to one or more biologically active molecule(s). The term "antibody" is used herein in the broadest sense and includes polyclonal and monoclonal antibodies, such as intact antibodies and functional (antigen-binding) fragments thereof. The term encompasses genetically engineered and/or otherwise modified forms of immunoglobulins, such as intrabodies, peptibodies, chimeric antibodies, fully human antibodies, humanized antibodies, and heteroconjugate antibodies, multi-specific (e.g., bispecific) antibodies, diabodies, triabodies, and tetrabodies, tandem di-scFv, and tandem tri-scFv.

As used herein, the terms "at least one" item or "one or more" item each include a single item selected from the list as well as mixtures of two or more items selected from the list.

The terms "administration" or "administer" refers to the act of injecting or otherwise physically delivering a substance as it exists outside the body (*e.g.,* an anti-PD-1 antibody) into a patient, such as by oral, mucosal, intradermal, intravenous, subcutaneous, intramuscular delivery, and/or any other methods of physical delivery described herein or known in the art.

As used herein, unless otherwise indicated, "antibody fragment" or "antigen binding fragment" refers to a fragment of an antibody that retains the ability to bind specifically to the antigen, e.g., fragments that retain one or more CDR regions and the ability to bind specifically to the antigen. An antibody that "specifically binds to" PD-1 is an antibody that exhibits preferential binding to PD-1 (as appropriate) as compared to other proteins, but this specificity does not require absolute binding specificity. An antibody is considered "specific" for its intended target if its binding is determinative of the presence of the target protein in a sample, *e.g*., without producing undesired results such as false positives. Antibodies, or binding fragments thereof, will bind to the target protein with an affinity that is at least two-fold greater, preferably at least ten times greater, more preferably at least 20-times greater, and most preferably at least 100-times greater than the affinity with non-target proteins.

Antigen binding portions include, for example, Fab, Fab', F(ab')2, Fd, Fv, fragments including CDRs, and single chain variable fragment antibodies (scFv), and polypeptides that contain at least a portion of an immunoglobulin that is sufficient to confer specific antigen binding to the antigen (*e.g.,* PD-1). Depending on the antibody amino acid sequence of the constant region of its heavy chains, immunoglobulins can be assigned to different classes. There are five major classes of immunoglobulins: IgA, IgD, IgE, IgG, and IgM, and several of these may be further divided into subclasses (isotypes), *e.g.,* IgG1, IgG2, IgG3, IgG4, IgA1, and IgA2. The heavy-chain constant regions that correspond to the different classes of immunoglobulins are called alpha, delta, epsilon, gamma, and mu, respectively. The subunit structures and three-dimensional configurations of different classes of immunoglobulins are well known. An antigen-binding fragment of a full-length antibody may be used in making an immunoconjugate of the present disclosure. Examples of antibody fragments include, but are not limited to, Fv, Fab, Fab', Fab'-SH, F(ab')₂; recombinant IgG (rlgG) fragments; diabodies; linear antibodies; single-chain antibody molecules (e.g., scFv or sFv); single domain antibodies (e.g., sdAb, sdFv, nanobodies); and multi-specific antibodies formed from antibody fragments. In certain embodiments, the fragments are single-chain antibody fragments comprising a variable heavy chain region and/or a variable light chain region, such as scFvs.

An "antigen" is a structure to which an antibody can selectively bind. A target antigen may be a polypeptide, carbohydrate, nucleic acid, lipid, hapten, or other naturally occurring or synthetic compound. In some embodiments, the target antigen is a polypeptide. In certain embodiments, an antigen is associated with a cell, for example, is present on or in a cell, for example, a cancer cell.

"Advanced solid tumor malignancy" and "advanced solid tumor" are used interchangeably to refer to a tumor for which curative resection is not possible. Advanced solid tumors include, but are not limited to, metastatic tumors in bone, brain, breast, liver, lungs, lymph node, pancreas, prostate, and soft tissue (sarcoma).

As used herein, the term "antibody" refers to any form of immunoglobulin molecule that exhibits the desired biological or binding activity. Thus, it is used in the broadest sense and specifically covers, but is not limited to, monoclonal antibodies (including full length monoclonal antibodies), polyclonal antibodies, multispecific antibodies (*e.g.,* bispecific antibodies), humanized, fully human antibodies, and chimeric antibodies. "Parental antibodies" are antibodies obtained by exposure of an immune system to an antigen prior to modification of the antibodies for an intended use, such as humanization of an antibody for use as a human therapeutic. As used herein, the term "antibody" encompasses not only intact polyclonal or monoclonal antibodies, but also, unless otherwise specified, any antigen binding portion thereof that competes with the intact antibody for specific binding, fusion proteins comprising an antigen binding portion, and any other modified configuration of the immunoglobulin molecule that comprises an antigen recognition site.

In general, the basic antibody structural unit comprises a tetramer. Each tetramer includes two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The variable regions of each light/heavy chain pair form the antibody binding site. Thus, in general, an intact antibody has two binding sites. The carboxy-terminal portion of the heavy chain may define a constant region primarily responsible for effector function. Typically, human light chains are classified as kappa and lambda light chains. Furthermore, human heavy chains are typically classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989).

"Biotherapeutic agent" means a biological molecule, such as an antibody or fusion protein, that blocks ligand/receptor signaling in any biological pathway that supports tumor maintenance and/or growth or suppresses the anti-tumor immune response.

The term "carrier," as used herein, encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body of a patient.

As used herein, the term "comprising" may include the embodiments "consisting of" and "consisting essentially of." The terms "comprise(s)," "include(s)," "having," "has," "may," "contain(s)," and variants thereof, as used herein, are intended to be open-ended transitional phrases, terms, or words that require the presence of the named ingredients/steps and permit the presence of other ingredients/steps. However, such description should be construed as also describing compositions or processes as "consisting of" and "consisting essentially of" the enumerated components, which allows the presence of only the named components or compounds, along with any acceptable carriers or fluids, and excludes other components or compounds.

A "CDR" refers to one of three hypervariable regions (H1, H2, or H3) within the non-framework region of the antibody V_{H} β-sheet framework, or one of three hypervariable regions (L1, L2, or L3) within the non-framework region of the antibody V_{L} β-sheet framework. Accordingly, CDRs are variable region sequences interspersed within the framework region sequences. CDR regions are well known to those skilled in the art and have been defined by, for example, Kabat as the regions of most hypervariability within the antibody variable domains. CDR region sequences also have been defined structurally by Chothia as those residues that are not part of the conserved β -sheet framework, and thus are able to adapt to different conformations. Both terminologies are well recognized in the art. CDR region sequences have also been defined by AbM, Contact, and IMGT. The positions of CDRs within a canonical antibody variable region have been determined by comparison of numerous structures (Al-Lazikani et al., 1997, J. Mol. Biol. 273:927-48; Morea et al., 2000, Methods 20:267-79). Because the number of residues within a hypervariable region varies in different antibodies, additional residues relative to the canonical positions are conventionally numbered with a, b, c and so forth next to the residue number in the canonical variable region numbering scheme (Al-Lazikani *et al.,* supra). Such nomenclature is similarly well known to those skilled in the art. Correspondence between the numbering system, including, for example, the Kabat numbering and the IMGT unique numbering system, is well known to one skilled in the art and shown below in Table 1. In some embodiments, the CDRs are as defined by the Kabat numbering system. In other embodiments, the CDRs are as defined by the IMGT numbering system. In yet other embodiments, the CDRs are as defined by the AbM numbering system. In still other embodiments, the CDRs are as defined by the Chothia numbering system. In yet other embodiments, the CDRs are as defined by the Contact numbering system.

**Table 1. Correspondence between the CDR Numbering Systems**

| | Kabat + Chothia | IMGT | Kabat | AbM | Chothia | Contact |
|---|---|---|---|---|---|---|
| V_{H} CDR1 | 26-35 | 27-38 | 31-35 | 26-35 | 26-32 | 30-35 |
| V_{H} CDR2 | 50-65 | 56-65 | 50-65 | 50-58 | 52-56 | 47-58 |
| V_{H} CDR3 | 95-102 | 105-117 | 95-102 | 95-102 | 95-102 | 93-101 |
| V_{L} CDR1 | 24-34 | 27-38 | 24-34 | 24-34 | 24-34 | 30-36 |
| V_{L} CDR2 | 50-56 | 56-65 | 50-56 | 50-56 | 50-56 | 46-55 |
| V_{L} CDR3 | 89-97 | 105-117 | 89-97 | 89-97 | 89-97 | 89-96 |

"Chemotherapeutic agent" is a chemical compound useful in the treatment of cancer. Classes of chemotherapeutic agents include, but are not limited to: alkylating agents (including platinum-containing chemotherapeutic agents), antimetabolites, kinase inhibitors, spindle poison plant alkaloids, cytoxic/antitumor antibiotics, topoisomerase inhibitors, photosensitizers, anti-estrogens and selective estrogen receptor modulators (SERMs), anti-progesterones, estrogen receptor down-regulators (ERDs), estrogen receptor antagonists, leutinizing hormone-releasing hormone agonists, anti-androgens, aromatase inhibitors, EGFR inhibitors, VEGF inhibitors, and anti-sense oligonucleotides that inhibit expression of genes implicated in abnormal cell proliferation or tumor growth. Chemotherapeutic agents useful in the treatment methods of the present disclosure include cytostatic and/or cytotoxic agents.

"Chimeric antibody" refers to an antibody in which a portion of the heavy and/or light chain contains sequences derived from a particular species (*e.g.,* human) or belonging to a particular antibody class or subclass, while the remainder of the chain(s) is derived from another species (*e.g.,* mouse) or belonging to another antibody class or subclass, as well as fragments of such antibodies, so long as they exhibit the desired biological activity.

As used herein, the terms "combination therapy" and "therapeutic combination" refer to treatments in which at least one anti-human PD-1 antibody (or antigen-binding fragment thereof) and Immunoconjugate of Formula (I), and optionally additional therapeutic agents, each are administered to a patient in a coordinated manner, over an overlapping period of time. The period of treatment with the at least one anti-human PD-1 antibody (or antigen-binding fragment thereof) (the "anti-PD-1 treatment") is the period of time that a patient undergoes treatment with the anti-human PD-1 antibody (or antigen-binding fragment thereof); that is, the period of time from the initial dosing with the anti-human PD-1 antibody (or antigen-binding fragment thereof) through the final day of a treatment cycle. Similarly, the period of treatment with the Immunoconjugate of Formula (I) (the "Immunoconjugate of Formula (I) treatment") is the period of time that a patient undergoes treatment with the Immunoconjugate of Formula (I); that is, the period of time from the initial dosing with the Immunoconjugate of Formula (I) through the final day of a treatment cycle. In the methods and therapeutic combinations described herein, the anti-PD-1 treatment overlaps by at least one day the Immunoconjugate of Formula (I) treatment. In certain embodiments, the anti-PD-1 treatment and the Immunoconjugate of Formula (I) treatment are coextensive. In embodiments, the anti-PD-1 treatment begins prior to the Immunoconjugate of Formula (I) treatment. In embodiments, the Immunoconjugate of Formula (I) treatment begins prior to the anti-PD-1 treatment. In embodiments, the anti-PD-1 treatment is terminated prior to termination of Immunoconjugate of Formula (I) treatment. In embodiments, the Immunoconjugate of Formula (I) treatment is terminated prior to termination of the anti-PD-1 treatment.

The terms "complementarity-determining region" and "CDR," which are synonymous with "hypervariable region" or "HVR," refer to subregions within the antibody variable domains, which confer the antibody's specificity and/or affinity for its antigen. In general, there are three CDRs in each heavy chain variable domain (HCDR1, HCDR2, and HCDR3) and three CDRs in each light chain variable domain (LCDR1, LCDR2, and LCDR3). "Framework regions" ("FRs") refer to the non-CDR portions of the variable domains. In general, there are four FRs in each full-length heavy chain variable domain and four FRs in each full-length light chain variable domain. The precise amino acid sequence boundaries of a given CDR or FR can be readily determined using any of several well-known schemes, including those described by Kabat et al., 5th Ed., Public Health Service, National Institutes of Health, Bethesda, MD (1991) ("Kabat" numbering scheme); Al-Lazikani et al., JMB 273,927-948 (1997) ("Chothia" numbering scheme); MacCallum et al., J. Mol. Biol. 262:732-745 (1996) ("contact" numbering scheme); Lefranc et al., Dev Comp Immunol. 27(1):55-77 (2003) ("IMGT" numbering scheme); and Honegger and Pluckthun, J Mol Biol, 309(3):657-70 (2001) ("Aho" numbering scheme).

The boundaries of a given CDR or FR may vary depending on the scheme used for identification. For example, the Kabat scheme is based on sequence alignments, while the Chothia scheme is based on structural information. Numbering for both the Kabat and Chothia schemes is based upon the most common antibody region sequence lengths, with insertions accommodated by insertion letters, for example, "30a." The two schemes place certain insertions and deletions ("indels") at different positions, resulting in differential numbering. The contact scheme is based on analysis of complex crystal structures and is similar in many respects to the Chothia numbering scheme. Unless indicated otherwise, the CDRs of the antibodies referred to herein may be identified according to any of the Kabat, Chothia, IMGT, and contact methods.

"Conservatively modified variants" or "conservative substitution" refers to substitutions of amino acids in a protein with other amino acids having similar characteristics (*e.g*., charge, side-chain size, hydrophobicity/hydrophilicity, backbone conformation and rigidity, *etc.*)*,* such that the changes can frequently be made without altering the biological activity or other desired property of the protein, such as antigen affinity and/or specificity. Those of skill in this art recognize that, in general, single amino acid substitutions in non-essential regions of a polypeptide do not substantially alter biological activity (*see, e.g.,* Watson et al. (1987) Molecular Biology of the Gene, The Benjamin/Cummings Pub. Co., p. 224 (4th Ed.)). In addition, substitutions of structurally or functionally similar amino acids are less likely to disrupt biological activity. Exemplary conservative substitutions are set forth in Table 2 below.

**Table 2. Exemplary Conservative Amino Acid Substitutions**

| Original residue | Conservative substitution |
|---|---|
| Ala (A) | Gly; Ser |
| Arg (R) | Lys; His |
| Asn (N) | Gln; His |
| Asp (D) | Glu; Asn |
| Cys (C) | Ser; Ala |
| Gln (Q) | Asn |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro (P) | Ala |
| Ser (S) | Thr |
| Thr (T) | Ser |
| Trp (W) | Tyr; Phe |
| Tyr (Y) | Trp; Phe |
| Val (V) | Ile; Leu |

The term "DAR" or "Drug Antibody Ratio," as used herein, refers to: (a) the number of linker/drug moieties attached to an antibody in a single Antibody-Drug Conjugate molecule (i.e., "n"), which is an integer from 0 to 10, such as an integer from 1 to 10; or (b) the average number of linker/drug moieties attached to an antibody in a composition comprising more than one Antibody-Drug Conjugate molecule (i.e., "*n̅*"), which is a decimal or integer from 0 to 10, such as a decimal or integer from 1 to 10. Accordingly, in one embodiment, for an Antibody-Drug Conjugate of the present disclosure, the DAR is an integer or decimal from 0 to 10, 0 to 9, 0 to 8, from 0 to 7, from 0 to 6, from 0 to 5, from 0 to 4, from 0 to 3, from 0 to 2, and from 0 to 1. In some embodiments, for the antibody-drug conjugate of the present disclosure, the DAR is an integer or decimal of 1 to 10, 1 to 9, 1 to 8, 1 to 7, 1 to 6, 1 to 5, 1 to 4, 1 to 3, 1 to 2, 2 to 10, 2 to 9, 2 to 8, 2 to 7, 2 to 6, 2 to 5, 2 to 4, 2 to 3, 3 to 10, 3 to 9, 3 to 8, 3 to 7, 3 to 6, 3 to 5, 3 to 4, 4 to 10, 4 to 9, 4 to 8, 4 to 7, 4 to 6, 4 to 5, 5 to 10, 5 to 9, 5 to 8, 5 to 7, 5 to 6, 6 to 10, 6 to 9, 6 to 8, 6 to 7, 7 to 10, 7 to 9, 7 to 8, 8 to 10, 8 to 9, or 9 to 10. In some embodiments, for an Antibody-Drug Conjugate of the present disclosure, the DAR is an integer or decimal from 1 to 8, 1 to 4, 2 to 5, 3 to 6, 4 to 7, 5 to 8, and 6 to 8. In other embodiments, for an Antibody-Drug Conjugates of the present disclosure, the DAR is an integer or decimal from 1 to 3, 2 to 4, 3 to 5, 4 to 6, 5 to 7, and 6 to 8. In further embodiments, for an Antibody-Drug Conjugate of the present disclosure, the DAR is an integer or decimal from 1 to 2, 2 to 3, 3 to 4, 4 to 5, 5 to 6, 6 to 7, and 7 to 8. Likewise, for a composition comprising an Antibody-Drug Conjugate of the present disclosure, the DAR for the composition is an average of the DAR for all of the Antibody-Drug Conjugate molecules present in said composition. As such, for a composition comprising an Antibody-Drug Conjugate of the present disclosure, the DAR of the composition is an integer or decimal from 0 to 10, 0 to 9, 0 to 8, from 0 to 7, from 0 to 6, from 0 to 5, from 0 to 4, from 0 to 3, from 0 to 2, and from 0 to 1. In some embodiments, for a composition comprising the antibody-drug conjugate of the present disclosure, the DAR of the composition is an integer or decimal of 1 to 10, 1 to 9, 1 to 8, 1 to 7, 1 to 6, 1 to 5, 1 to 4, 1 to 3, 1 to 2, 2 to 10, 2 to 9, 2 to 8, 2 to 7, 2 to 6, 2 to 5, 2 to 4, 2 to 3, 3 to 10, 3 to 9, 3 to 8, 3 to 7, 3 to 6, 3 to 5, 3 to 4, 4 to 10, 4 to 9, 4 to 8, 4 to 7, 4 to 6, 4 to 5, 5 to 10, 5 to 9, 5 to 8, 5 to 7, 5 to 6, 6 to 10, 6 to 9, 6 to 8, 6 to 7, 7 to 10, 7 to 9, 7 to 8, 8 to 10, 8 to 9, or 9 to 10. In additional embodiments, for a composition comprising an Antibody-Drug Conjugate of the present disclosure, the DAR of the composition is an integer or decimal from 1 to 4, 2 to 5, 3 to 6, 4 to 7, 5 to 8, and 6 to 8. In other embodiments, for a composition comprising an Antibody-Drug Conjugate of the present disclosure, the DAR of the composition is an integer or decimal from 1 to 3, 2 to 4, 3 to 5, 4 to 6, 5 to 7, and 6 to 8. In further embodiments, for a composition comprising an Antibody-Drug Conjugate of the present disclosure, the DAR of the composition is an integer or decimal from 1 to 2, 2 to 3, 3 to 4, 4 to 5, 5 to 6, 6 to 7, and 7 to 8. The term "composition" as used above, is understood to encompass pharmaceutical compositions.

The therapeutic agents and compositions provided by the present disclosure can be administered via any suitable enteral route or parenteral route of administration. The term "enteral route" of administration refers to the administration via any part of the gastrointestinal tract. Examples of enteral routes include oral, mucosal, buccal, and rectal route, or intragastric route. "Parenteral route" of administration refers to a route of administration other than enteral route. Examples of parenteral routes of administration include intravenous, intramuscular, intradermal, intraperitoneal, intratumor, intravesical, intraarterial, intrathecal, intracapsular, intraorbital, intracardiac, transtracheal, intraarticular, subcapsular, subarachnoid, intraspinal, epidural and intrasternal, subcutaneous, or topical administration. The therapeutic agents and compositions of the disclosure can be administered using any suitable method, such as by oral ingestion, nasogastric tube, gastrostomy tube, injection, infusion, implantable infusion pump, and osmotic pump. A suitable route and method of administration may vary depending on a number of factors such as the specific therapeutic agent being used, the rate of absorption desired, specific formulation or dosage form used, type or severity of the disorder being treated, the specific site of action, and conditions of the patient, and can be readily selected by a person skilled in the art.

"Homology" refers to sequence similarity between two polypeptide sequences when they are optimally aligned. When a position in both of the two compared sequences is occupied by the same amino acid monomer subunit, *e.g.,* if a position in a light chain CDR of two different Abs is occupied by alanine, then the two Abs are homologous at that position. The percent of homology is the number of homologous positions shared by the two sequences divided by the total number of positions compared × 100. For example, if 8 of 10 of the positions in two sequences are matched when the sequences are optimally aligned then the two sequences are 80% homologous. Generally, the comparison is made when two sequences are aligned to give maximum percent homology. For example, the comparison can be performed by a BLAST algorithm wherein the parameters of the algorithm are selected to give the largest match between the respective sequences over the entire length of the respective reference sequences.

The following references relate to BLAST algorithms often used for sequence analysis: BLAST ALGORITHMS: Altschul, S.F., et al., (1990) J. Mol. Biol. 215:403-410; Gish, W., et al., (1993) Nature Genet. 3:266-272; Madden, T.L., et al., (1996) Meth. Enzymol. 266:131-141; Altschul, S.F., et al., (1997) Nucleic Acids Res. 25:3389-3402; Zhang, J., et al., (1997) Genome Res. 7:649-656; Wootton, J.C., et al., (1993) Comput. Chem. 17:149-163; Hancock, J.M. et al., (1994) Comput. Appl. Biosci. 10:67-70; ALIGNMENT SCORING SYSTEMS: Dayhoff, M.O., et al., "A model of evolutionary change in proteins." in Atlas of Protein Sequence and Structure, (1978) vol. 5, suppl. 3. M.O. Dayhoff (ed.), pp. 345-352, Natl. Biomed. Res. Found., Washington, DC; Schwartz, R.M., et al., "Matrices for detecting distant relationships." in Atlas of Protein Sequence and Structure, (1978) vol. 5, suppl. 3." M.O. Dayhoff (ed.), pp. 353-358, Natl. Biomed. Res. Found., Washington, DC; Altschul, S.F., (1991) J. Mol. Biol. 219:555-565; States, D.J., et al., (1991) Methods 3:66-70; Henikoff, S., et al., (1992) Proc. Natl. Acad. Sci. USA 89:10915-10919; Altschul, S.F., et al., (1993) J. Mol. Evol. 36:290-300; ALIGNMENT STATISTICS: Karlin, S., et al., (1990) Proc. Natl. Acad. Sci. USA 87:2264-2268; Karlin, S., et al., (1993) Proc. Natl. Acad. Sci. USA 90:5873-5877; Dembo, A., et al., (1994) Ann. Prob. 22:2022-2039; and Altschul, S.F. "Evaluating the statistical significance of multiple distinct local alignments." in Theoretical and Computational Methods in Genome Research (S. Suhai, ed.), (1997) pp. 1-14, Plenum, New York.

"Human antibody" refers to an antibody that comprises human immunoglobulin protein sequences or derivatives thereof. A human antibody may contain murine carbohydrate chains if produced in a mouse, in a mouse cell, or in a hybridoma derived from a mouse cell. Similarly, "mouse antibody" or "rat antibody" refer to an antibody that comprises only mouse or rat immunoglobulin sequences or derivatives thereof, respectively.

"Humanized antibody" refers to forms of antibodies that contain sequences from non-human (*e.g.,* murine) antibodies as well as human antibodies. Such antibodies contain minimal sequence derived from non-human immunoglobulin. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. The prefix "hum", "hu" or "h" may be added to antibody clone designations when necessary to distinguish humanized antibodies from parental rodent antibodies. The humanized forms of rodent antibodies will generally comprise the same CDR sequences of the parental rodent antibodies, although certain amino acid substitutions may be included to increase affinity, increase stability of the humanized antibody, or for other reasons.

An "intact" antibody is one comprising an antigen-binding site as well as a CL and at least heavy chain constant regions, CH1, CH2 and CH3. The constant regions may include human constant regions or amino acid sequence variants thereof. In certain embodiments, an intact antibody has one or more effector functions.

As used herein, the term "immune response" relates to any one or more of the following: specific immune response, non-specific immune response, both specific and non-specific response, innate response, primary immune response, adaptive immunity, secondary immune response, memory immune response, immune cell activation, immune cell-proliferation, immune cell differentiation, and cytokine expression.

As used herein, the term "immune response" relates to any one or more of the following: specific immune response, non-specific immune response, both specific and non-specific response, innate response, primary immune response, adaptive immunity, secondary immune response, memory immune response, immune cell activation, immune cellular proliferation, immune cell differentiation, and cytokine expression.

The term "isolated" as used in reference to an antibody or fragment thereof refers to the purification status and, in such context, means the named molecule is substantially free of other biological molecules such as nucleic acids, proteins, lipids, carbohydrates, or other material such as cellular debris and growth media. Generally, the term "isolated" is not intended to refer to a complete absence of such material or to an absence of water, buffers, or salts, unless they are present in amounts that substantially interfere with experimental or therapeutic use of the binding compound as described herein.

"Kabat" as used herein means an immunoglobulin alignment and numbering system pioneered by Elvin A. Kabat ((1991) Sequences of Proteins of Immunological Interest, 5th Ed. Public Health Service, National Institutes of Health, Bethesda, Md.).

"Monoclonal antibody" or "mAb" or "Mab", as used herein, refers to a population of substantially homogeneous antibodies, *i.e.,* the antibody molecules comprising the population are identical in amino acid sequence except for possible naturally occurring mutations that may be present in minor amounts. In contrast, conventional (polyclonal) antibody preparations typically include a multitude of different antibodies having different amino acid sequences in their variable domains, particularly their CDRs, which are often specific for different epitopes. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al. (1975) Nature 256: 495, or may be made by recombinant DNA methods (*see, e.g.,* U.S. Pat. No. 4,816,567). The "monoclonal antibodies" may also be isolated from phage antibody libraries using the techniques described in Clackson et al. (1991) Nature 352: 624-628 and Marks et al. (1991) J. Mol. Biol. 222: 581-597, for example. *See also* Presta (2005) J. Allergy Clin. Immunol. 116:731.

"PD-1 antagonist" means any chemical compound or biological molecule that blocks binding of PD-L1 expressed on a cancer cell to PD-1 expressed on an immune cell (T cell, B cell or NKT cell) and preferably also blocks binding of PD-L2 expressed on a cancer cell to the immune-cell expressed PD-1. Alternative names or synonyms for PD-1 and its ligands include: PDCD1, PD1, CD279 and SLEB2 for PD-1; PDCD1L1, PDL1, B7H1, B7-4, CD274 and B7-H for PD-L1; and PDCD1L2, PDL2, B7-DC, Btdc and CD273 for PD-L2. In any of the treatment methods, compositons, and uses of the present disclosure in which a human individual is being treated, the PD-1 antagonist blocks binding of human PD-L1 to human PD-1, and preferably blocks binding of both human PD-L1 and PD-L2 to human PD-1. Human PD-1 amino acid sequences can be found in NCBI Locus No.: NP_005009. Human PD-L1 and PD-L2 amino acid sequences can be found in NCBI Locus No.: NP_054862 and NP_079515, respectively.

"Pembrolizumab" (formerly known as MK-3475, SCH 900475 and lambrolizumab) alternatively referred to herein as "pembro," is a humanized IgG4 mAb with the structure described in WHO Drug Information, Vol. 27, No. 2, pages 161-162 (2013) and which comprises the heavy and light chain amino acid sequences and CDRs described in Table 3. Pembrolizumab has been approved by the U.S. FDA as described in the Prescribing Information for KEYTRUDA^{®} (Merck & Co., Inc., Rahway, NJ USA; initial U.S. approval 2014, updated February 2023). The term pembrolizumab includes mAb's having a structure as described above (*Id*.) but which do not include the C-terminal lysine in the heavy chain.

"Pembrolizumab variant" as used herein means a monoclonal antibody that comprises heavy chain and light chain sequences that are identical to those in pembrolizumab, except for having three, two or one conservative amino acid substitutions at positions that are located outside of the light chain CDRs and six, five, four, three, two or one conservative amino acid substitutions that are located outside of the heavy chain CDRs, e.g., the variant positions are located in the framework regions or the constant region, and optionally has a deletion of the C-terminal lysine residues of the heavy chain. In other words, pembrolizumab and a pembrolizumab variant comprise identical CDR sequences, but differ from each other due to having a conservative amino acid substitution at no more than three or six other positions in their full length light and heavy chain sequences, respectively. A pembrolizumab variant is substantially the same as pembrolizumab with respect to the following properties: binding affinity to PD-1 and ability to block the binding of each of PD-L1 and PD-L2 to PD-1.

"Platinum-containing chemotherapy" (also known as platins) refers to the use of chemotherapeutic agent(s) used to treat cancer that are coordination complexes of platinum. Platinum-containing chemotherapeutic agents are alkylating agents that crosslink DNA, resulting in ineffective DNA mismatch repair and generally leading to apoptosis. Examples of platins include cisplatin, carboplatin, and oxaliplatin.

The term "patient" (alternatively "subject") as used herein refers to a mammal that has been the object of treatment, observation, or experiment. The mammal may be male or female. The mammal may be one or more selected from the group consisting of humans, bovine (*e.g.,* cows), porcine (*e.g.,* pigs), ovine (*e.g.,* sheep), capra (*e.g.,* goats), equine (*e.g.,* horses), canine (*e.g.,* domestic dogs), feline (*e.g.,* house cats), Lagomorpha (rabbits), rodents (*e.g.,* rats or mice), Procyon lotor (*e.g.,* raccoons). In particular embodiments, the patient is human.

The term "patient in need thereof" as used herein refers to a patient diagnosed with, or suspected of having, a cellular proliferative disorder, such as a cancer, as defined herein.

The term "pharmaceutically acceptable carrier" refers to any inactive substance that is suitable for use in a formulation for the delivery of a therapeutic agent. A carrier may be an antiadherent, binder, coating, disintegrant, filler or diluent, preservative (such as antioxidant, antibacterial, or antifungal agent), sweetener, absorption delaying agent, wetting agent, emulsifying agent, buffer, and the like. Examples of suitable pharmaceutically acceptable carriers include water, ethanol, polyols (such as glycerol, propylene glycol, polyethylene glycol, and the like), dextrose, vegetable oils (such as olive oil), saline, buffer, buffered saline, and isotonic agents such as sugars, polyalcohols, sorbitol, and sodium chloride.

The term "pharmaceutically acceptable salt" refers to a salt that possesses effectiveness similar to the parent compound and that is not biologically or otherwise undesirable (*e.g.,* is neither toxic nor otherwise deleterious to the recipient thereof). Thus, an embodiment of the present disclosure provides pharmaceutically acceptable salts of the compounds of the present disclosure. The term "salt(s)", as employed herein, denotes any of the following: acidic salts formed with inorganic and/or organic acids, as well as basic salts formed with inorganic and/or organic bases. Salts of compounds of the present disclosure may be formed by methods known to those of ordinary skill in the art, for example, by reacting a compound of the present disclosure with an amount of acid or base, such as an equivalent amount, in a medium such as one in which the salt precipitates or in aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates ("mesylates"), naphthalenesulfonates, nitrates, oxalates, phosphates, propionates, salicylates, succinates, sulfates, tartarates, thiocyanates, toluenesulfonates (also known as tosylates) and the like. Suitable salts include acid addition salts that may, for example, be formed by mixing a solution of a compound with a solution of a pharmaceutically acceptable acid such as hydrochloric acid, sulfuric acid, acetic acid, trifluoroacetic acid, or benzoic acid. Additionally, acids that are generally considered suitable for the formation of pharmaceutically useful salts from basic pharmaceutical compounds are discussed, for example, by P. Stahl et al., Camille G. (eds.), Handbook of Pharmaceutical Salts. Properties, Selection and Use. (2002) Zurich: Wiley-VCH; S. Berge et al., Journal of Pharmaceutical Sciences (1977) 66(1) 1-19; P. Gould, International J. of Pharmaceutics (1986) 33 201-217; Anderson et al., The Practice of Medicinal Chemistry (1996), Academic Press, New York; and in The Orange Book (Food & Drug Administration, Washington, D.C. on their website). These disclosures are incorporated herein by reference thereto.

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamine, *t*-butyl amine, choline, and salts with amino acids such as arginine, lysine and the like. Basic nitrogen-containing groups may be quarternized with agents such as lower alkyl halides (*e.g.,* methyl, ethyl, and butyl chlorides, bromides and iodides), dialkyl sulfates (*e.g.,* dimethyl, diethyl, and dibutyl sulfates), long chain halides (*e.g.,* decyl, lauryl, and stearyl chlorides, bromides and iodides), aralkyl halides (*e.g.,* benzyl and phenethyl bromides), and others. Compounds carrying an acidic moiety can be mixed with suitable pharmaceutically acceptable salts to provide, for example, alkali metal salts (*e.g.,* sodium or potassium salts), alkaline earth metal salts (*e.g.,* calcium or magnesium salts), and salts formed with suitable organic ligands such as quaternary ammonium salts. Also, in the case of an acid (-COOH) or alcohol group being present, pharmaceutically acceptable esters can be employed to modify the solubility or hydrolysis characteristics of the compound.

All such acid salts and base salts are intended to be pharmaceutically acceptable salts within the scope of the present disclosure and all acid and base salts are considered equivalent to the free forms of the corresponding compounds for purposes of the present disclosure.

In addition, when a compound of the present disclosure contains both a basic moiety, such as, but not limited to an aliphatic primary, secondary, tertiary or cyclic amine, an aromatic or heteroaryl amine, pyridine or imidazole, and an acidic moiety, such as, but not limited to tetrazole or carboxylic acid, zwitterions ("inner salts") may be formed and are included within the terms "salt(s)" as used herein. It is understood that certain compounds of the present disclosure may exist in zwitterionic form, having both anionic and cationic centers within the same compound and a net neutral charge. Such zwitterions are included within the present disclosure.

"RECIST 1.1 Response Criteria" as used herein means the definitions set forth in Eisenhauer, E.A. et al., Eur. J. Cancer 45:228-247 (2009) for target lesions or nontarget lesions, as appropriate based on the context in which response is being measured.

"Responder patient" when referring to a specific anti-tumor response to a treatment described herein, means the patient exhibited an anti-tumor response.

"Sustained response" means a sustained therapeutic effect after cessation of treatment as described herein. In some embodiments, the sustained response has a duration that is at least the same as the treatment duration, or at least 1.5, 2.0, 2.5 or 3 times longer than the treatment duration.

The term "simultaneous administration" as used herein in relation to the administration of medicaments refers to the administration of medicaments such that the individual medicaments are present within a patient at the same time. In addition to the concomitant administration of medicaments (via the same or alternative routes), simultaneous administration may include the administration of the medicaments (via the same or an alternative route) at different times.

"Sustained response" means a sustained therapeutic effect after cessation of treatment as described herein. In some embodiments, the sustained response has a duration that is at least the same as the treatment duration, or at least 1.5, 2.0, 2.5 or 3 times longer than the treatment duration.

"Treat" or "treating" a cellular proliferative disorder as used herein means to administer a combination therapy of an anti-human PD-1 antibody (or antigen binding fragment thereof) and an Immunoconjugate of Formula (I) to a patient having a cellular proliferative disorder, such as cancer, or diagnosed with a cellular proliferative disorder, such as cancer, to achieve at least one positive therapeutic effect, such as, for example, reduced number of cancer cells, reduced tumor size, reduced tumor burden, reduced rate of cancer cell infiltration into peripheral organs, or reduced rate of tumor metastasis or tumor growth, comprising administration by oral, mucosal, intradermal, intravenous, subcutaneous, intramuscular delivery, and/or any other methods of physical delivery described herein or known in the art. Typically, the agent(s) of the treatment method are administered in an amount effective to alleviate one or more disease symptoms in the treated patient or population, whether by inducing the regression of or inhibiting the progression of such symptom(s) by any clinically measurable degree. The amount of the agent(s) of the treatment method that is effective to alleviate any particular disease symptom may vary according to factors such as the disease state, age, and weight of the patient, and the ability of the therapeutic combination to elicit a desired response in the patient. Whether a disease symptom has been alleviated can be assessed by any clinical measurement typically used by physicians or other skilled healthcare providers to assess the severity or progression status of that symptom. "Treatment" may include one or more of the following: inducing/increasing an antitumor immune response, decreasing the number of one or more tumor markers, halting or delaying the growth of a tumor or blood cancer or progression of disease such as cancer, stabilization of disease, inhibiting the growth or survival of tumor cells, eliminating or reducing the size of one or more cancerous lesions or tumors, decreasing the level of one or more tumor markers, ameliorating or abrogating the clinical manifestations of disease, reducing the severity or duration of the clinical symptoms, prolonging the survival of the treated patient relative to the expected survival in a similar untreated patient, and inducing complete or partial remission of a cancerous condition, wherein the disease is cancer, more specifically, non-small cell lung cancer.

The amount of a therapeutic agent that is effective to alleviate any particular disease symptom may vary according to factors such as the disease state, age, and weight of the patient, and the ability of the drug to elicit a desired response in the patient. Whether a disease symptom has been alleviated can be assessed by any clinical measurement typically used by physicians or other skilled healthcare providers to assess the severity or progression status of that symptom.

Positive therapeutic effects in cancer can be measured in a number of ways (*See,* W. A. Weber, J. Nucl. Med. 50:1S-10S (2009)). For example, with respect to tumor growth inhibition, according to NCI standards, a T/C ≦ 42% is the minimum level of anti-tumor activity. A T/C < 10% is considered a high anti-tumor activity level, with T/C (%) = Median tumor volume of the treated/Median tumor volume of the control × 100. In some embodiments, the treatment achieved by a therapy of the disclosure is any of PR, CR, OR, PFS, DFS, and OS. PFS, also referred to as "Time to Tumor Progression" indicates the length of time during and after treatment that the cancer does not grow, and includes the amount of time patients have experienced a CR or PR, as well as the amount of time patients have experienced SD. DFS refers to the length of time during and after treatment that the patient remains free of disease. OS refers to a prolongation in life expectancy as compared to naive or untreated individuals or patients. In some embodiments, response to a therapy of the disclosure is any of PR, CR, PFS, DFS, or OR that is assessed using RECIST 1.1 response criteria. The treatment regimen for a therapy of the disclosure that is effective to treat a cancer patient may vary according to factors such as the disease state, age, and weight of the patient, and the ability of the therapy to elicit an anti-cancer response in the patient. While an embodiment of any of the aspects of the disclosure may not be effective in achieving a positive therapeutic effect in every patient, it should do so in a statistically significant number of patients as determined by any statistical test known in the art such as the Student's t-test, the chi²-test, the U-test according to Mann and Whitney, the Kruskal- Wallis test (H-test), Jonckheere-Terpstra-test and the Wilcoxon-test.

The terms "treatment regimen", "dosing protocol", and "dosing regimen" are used interchangeably to refer to the dose and timing of administration of each therapeutic agent in a combination therapy of the disclosure.

"Tumor" as it applies to a patient diagnosed with, or suspected of having, a cancer refers to a malignant or potentially malignant neoplasm or tissue mass of any size, and includes primary tumors and secondary neoplasms. A solid tumor is an abnormal growth or mass of tissue that usually does not contain cysts or liquid areas. Different types of solid tumors are named for the type of cells that form them. Examples of solid tumors are sarcomas, carcinomas, and lymphomas. Leukemias (cancers of the blood) generally do not form solid tumors (National Cancer Institute, Dictionary of Cancer Terms).

"Tumor burden" also referred to as "tumor load", refers to the total amount of tumor material distributed throughout the body. Tumor burden refers to the total number of cancer cells or the total size of tumor(s), throughout the body, including lymph nodes and bone narrow. Tumor burden can be determined by a variety of methods known in the art, such as, *e.g.,* by measuring the dimensions of tumor(s) upon removal from the patient, *e.g.,* using calipers, or while in the body using imaging techniques, *e.g.,* ultrasound, bone scan, computed tomography (CT) or magnetic resonance imaging (MRI) scans.

The term "tumor size" refers to the total size of the tumor which can be measured as the length and width of a tumor. Tumor size may be determined by a variety of methods known in the art, such as, *e.g.,* by measuring the dimensions of tumor(s) upon removal from the patient, *e.g.,* using calipers, or while in the body using imaging techniques, *e.g.,* bone scan, ultrasound, CT or MRI scans.

"Variable regions" as used herein means the segment of IgG chains which is variable in sequence between different antibodies. A "variable region" of an antibody refers to the variable region of the antibody light chain or the variable region of the antibody heavy chain, either alone or in combination. The variable region of the heavy chain may be referred to as "V_{H}." The variable region of the light chain may be referred to as "V_{L}."

Typically, the variable regions of both the heavy and light chains comprise three hypervariable regions, also called complementarity determining regions (CDRs), which are located within relatively conserved framework regions (FR). The CDRs are usually aligned by the framework regions, enabling binding to a specific epitope. In general, from N-terminal to C-terminal, both light and heavy chains variable domains comprise FR1, CDR1, FR2, CDR2, FR3, CDR3, and FR4. As referred to herein the light chain CDRs are CDRL1, CDRL2 and CDRL3, respectively, and the heavy chain CDRs are CDRH1, CDRH2 and CDRH3, respectively. The assignment of amino acids to each domain is, generally, in accordance with the definitions of Sequences of Proteins of Immunological Interest, Kabat, et al.; National Institutes of Health, Bethesda, Md.; 5th ed.; NIH Publ. No. 91-3242 (1991); Kabat (1978) Adv. Prot. Chem. 32:1-75; Kabat, et al., (1977) J. Biol. Chem. 252:6609-6616; Chothia, et al., (1987) J Mol. Biol. 196:901-917 or Chothia, et al., (1989) Nature 342:878-883.

The term "variant" when used in relation to an antibody (*e.g.,* an anti-PD-1 antibody, or an anti-TROP2 antibody) or an amino acid region within the antibody may refer to a peptide or polypeptide comprising one or more (such as, for example, about 1 to about 25, about 1 to about 20, about 1 to about 15, about 1 to about 10, or about 1 to about 5) amino acid sequence substitutions, deletions, and/or additions as compared to a native or unmodified sequence. For example, a variant of an anti-PD-1 antibody may result from one or more (such as, for example, about 1 to about 25, about 1 to about 20, about 1 to about 15, about 1 to about 10, or about 1 to about 5) changes to an amino acid sequence of a native or previously unmodified anti-PD-1 antibody. Variants may be naturally occurring or may be artificially constructed. Polypeptide variants may be prepared from the corresponding nucleic acid molecules encoding the variants. In specific embodiments, an antibody variant (*e.g.,* an anti-PD-1 antibody variant) at least retains the antibody functional activity. In some embodiments, an anti-PD-1 antibody variant binds to PD-1 and/or is antagonistic to PD-1 activity.

Where aspects or embodiments of the disclosure are described in terms of a Markush group or other grouping of alternatives, the present disclosure encompasses not only the entire group listed as a whole, but each member of the group individually and all possible subgroups of the main group, but also the main group absent one or more of the group members. The present disclosure also envisages the explicit exclusion of one or more of any of the group members in the claims.

All ranges disclosed herein are inclusive of the recited endpoint and independently combinable (for example, the range of "from 50 mg to 500 mg" is inclusive of the endpoints, 50 mg and 500 mg, and all the intermediate values). The endpoints of the ranges and any values disclosed herein are not limited to the precise range or value; they are sufficiently imprecise to include values approximating these ranges and/or values.

Unless otherwise defined, all technical and scientific terms used herein have the same meaning as commonly understood by one of ordinary skill in the art to which this disclosure relates. In case of conflict, the present specification, including definitions, will control. Throughout this specification and claims, the word "comprise," or variations such as "comprises" or "comprising" will be understood to imply the inclusion of a stated integer or group of integers but not the exclusion of any other integer or group of integers. Unless otherwise required by context, singular terms shall include pluralities and plural terms shall include the singular. Any example(s) following the term *"e.g."* or "for example" is not meant to be exhaustive or limiting.

Exemplary methods and materials are described herein, although methods and materials similar or equivalent to those described herein can also be used in the practice or testing of the present disclosure. The materials, methods, and examples are illustrative only and not intended to be limiting.

The following Abbreviations are used herein, and are defined as follows:
- AE: Adverse Effect
- ALK: Anaplastic lymphoma kinase
- ALP: Alkaline phosphatase
- ALT: Alanine transaminase
- AST: Aspartate aminotransferase
- BRAF: v-raf murine sarcoma viral oncogene homolog B1
- CDR: Complementary determining region
- DFS: Disease free survival
- DLT: Dose limiting toxicity
- EGFR: Epidermal growth factor receptor
- EGFR-TKI: Epidermal growth factor tyrosine kinase inhibitor
- FR: Framework region
- HBV-DNA: Hepatitis B virus DNAs
- IgG: Immunoglobulin G
- IgG1: Immunoglobulin G subclass 1
- IHC: Immune histochemistry
- irAE: Immune-related adverse effect
- mAb: Monoclonal antibody
- MPK or mpk: Milligram per kilogram
- mTPI: Modified toxicity probability interval
- mTPI-2: Modified toxicity probability interval 2
- n: Number of patients in a treatment group
- NCI: National Cancer Institute
- NTRK: Neurotrophic tyrosine receptor kinase
- OR: Overall response
- ORR: Overall response rate
- OS: Overall survival
- PBS: Phosphate-buffered saline
- PD-1: Programmed cell death protein 1
- PFS: Progression free survival
- PR: Partial response
- *p*-values: Calculated probability
- QD: One dose per day
- RECIST: Response Evaluation Criteria in Solid Tumors
- ROSI: Round spermatid injection
- RP2D: Recommended phase 2 dose
- SD: Stable disease
- SEM: Standard error of the mean
- T/C: Median tumor volume of the treated animal/Median tumor volume of the control animal
- TPS: Tissue polypeptide specific antigen
- ULN: Upper limit of normal
- V_{H}: immunoglobulin heavy chain variable region
- V_{K}: immunoglobulin kappa light chain variable region
- V_{L}: immunoglobulin light chain variable region

Additional abbreviations may be defined throughout this disclosure.

The present disclosure relates to a therapeutic combinations, wherein the therapeutic combinations comprise: (a) an anti-human PD-1 antibody (or antigen-binding fragment thereof); and (b) an Immunoconjugate of Formula (I).

The present disclosure relates to methods of treating a cellular proliferative disorder as defined herein, wherein the method comprises administering to a human patient in need thereof a combination therapy that comprises (a) an anti-human PD-1 antibody (or antigen-binding fragment thereof); and (b) an Immunoconjugate of Formula (I).

In some embodiments of the methods and the therapeutic combination, the anti-human PD-1 antibody is pembrolizumab and the Immunoconjugate of Formula (I) is the compound of Formula I.

In preferred embodiments, the Immunoconjugate of Formula (I) is Immunoconjugate A, which is described in US Patent Publication No. 20200347075. Immunoconjugate A has the structure: 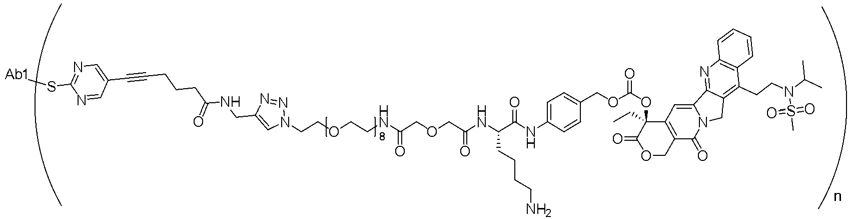
wherein n is an integer or decimal from 1 to 8; and
Ab1 is sacituzumab.

### PD-1 antagonists or anti-human PD-1 monoclonal antibodies useful in the present disclosure

Examples of mAbs that bind to human PD-1, useful in the methods, compositions, kits, and uses of the present disclosure, are described in US 7,521,051, US 8,008,449, and US 8,354,509. Specific anti-human PD-1 mAbs useful as the PD-1 antagonist in the methods, compositions, kits, and uses of the present disclosure include: pembrolizumab (formerly known as MK-3475, SCH 900475 and lambrolizumab), a humanized IgG4 mAb with the structure described in WHO Drug Information, Vol. 27, No. 2, pages 161-162 (2013) and which comprises the heavy and light chain amino acid sequences shown in Table 3, and the humanized antibodies h409A11, h409A16 and h409A17, which are described in WO 2008/156712.

Additional examples of PD-1 antagonists useful in the methods, compositions, kits, and uses of the present disclosure include, but are not limited to: nivolumab (OPDIVO^{®}, Bristol-Myers Squibb Company, Princeton, NJ, USA), cemiplimab (LIBTAYO^{®}, Regeneron Pharmaceuticals, Inc., Tarrytown, NY and Sanofi-Aventis U.S. LLC., Bridgewater, NJ) and dostarlimab (JEMPERLI^{®}, GlaxoSmithKline LLC, Philadelphia, PA).

Provided herein are PD-1 antagonists or anti-human PD-1 monoclonal antibodies that can be used in any of the methods, compositions, kits, and uses disclosed herein, including any chemical compound or biological molecule that blocks binding of PD-L1 to PD-1 and preferably also blocks binding of PD-L2 to PD-1.

Any monoclonal antibodies that bind to a PD-1 polypeptide, a PD-1 polypeptide fragment, a PD-1 peptide, or a PD-1 epitope and block the interaction between PD-1 and its ligand PD-L1 or PD-L2 can be used. In some embodiments, the anti-human PD-1 monoclonal antibody binds to a PD-1 polypeptide, a PD-1 polypeptide fragment, a PD-1 peptide, or a PD-1 epitope and blocks the interaction between PD-1 and PD-L1. In other embodiments, the anti-human PD-1 monoclonal antibody binds to a PD-1 polypeptide, a PD-1 polypeptide fragment, a PD-1 peptide, or a PD-1 epitope and blocks the interaction between PD-1 and PD-L2. In yet other embodiments, the anti-human PD-1 monoclonal antibody binds to a PD-1 polypeptide, a PD-1 polypeptide fragment, a PD-1 peptide, or a PD-1 epitope and blocks the interaction between PD-1 and PD-L1 and the interaction between PD-1 and PD-L2.

In certain embodiments, the anti-human PD-1 monoclonal antibody is selected from the group consisting of pembrolizumab, nivolumab, cemiplimab, dostarlimab, pidilizumab (U.S. Pat. No. 7,332,582), AMP-514 (MedImmune LLC, Gaithersburg, MD), PDR001 (U.S. Pat. No. 9,683,048), tislelizumab BGB-A317 (U.S. Pat. No. 8,735,553), and INCMGA00012 or MGA012 (MacroGenics, Rockville, MD). In one embodiment, the anti-human PD-1 monoclonal antibody is pembrolizumab. In one embodiment, the anti-human PD-1 monoclonal antibody is pembrolizumab. In another embodiment, the anti-human PD-1 monoclonal antibody is nivolumab. In another embodiment, the anti-human PD-1 monoclonal antibody is cemiplimab. In yet another embodiment, the anti-human PD-1 monoclonal antibody is pidilizumab. In one embodiment, the anti-human PD-1 monoclonal antibody is AMP-514. In another embodiment, the anti-human PD-1 monoclonal antibody is PDR001. In yet another embodiment, the anti-human PD-1 monoclonal antibody is BGB-A317. In still another embodiment, the anti-human PD-1 monoclonal antibody is MGA012.

In some embodiments, an anti-human PD-1 antibody or antigen binding fragment thereof for use in the methods and uses disclosed herein comprises three light chain CDRs of CDRL1, CDRL2 and CDRL3 and/or three heavy chain CDRs of CDRH1, CDRH2 and CDRH3.

In one embodiment, CDRL1 has the amino acid sequence as set forth in SEQ ID NO:1 or a variant of the amino acid sequence as set forth in SEQ ID NO:1, CDRL2 has the amino acid sequence as set forth in SEQ ID NO:2 or a variant of the amino acid sequence as set forth in SEQ ID NO:2, and CDRL3 has the amino acid sequence as set forth in SEQ ID NO:3 or a variant of the amino acid sequence as set forth in SEQ ID NO:3.

In one embodiment of the anti-human PD-1 antibody or antigen binding fragment thereof, CDRH1 has the amino acid sequence as set forth in SEQ ID NO:6 or a variant of the amino acid sequence as set forth in SEQ ID NO:6, CDRH2 has the amino acid sequence as set forth in SEQ ID NO:7 or a variant of the amino acid sequence as set forth in SEQ ID NO:7, and CDRH3 has the amino acid sequence as set forth in SEQ ID NO:8 or a variant of the amino acid sequence as set forth in SEQ ID NO:8.

In one embodiment of the anti-human PD-1 antibody or antigen binding fragment thereof, the three light chain CDRs have the amino acid sequences as set forth in SEQ ID NO:1, SEQ ID NO:2, and SEQ ID NO:3 and the three heavy chain CDRs have the amino acid sequences as set forth in SEQ ID NO:6, SEQ ID NO:7 and SEQ ID NO:8.

In an alternative embodiment of the anti-human PD-1 antibody or antigen binding fragment thereof, CDRL1 has the amino acid sequence as set forth in SEQ ID NO:11 or a variant of the amino acid sequence as set forth in SEQ ID NO:11, CDRL2 has the amino acid sequence as set forth in SEQ ID NO:12 or a variant of the amino acid sequence as set forth in SEQ ID NO:12, and CDRL3 has the amino acid sequence as set forth in SEQ ID NO:13 or a variant of the amino acid sequence as set forth in SEQ ID NO:13.

In one embodiment of the anti-human PD-1 antibody or antigen binding fragment thereof, CDRH1 has the amino acid sequence as set forth in SEQ ID NO:16 or a variant of the amino acid sequence as set forth in SEQ ID NO:16, CDRH2 has the amino acid sequence as set forth in SEQ ID NO:17 or a variant of the amino acid sequence as set forth in SEQ ID NO:17, and CDRH3 has the amino acid sequence as set forth in SEQ ID NO:18 or a variant of the amino acid sequence as set forth in SEQ ID NO:18.

In an alternative embodiment of the anti-human PD-1 antibody or antigen binding fragment thereof, the three light chain CDRs have the amino acid sequences as set forth in SEQ ID NO:11, SEQ ID NO:12, and SEQ ID NO:13 and the three heavy chain CDRs have the amino acid sequences as set forth in SEQ ID NO: 16, SEQ ID NO: 17 and SEQ ID NO: 18.

In a further embodiment, CDRL1 has the amino acid sequence as set forth in SEQ ID NO:21 or a variant of the amino acid sequence as set forth in SEQ ID NO:21, CDRL2 has the amino acid sequence as set forth in SEQ ID NO:22 or a variant of the amino acid sequence as set forth in SEQ ID NO:22, and CDRL3 has the amino acid sequence as set forth in SEQ ID NO:23 or a variant of the amino acid sequence as set forth in SEQ ID NO:23.

In yet another embodiment of the anti-human PD-1 antibody or antigen binding fragment thereof, CDRH1 has the amino acid sequence as set forth in SEQ ID NO:24 or a variant of the amino acid sequence as set forth in SEQ ID NO:24, CDRH2 has the amino acid sequence as set forth in SEQ ID NO: 25 or a variant of the amino acid sequence as set forth in SEQ ID NO:25, and CDRH3 has the amino acid sequence as set forth in SEQ ID NO:26 or a variant of the amino acid sequence as set forth in SEQ ID NO:26.

In another embodiment of the anti-human PD-1 antibody or antigen binding fragment thereof, the three light chain CDRs have the amino acid sequences as set forth in SEQ ID NO:21, SEQ ID NO:22, and SEQ ID NO:23 and the three heavy chain CDRs have the amino acid sequences as set forth in SEQ ID NO:24, SEQ ID NO:25 and SEQ ID NO:26.

In a sub embodiment of the anti-human PD-1 embodiments above, the anti-human PD-1 antibody or antigen binding fragment is an antibody.

Some anti-human PD-1 antibody and antigen binding fragments disclosed herein comprise a light chain variable region and a heavy chain variable region. In some embodiments, the light chain variable region comprises the amino acid sequence as set forth in SEQ ID NO:4 or a variant of the amino acid sequence as set forth in SEQ ID NO:4, and the heavy chain variable region comprises the amino acid sequence as set forth in SEQ ID NO:9 or a variant of the amino acid sequence as set forth in SEQ ID NO:9. In further embodiments, the light chain variable region comprises the amino acid sequence as set forth in SEQ ID NO: 14 or a variant of the amino acid sequence as set forth in SEQ ID NO:14, and the heavy chain variable region comprises the amino acid sequence as set forth in SEQ ID NO:19 or a variant of the amino acid sequence as set forth in SEQ ID NO:19. In further embodiments, the heavy chain variable region comprises the amino acid sequence as set forth in SEQ ID NO:27 or a variant of the amino acid sequence as set forth in SEQ ID NO:27 and the light chain variable region comprises the amino acid sequence as set forth in SEQ ID NO:28 or a variant of the amino acid sequence as set forth in SEQ ID NO:28, the amino acid sequence as set forth in SEQ ID NO:29 or a variant of the amino acid sequence as set forth in SEQ ID NO:29, or the amino acid sequence as set forth in SEQ ID NO:30 or a variant of the amino acid sequence as set forth in SEQ ID NO:30. In such embodiments, a light chain variable region or heavy chain variable region sequence is identical to the reference sequence except having one, two, three, four or five amino acid substitutions. In some embodiments, the substitutions are in the framework region (*i.e.,* outside of the CDRs). In some embodiments, one, two, three, four or five of the amino acid substitutions are conservative substitutions.

In one embodiment of the methods, kits or uses disclosed herein, the anti-human PD-1 antibody or antigen binding fragment comprises a light chain variable region comprising or consisting of the amino acid sequence as set forth in SEQ ID NO:4 and a heavy chain variable region comprising or consisting of the amino acid sequence as set forth in SEQ ID NO:9. In a further embodiment, the anti-human PD-1 antibody or antigen binding fragment comprises a light chain variable region comprising or consisting of the amino acid sequence as set forth in SEQ ID NO:14 and a heavy chain variable region comprising or consisting of the amino acid sequence as set forth in SEQ ID NO:19. In one embodiment of the compositions, methods, kits, and uses disclosed herein, the anti-human PD-1 antibody or antigen binding fragment comprises a light chain variable region comprising or consisting of the amino acid sequence as set forth in SEQ ID NO:28 and a heavy chain variable region comprising or consisting of the amino acid sequence as set forth in SEQ ID NO:27. In a further embodiment, the anti-human PD-1 antibody or antigen binding fragment comprises a light chain variable region comprising or consisting of the amino acid sequence as set forth in SEQ ID NO:29 and a heavy chain variable region comprising or consisting of the amino acid sequence as set forth in SEQ ID NO:27. In another embodiment, the antibody or antigen binding fragment comprises a light chain variable region comprising or consisting of the amino acid sequence as set forth in SEQ ID NO:30 and a heavy chain variable region comprising or consisting of the amino acid sequence as set forth in SEQ ID NO:27.

In another embodiment, the methods, kits or uses disclosed herein comprise an anti-human PD-1 antibody or antigen binding protein that has a V_{L} domain and/or a V_{H} domain with at least 99%, 98%, 97%, 96%, 95%, 90%, 85%, 80%, 75% or 50% sequence homology to one of the V_{L} domains or V_{H} domains described above, and exhibits specific binding to PD-1. In another embodiment, the anti-human PD-1 antibody or antigen binding protein of the present methodsm comprises V_{L} and V_{H} domains having up to 1, 2, 3, 4, or 5 or more amino acid substitutions, and exhibits specific binding to PD-1.

In any of the embodiments above, the PD-1 antagonist may be a full-length anti-PD-1 antibody or an antigen binding fragment thereof that specifically binds human PD-1. In certain embodiments, the PD-1 antagonist is a full-length anti-PD-1 antibody selected from any class of immunoglobulins, including IgM, IgG, IgD, IgA, and IgE. Preferably, the antibody is an IgG antibody. Any isotype of IgG can be used, including IgG₁, IgG₂, IgG₃, and IgG₄. Different constant domains may be appended to the V_{L} and V_{H} regions provided herein. For example, if a particular intended use of an antibody (or fragment) disclosed herein were to call for altered effector functions, a heavy chain constant domain other than IgG1 may be used. Although IgG1 antibodies provide for long half-life and for effector functions, such as complement activation and antibody-dependent cellular cytotoxicity, such activities may not be desirable for all uses of the antibody. In such instances an IgG4 constant domain, for example, may be used.

In certain embodiments, the PD-1 antagonist is an anti-PD-1 antibody comprising a light chain comprising or consisting of a sequence of amino acid residues as set forth in SEQ ID NO:5 and a heavy chain comprising or consisting of a sequence of amino acid residues as set forth in SEQ ID NO:10. In alternative embodiments, the PD-1 antagonist is an anti-PD-1 antibody comprising a light chain comprising or consisting of a sequence of amino acid residues as set forth in SEQ ID NO:15 and a heavy chain comprising or consisting of a sequence of amino acid residues as set forth in SEQ ID NO:20. In further embodiments, the PD-1 antagonist is an anti-PD-1 antibody comprising a light chain comprising or consisting of a sequence of amino acid residues as set forth in SEQ ID NO:32 and a heavy chain comprising or consisting of a sequence of amino acid residues as set forth in SEQ ID NO:31. In additional embodiments, the PD-1 antagonist is an anti-PD-1 antibody comprising a light chain comprising or consisting of a sequence of amino acid residues as set forth in SEQ ID NO:33 and a heavy chain comprising or consisting of a sequence of amino acid residues as set forth in SEQ ID NO:31. In yet additional embodiments, the PD-1 antagonist is an anti-PD-1 antibody comprising a light chain comprising or consisting of a sequence of amino acid residues as set forth in SEQ ID NO:34 and a heavy chain comprising or consisting of a sequence of amino acid residues as set forth in SEQ ID NO:31. In some compositions, methods, kits, and uses disclosed herein, the PD-1 antagonist is pembrolizumab or a pembrolizumab biosimilar. In some compositions, methods, kits, and uses disclosed herein, the PD-1 antagonist is nivolumab or a nivolumab biosimilar. In one such embodiment, the agent is pembrolizumab. In another such embodiment, the agent is nivolumab. In another such embodiment, the agent is cemiplimab. In another such embodiment, the agent is dostarlimab.

Ordinarily, amino acid sequence variants of the anti-PD-1 antibodies and antigen binding fragments disclosed herein will have an amino acid sequence having at least 75% amino acid sequence identity with the amino acid sequence of a reference antibody or antigen binding fragment (e.g. heavy chain, light chain, V_{H}, V_{L}, or humanized sequence), more preferably at least 80%, more preferably at least 85%, more preferably at least 90%, and most preferably at least 95, 98, or 99%. Identity or homology with respect to a sequence is defined herein as the percentage of amino acid residues in the candidate sequence that are identical with the anti-PD-1 residues, after aligning the sequences and introducing gaps, if necessary, to achieve the maximum percent sequence identity, and not considering any conservative substitutions as part of the sequence identity. None of N-terminal, C-terminal, or internal extensions, deletions, or insertions into the antibody sequence shall be construed as affecting sequence identity or homology.

Sequence identity refers to the degree to which the amino acids of two polypeptides are the same at equivalent positions when the two sequences are optimally aligned. Sequence identity can be determined using a BLAST algorithm wherein the parameters of the algorithm are selected to give the largest match between the respective sequences over the entire length of the respective reference sequences. The following references relate to BLAST algorithms often used for sequence analysis: BLAST ALGORITHMS: Altschul, S.F., et al., (1990) J. Mol. Biol. 215:403-410; Gish, W., et al., (1993) Nature Genet. 3:266-272; Madden, T.L., et al., (1996) Meth. Enzymol. 266:131-141; Altschul, S.F., et al., (1997) Nucleic Acids Res. 25:3389-3402; Zhang, J., et al., (1997) Genome Res. 7:649-656; Wootton, J.C., et al., (1993) Comput. Chem. 17:149-163; Hancock, J.M. et al., (1994) Comput. Appl. Biosci. 10:67-70; ALIGNMENT SCORING SYSTEMS: Dayhoff, M.O., et al., "A model of evolutionary change in proteins." in Atlas of Protein Sequence and Structure, (1978) vol. 5, suppl. 3. M.O. Dayhoff (ed.), pp. 345-352, Natl. Biomed. Res. Found., Washington, DC; Schwartz, R.M., et al., "Matrices for detecting distant relationships." in Atlas of Protein Sequence and Structure, (1978) vol. 5, suppl. 3." M.O. Dayhoff (ed.), pp. 353-358, Natl. Biomed. Res. Found., Washington, DC; Altschul, S.F., (1991) J. Mol. Biol. 219:555-565; States, D.J., et al., (1991) Methods 3:66-70; Henikoff, S., et al., (1992) Proc. Natl. Acad. Sci. USA 89:10915-10919; Altschul, S.F., et al., (1993) J. Mol. Evol. 36:290-300; ALIGNMENT STATISTICS: Karlin, S., et al., (1990) Proc. Natl. Acad. Sci. USA 87:2264-2268; Karlin, S., et al., (1993) Proc. Natl. Acad. Sci. USA 90:5873-5877; Dembo, A., et al., (1994) Ann. Prob. 22:2022-2039; and Altschul, S.F. "Evaluating the statistical significance of multiple distinct local alignments." in Theoretical and Computational Methods in Genome Research (S. Suhai, ed.), (1997) pp. 1-14, Plenum, New York.

Likewise, either class of light chain can be used in the compositions and methods herein. Specifically, kappa, lambda, or variants thereof are useful in the present compositions and compositions, methods, kits, and uses disclosed herein.

**Table 3. Exemplary PD-1 Antibody Sequences**

| **Antibody Feature** | **Amino Acid Sequence** | **SEQ ID NO.** |
|---|---|---|
| **Pembrolizumab Light Chain** | | |
| CDR1 | RASKGVSTSGYSYLH | 1 |
| CDR2 | LASYLES | 2 |
| CDR3 | QHSRDLPLT | 3 |
| Variable Region | | 4 |
| Light Chain | EIVLTQSPATLSLSPGERATLSCRASKGVSTSGYSYLHWY QQKPGQAPRLLIYLASYLESGVPARFSGSGSGTDFTLTISS LEPEDFAVYYCQHSRDLPLTFGGGTKVEIKRTVAAPSVFI FPPSDEQLKSGTASVVCLLNNFYPREAKVQWKVDNALQ SGNSQESVTEQDSKDSTYSLSSTLTLSKADYEKHKVYAC EVTHQGLSSPVTKSFNRGEC | 5 |

| **Pembrolizumab Heavy Chain** | | |
|---|---|---|
| CDR1 | NYYMY | 6 |
| CDR2 | GINPSNGGTNFNEKFKN | 7 |
| CDR3 | RDYRFDMGFDY | 8 |
| Variable Region | | 9 |
| Heavy Chain | QVQLVQSGVEVKKPGASVKVSCKASGYTFTNYYMYWV RQAPGQGLEWMGGINPSNGGTNFNEKFKNRVTLTTDSST TTAYMELKSLQFDDTAVYYCARRDYRFDMGFDYWGQG TTVTVSSASTKGPSVFPLAPCSRSTSESTAALGCLVKDYF PEPVTVSWNSGALTSGVHTFPAVLQSSGLYSLSSVVTVPS SSLGTKTYTCNVDHKPSNTKVDKRVESKYGPPCPPCPAP EFLGGPSVFLFPPKPKDTLMISRTPEVTCVVVDVSQEDPE VQFNWYVDGVEVHNAKTKPREEQFNSTYRVVSVLTVLH QDWLNGKEYKCKVSNKGLPSSIEKTISKAKGQPREPQVY TLPPSQEEMTKNQVSLTCLVKGFYPSDIAVEWESNGQPE NNYKTTPPVLDSDGSFFLYSRLTVDKSRWQEGNVFSCSV MHEALHNHYTQKSLSLSLGK | 10 |

| **Nivolumab Light Chain** | | |
|---|---|---|
| CDR1 | RASQSVSSYLA | 11 |
| CDR2 | DASNRAT | 12 |
| CDR3 | QQSSNWPRT | 13 |
| Variable Region | | 14 |
| Light Chain | EIVLTQSPATLSLSPGERATLSCRASQSVSSYLAWYQQKPG QAPRLLIYDASNRATGIPARFSGSGSGTDFTLTISSLEPEDF AVYYCQQSSNWPRTFGQGTKVEIKRTVAAPSVFIFPPSDE QLKSGTASVVCLLNNFYPREAKVQWKVDNALQSGNSQE SVTEQDSKDSTYSLSSTLTLSKADYEKHKVYACEVTHQG LSSPVTKSFNRGEC | 15 |

| **Nivolumab Heavy Chain** | | |
|---|---|---|
| CDR1 | NSGMH | 16 |
| CDR2 | VIWYDGSKRYYADSVKG | 17 |
| CDR3 | NDDY | 18 |
| Variable Region | | 19 |
| Heavy Chain | QVQLVESGGGVVQPGRSLRLDCKASGITFSNSGMHWVR QAPGKGLEWVAVIWYDGSKRYYADSVKGRFTISRDNSKN TLFLQMNSLRAEDTAVYYCATNDDYWGQGTLVTVSSAS TKGPSVFPLAPCSRSTSESTAALGCLVKDYFPEPVTVSWN SGALTSGVHTFPAVLQSSGLYSLSSVVTVPSSSLGTKTYTC NVDHKPSNTKVDKRVESKYGPPCPPCPAPEFLGGPSVFLF PPKPKDTLMISRTPEVTCVVVDVSQEDPEVQFNWYVDGV EVHNAKTKPREEQFNSTYRVVSVLTVLHQDWLNGKEYK CKVSNKGLPSSIEKTISKAKGQPREPQVYTLPPSQEEMTK NQVSLTCLVKGFYPSDIAVEWESNGQPENNYKTTPPVLDS DGSFFLYSRLTVDKSRWQEGNVFSCSVMHEALHNHYTQ KSLSLSLGK | 20 |

**Table 4. Additional PD-1 Antibodies and Antigen Binding Fragments Useful in the Methods and Uses disclosed herein.**

| A. Antibodies and antigen binding fragments comprising light and heavy chain CDRs of hPD-1.08A in WO2008/156712 | | |
|---|---|---|
| CDRL1 | RASKSVSTSGFSYLH | SEQ ID NO:21 |
| CDRL2 | LASNLES | SEQ ID NO:22 |
| CDRL3 | QHSWELPLT | SEQ ID NO:23 |
| CDRH1 | SYYLY | SEQ ID NO:24 |
| CDRH2 | GVNPSNGGTNFSEKFKS | SEQ ID NO:25 |
| CDRH3 | RDSNYDGGFDY | SEQ ID NO:26 |

| B. Antibodies and antigen binding fragments comprising the mature h109A heavy chain variable region and one of the mature K09A light chain variable regions in WO 2008/156712 | | |
|---|---|---|
| Heavy chain VR | | SEQ ID NO:27 |
| Light chain VR | | SEQ ID NO:28 or |
| | EIVLTQSPLSLPVTPGEPASISCASKGVSTSGYSYLHWYLQK PGQSPQLLIYLASYLESGVPDRFSGSGSGTDFTLKISRVEAE DVGVYYCHSRDLPLTFGQGTKLEIK | SEQ ID NO:29 or |
| | DIVMTQTPLSLPVTPGEPASISCASKGVSTSGYSYLHWYLQ KPGQSPQLLIYLASYLESGVPDRFSGSGSGTAFTLKISRVEA EDVGLYYC QHSRDLPLTFGQGTKLEIK | SEQ ID NO:30 |

| C. Antibodies and antigen binding fragments comprising the mature 409 heavy chain and one of the mature K09A light chains in WO 2008/156712 | | |
|---|---|---|
| Heavy chain | QVQLVQSGVEVKKPGASVKVSCASGYTFTNYYMWVRQA PGQGLEWMGGINPSNGGTNFNEKFKNRVTLTTDSSTTTAY MELKSLQFDDTAVYYCARRDYRFDMFDYWGQGTTVTVSS ASTKGPSVFPLAPCSRSTSESTAALGCLVKDYFPEPVTVSW NSGALTSGVHTFPAVLQSSGLYSLSSVVTVPSSSLGTKTYTC NVDHKPSNTKVDKRVESKYGPPCPPCPAPEFLGGPSVFLFP PKPKDTLMSRTPEVTCVVVDVSQEDPEVQFNWYVDGVEVH NAKTKPREEQFNSTYRVVSVLTVLHQDWLNGKEYKCKVS NKGLPSSIEKTISKAKGQPREPQVYTLPPSQEEMTKNQVSL TCLVKGFYPSDIAVEWESNGQPENNYKTTPPVLDSDGSFFL YSRLTVDKSRWQEGNVFSCSVMHEALHNHYTQKSLSLSL GK | SEQ ID NO:31 |
| Light chain | EIVLTQSPATLSLSPGERATLSCRASKGVSTSGYSYLHWYQ QKPGQAPRLLIYLASYLESGVPARFSGSGSGTDFTLTISSLE PEDFAVYYCQHSRDLPLTFGGGTKVEIKRTVAAPSVFIFPPS DEQLKSGTASVVCLLNNFYPREAKVQWKVDNALQSGNSQ ESVTEQDSKDSTYSLSSTLTLSKADYEKHKVYACEVTHQG LSSPVTKSFNRGEC | SEQ ID NO:32 or |
| | EIVLTQSPLSLPVTPGEPASISCRASKGVSTSGYSYLHWYLQ KPGQSPQLLIYLASYLESGVPDRFSGSGSGTDFTLKISRVEA EDVGVYYCHSRDLPLTFGQGTKLEIKRTVAAPSVFIFPPSDE QLKSGTASVVCLNNFYPREAKVQWKVDNALQSGNSQESV TEQDSKDSTYSLSSTLTLSKADYEKHKVYACETHQGLSSPV TKSFNRGEC | SEQ ID NO:33 or |
| | DIVMTQTPLSLPVTPGEPASISCRASKGVSTSGYSYLHWYL QKPGQSPQLLIYLASYLESGVPDRFSGSGSGTAFTLKISRVE AEDVGLYYCQHSRDLPLTFGQGTKLEIKRTVAAPSVFIFPPS DEQLKSGTASVVCLLNNFYPREAKVQWKVDNALQSGNSQ ESVTEQDSKDSTYSLSSTLTLSKADYEKHKVYACEVTHQG LSSPVTKSFNRGEC | SEQ ID NO:34 |

In embodiments of the combination therapy, pharmaceutical compositions, methods, kits, and uses disclosed herein, the anti-human PD-1 antibody (or antigen-binding fragment thereof) is an anti-PD-1 monoclonal antibody. In particular aspects of these embodiments, the anti-human PD-1 antibody (or antigen-binding fragment thereof) is selected from the group consisting of nivolumab, pembrolizumab, pidilizumab, and AMP-224. In specific aspects of these embodiments, the anti-human PD-1 antibody (or antigen-binding fragment thereof) is selected from nivolumab and pembrolizumab. In a more specific aspect, the anti-human PD-1 antibody (or antigen-binding fragment thereof) is nivolumab. In a further specific aspect, the anti-human PD-1 antibody (or antigen-binding fragment thereof) is pembrolizumab.

In embodiments of the combination therapy, methods, kits, and uses disclosed herein, the anti-human PD-1 antibody is pembrolizumab. In other such embodiments, the anti-human PD-1 antibody is nivolumab. In other such embodiments, the anti-human PD-1 antibody ispidilizumab. In other such embodiments, the anti-human PD-1 antibody is tislelizumab. In other such embodiments, the anti-human PD-1 antibody is AMP-514. In other such embodiments, the anti-human PD-1 antibody is cemiplimab. In other such embodiments, the anti-human PD-1 antibody is dostarlimab.

### The Immunoconjugate of Formula (I)

Immunoconjugates useful in the present disclosure are those immuoconjgates of Formula (I): 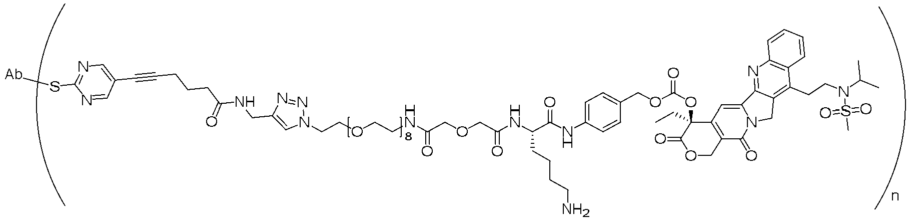
wherein Ab is an antibody that binds to Trop-2; and
and n is an integer from 1 to 10.

In certain embodiments, immunoconjugates useful in the present disclosure are those immuoconjgates of Formula (I): 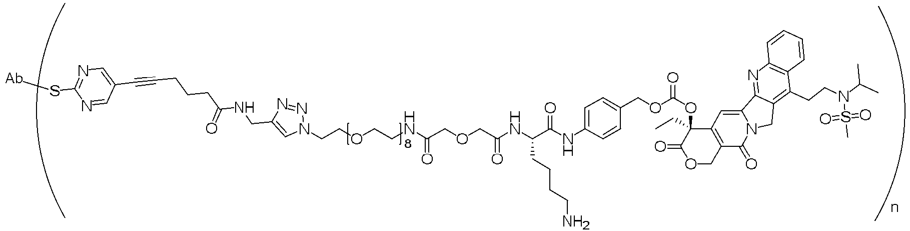
wherein n is an integer from 1 to 10; and
Ab is an antibody that binds to Trop-2 comprising:
   (i) a heavy chain variable region comprising an HCDR1 comprising the amino acid sequence of SEQ ID NO: 39; an HCDR2 comprising the amino acid sequence of SEQ ID NO: 40; and an HCDR3 comprising the amino acid sequence of SEQ ID NO: 41, and
   (ii) a light chain variable region comprising an LCDR1 comprising the amino acid sequence of SEQ ID NO: 42; an LCDR2 comprising the amino acid sequence of SEQ ID NO: 43; and an LCDR3 comprising the amino acid sequence of SEQ ID NO: 44.

In certain embodiments, immunoconjugates useful in the present disclosure are those immuoconjgates of Formula (I): 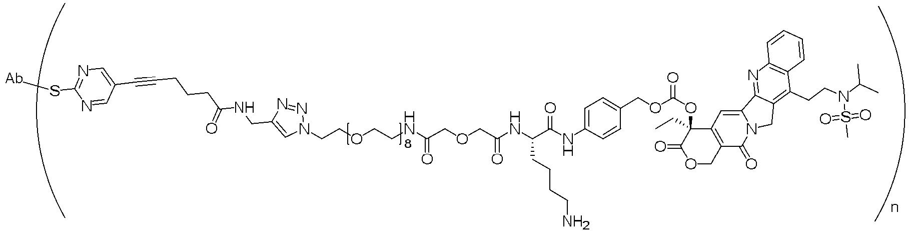
wherein n is an integer from 1 to 10; and
Ab is an antibody that binds to Trop-2 comprising:
   (a) a heavy chain variable region having the amino acid sequence of SEQ ID No.: 37; and (b) a light chain variable region having the amino acid sequence of SEQ ID No.:38.

In certain embodiments, immunoconjugates useful in the present disclosure are those immuoconjgates of Formula (I): 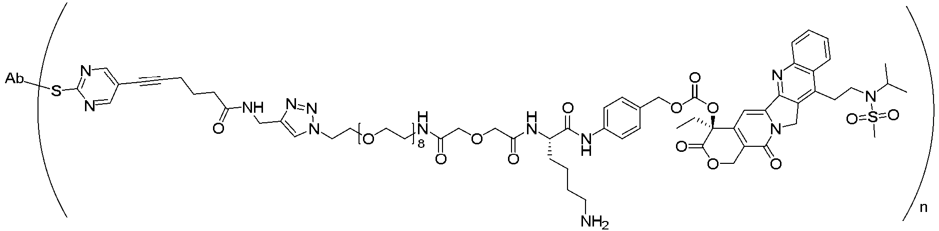
wherein n is integer from 1 to 10; and
Ab is an antibody that binds to Trop-2 comprising:
   (i) a heavy chain having the amino acid sequence of SEQ ID No.: 35; and
   (ii) a light chain having the amino acid sequence of SEQ ID No.:36.

In preferred embodiments, the Immunoconjugate of Formula (I) is Immunoconjugate A, which is described in US Patent Publication No. 20200347075. Immunoconjugate A has the structure: 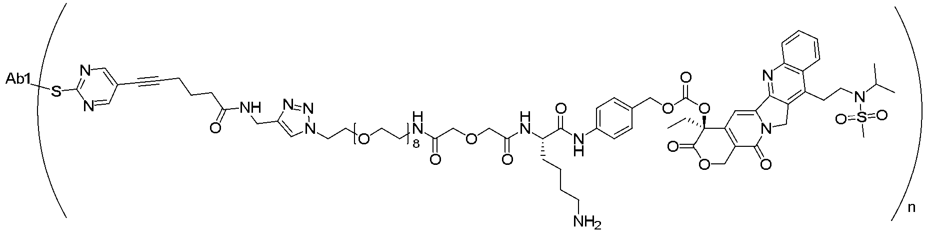
wherein n is an integer from 1 to 8; and
Ab1 is sacituzumab.

In other preferred embodiments, a composition comprising the Immunoconjugates of Formula (I) is administered wherein 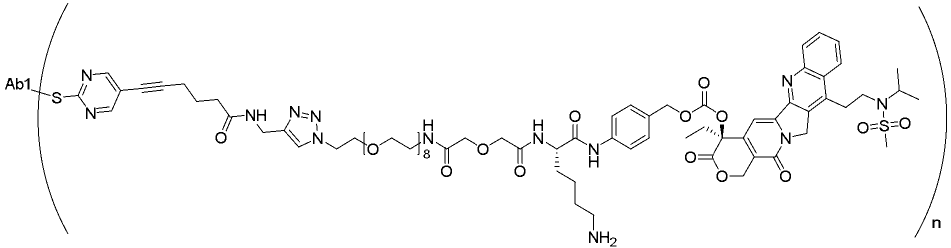
"DAR" of the composition is an integer or decimal from 0 to 8 and
Ab1 is sacituzumab.

In certain embodiments, DAR of the composition is an integer or decimal of 1 to 10, 1 to 9, 1 to 8, 1 to 7, 1 to 6, 1 to 5, 1 to 4, 1 to 3, 1 to 2, 2 to 10, 2 to 9, 2 to 8, 2 to 7, 2 to 6, 2 to 5, 2 to 4, 2 to 3, 3 to 10, 3 to 9, 3 to 8, 3 to 7, 3 to 6, 3 to 5, 3 to 4, 4 to 10, 4 to 9, 4 to 8, 4 to 7, 4 to 6, 4 to 5, 5 to 10, 5 to 9, 5 to 8, 5 to 7, 5 to 6, 6 to 10, 6 to 9, 6 to 8, 6 to 7, 7 to 10, 7 to 9, 7 to 8, 8 to 10, 8 to 9, or 9 to 10. In some embodiments, DAR of the composition is an integer or decimal of 1 to 8, 1 to 4, 2 to 5, 3 to 6, 4 to 7, 5 to 8, or 6 to 8. In some embodiments, the DAR of the composition is an integer or decimal of 1 to 3, 2 to 4, 3 to 5, 4 to 6, 5 to 7, or 6 to 8. In some embodiments, DAR of the composition is an integer or decimal of 1 to 2, 2 to 3, 3 to 4, 4 to 5, 5 to 6, 6 to 7, or 7 to 8. In some embodiments, the DAR of the composition is an integer or decimal of 5 to 8.

In some embodiments, DAR of the composition is 5.1, 5.2, 5.3, 5.4, 5.5, 5.5, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0.

In some embodiments of the present disclosure, the anti-Trop-2 antibody of the Immunoconjugates of Formula (I) is sacituzumab (or hRS7), which is described in US Patent Publication No. 2012/0237518. This antibody can also be obtained by screening through carrier design, construction of an antibody library displaying antibodies as disclosed in CN103476941A, or can be obtained by screening a G-MAB^{®} library of Sorrento Therapeutics, Inc.

Table 5 shows the SEQ ID NOs for the amino acid sequences of the heavy and light chain complementarity-determining regions (HCDRs and LCDRs), heavy and light chain variable domains (VH and VL), and heavy and light chains (HC and LC) of sacituzumab.

**Table 5. List of sacituzumab ("Abl") Amino Acid Sequences**

| **SEQ ID NO.** | **Description** | **Amino Acid Sequence** |
|---|---|---|
| 35 | Ab1 heavy chain | QVQLQQSGSELKKPGASVKVSCKASGYTFTNYGMNWVKQAP GQGLKWMGWINTYTGEPTYTDDFKGRFAFSLDTSVSTAYLQIS SLKADDTAVYFCARGGFGSSYWYFDVWGQGSLVTVSSASTKG PSVFPLAPSSKSTSGGTAALGCLVKDYFPEPVTVSWNSGALTSG VHTFPAVLQSSGLYSLSSVVTVPSSSLGTQTYICNVNHKPSNTK VDKRVEPKSCDKTHTCPPCPAPELLGGPSVFLFPPKPKDTLMIS RTPEVTCVVVDVSHEDPEVKFNWYVDGVEVHNAKTKPREEQ YNSTYRVVSVLTVLHQDWLNGKEYKCKVSNKALPAPIEKTISK AKGQPREPQVYTLPPSREEMTKNQVSLTCLVKGFYPSDIAVEW ESNGQPENNYKTTPPVLDSDGSFFLYSKLTVDKSRWQQGNVFS CSVMHEALHNHYTQKSLSLSPGK |
| 36 | Ab1 light chain | DIQLTQSPSSLSASVGDRVSITCKASQDVSIAVAWYQQKPGKAP KLLIYSASYRYTGVPDRFSGSGSGTDFTLTISSLQPEDFAVYYCQ QHYITPLTFGAGTKVEIKRTVAAPSVFIFPPSDEQLKSGTASVVC LLNNFYPREAKVQWKVDNALQSGNSQESVTEQDSKDSTYSLS STLTLSKADYEKHKVYACEVTHQGLSSPVTKSFNRGEC |
| 37 | Ab1 VH | QVQLQQSGSELKKPGASVKVSCKASGYTFTNYGMNWVKQAP GQGLKWMGWINTYTGEPTYTDDFKGRFAFSLDTSVSTAYLQIS SLKADDTAVYFCARGGFGSSYWYFDVWGQGSLVTVSS |
| 38 | Ab1 VL | DIQLTQSPSSLSASVGDRVSITCKASQDVSIAVAWYQQKPGKAP KLLIYSASYRYTGVPDRFSGSGSGTDFTLTISSLQPEDFAVYYCQ QHYITPLTFGAGTKVEIK |
| 39 | HCDR1 | NYGMN |
| 40 | HCDR2 | WINTYTGEPTYTDDFK |
| 41 | HCDR3 | GGFGSSYWYFDV |
| 42 | LCDR1 | ASQDVSIAVA |
| 43 | LCDR2 | SASYRYT |
| 44 | LCDR3 | QQHYITPLT |

In certain embodiments, the Immunoconjugate of Formula (I) comprises at least 1, 2, 3, 4, 5, 6, 7, 8, 9, or 10 linker/drug moieties per antibody (i.e., n is from 1 to 10). In certain embodiments, the Immunoconjugate of Formula (I) comprises one or more (e.g., 1 to 10, 1 to 9, 1 to 8, 1 to 7, 1 to 6, 1 to 5, 1 to 4, 1 to 3, or 1 to 2) linker/drug moieties.

In a particular embodiment, the Immunoconjugate of Formula (I) comprises from about 6 to about 8 linker/drug moieties per antibody (i.e., n is from about 6 to about 8). In a specific embodiment, the Immunoconjugate of Formula (I) is Immunoconjugate A, and n is from 6 to 8.

The Immunoconjugates of Formula (I) comprise antibodies or fragments thereof that are specific for human Trop-2 and thus can serve as excellent targeting moieties for delivering the conjugated payloads to cells (e.g., Trop-2-positive cells). In certain embodiments, an immunoconjugate used in a treatment regimen of the present disclosure is any anti-Trop-2 immunoconjugate that is described in US Patent Publication No. 20200347075.

### Uses of the Combination Therapies of the Present Disclosure

### Treatment or Prevention of Cellular Proliferation Disorders

The combination therapies disclosed herein are potentially useful in treating diseases or disorders including, but not limited to, cellular proliferative disorders. Cellular proliferative disorders include, but are not limited to, cancers, benign papillomatosis, and gestational trophoblastic diseases. The terms "cancer", "cancerous", or "malignant" refer to or describe the physiological condition in mammals that is typically characterized by unregulated cell growth.

Accordingly, in one aspect, the present disclosure relates to methods of treating a cellular proliferative disorder, said method comprising administering to a patient in need thereof a combination therapy that comprises (a) an anti-human PD-1 antibody (or antigen-binding fragment thereof); and (b) an Immunoconjugate of Formula (I), wherein the amounts administered are together effective to treat a cellular proliferative disorder.

In some embodiments, the present disclosure relates to a method for treating a cell proliferative disorder, the method comprises administering to a patient in need thereof a combination therapy, the combination therapy comprises: (a) an anti-human PD-1 antibody (or antigen-binding fragment thereof), a platinum-containing chemotherapeutic agent, and/or an EGFR-tyrosine kinase inhibitor (EGFR-TKI); and, (b) an immunoconjugate represented by Formula (I), wherein the amounts administered are such that together the aforementioned (a) and (b) components are effective in treating the cell proliferative disorder.

In some embodiments, the present disclosure provides a kit, comprising:
(a) an immunoconjugate represented by Formula (I); and
(b) an anti-human PD-1 antibody or antigen-binding fragment thereof, a platinum-containing chemotherapeutic agent, and/or an EGFR-tyrosine kinase inhibitor (EGFR-TKI).

In some embodiments, the present disclosure provides a kit, comprising:
(a) an immunoconjugate represented by Formula (I); and
(b) an anti-human PD-1 antibody or antigen-binding fragment thereof.

In Embodiment 1 of the kit, the kit further comprises an instruction for administering the Immunoconjugate of Formula (I) and the anti-human PD-1 antibody or antigen-binding fragment thereof to the human patient.

In some embodiments, the present disclosure provides a combination therapy for treating a cell proliferative disorder such as cancer in a human patient, comprising:
(a) an immunoconjugate represented by Formula (I); and
(b) an anti-human PD-1 antibody or antigen-binding fragment thereof for treating cell proliferative disorder, a platinum-containing chemotherapeutic agent, and/or an EGFR-tyrosine kinase inhibitor (EGFR-TKI).

In some embodiments, the present disclosure provides a combination therapy for treating a cell proliferative disorder such as cancer in a human patient, comprising:
(a) an immunoconjugate represented by Formula (I); and
(b) an anti-human PD-1 antibody or antigen-binding fragment thereof for treating cell proliferative disorder.

In some embodiments, the present disclosure provides a pharmaceutical combination for treating a cell proliferative disorder such as cancer in a human patient, the pharmaceutical combination comprises:
(a) an immunoconjugate represented by Formula (I); and
(b) an anti-human PD-1 antibody or antigen-binding fragment thereof for treating cell proliferative disorder, a platinum-containing chemotherapeutic agent, and/or an EGFR-tyrosine kinase inhibitor (EGFR-TKI).

In some embodiments, the present disclosure provides a pharmaceutical combination for treating a cell proliferative disorder such as cancer in a human patient, the pharmaceutical combination comprises:
(a) an immunoconjugate represented by Formula (I); and
(b) an anti-human PD-1 antibody or antigen-binding fragment thereof for treating cell proliferative disorder.

In some embodiments, the present disclosure provides a use of a pharmaceutical combination in the manufacture of a medicament for treating a cell proliferative disorder such as cancer in a human patient, the pharmaceutical combination comprises:
(a) an immunoconjugate represented by Formula (I); and
(b) an anti-human PD-1 antibody or antigen-binding fragment thereof for treating cell proliferative disorder, a platinum-containing chemotherapeutic agent, and/or an EGFR-tyrosine kinase inhibitor (EGFR-TKI).

In some embodiments, the present disclosure provides a use of a pharmaceutical combination in the manufacture of a medicament for treating a cell proliferative disorder such as cancer in a human patient, the pharmaceutical combination comprises:
(a) an immunoconjugate represented by Formula (I); and
(b) an anti-human PD-1 antibody or antigen-binding fragment thereof.

In some embodiments, the pharmaceutical combination or kit comprises:
(a) an immunoconjugate represented by Formula (I); and
(b) an anti-human PD-1 antibody or antigen-binding fragment thereof.

In some embodiments, the pharmaceutical combination or kit comprises:
(a) an immunoconjugate represented by Formula (I);
(b) an anti-human PD-1 antibody or antigen-binding fragment thereof; and
(c) a platinum-containing chemotherapeutic agent.

In some embodiments, the pharmaceutical combination or kit comprises:
(a) an immunoconjugate represented by Formula (I); and
(b) a platinum-containing chemotherapeutic agent.

In some embodiments, the pharmaceutical combination or kit comprises:
(a) an immunoconjugate represented by Formula (I); and
(b) an EGFR-tyrosine kinase inhibitor (EGFR-TKI).

In some embodiments, the platinum-containing chemotherapeutic agent is carboplatin or cisplatin; preferably carboplatin.

In some embodiments, the EGFR-tyrosine kinase inhibitor is osimertinib.

In some embodiments, the cell proliferative disorder is non-small cell lung cancer.

In some embodiments, the non-small cell lung cancer is EGFR wild-type; in some embodiments, the non-small cell lung cancer is EGFR-mutated NSCLC.

In some embodiments, the non-small cell lung cancer is ALK fusion gene-negative non-small cell lung cancer. In some embodiments, the non-small cell lung cancer is ALK fusion gene-positive non-small cell lung cancer.

In some embodiments, the non-small cell lung cancer is squamous cell carcinoma; in some embodiments, the non-small cell lung cancer is non-squamous cell carcinoma.

In some embodiments, the PD-L1 expression level in the non-small cell lung cancer is elevated, for example, with a tumor proportion score (TPS) ≥ 1%, a TPS of 1% to 49%, or a TPS ≥ 50%.

In some embodiments, the PD-L1 expression level in the non-small cell lung cancer is not elevated, for example, with a tumor proportion score (TPS) < 1%.

In some embodiments, the non-small cell lung cancer carries a 19 del mutation and/or a 21 L858R mutation.

In some embodiments, the non-small cell lung cancer has central nervous system metastasis; in some embodiments, the non-small cell lung cancer does not have central nervous system metastasis.

In some embodiments, the non-small cell lung cancer has not received any treatment, or has received a first-line treatment, or has received a second-line treatment or more, or has received a third-line treatment or more.

In some embodiments, the non-small cell lung cancer has been previously treated with only a first-generation EGFR TKI and has not been treated with a second- or third-generation EGFR TKI.

In some embodiments, the non-small cell lung cancer has been previously treated with only a first- or second-generation EGFR TKI and has not been treated with a third-generation EGFR TKI.

In some embodiments, the non-small cell lung cancer has been previously treated with a third-generation EGFR TKI.

In some embodiments, the non-small cell lung cancer is an EGFR wild-type and ALK fusion gene-negative non-small cell lung cancer.

In some embodiments, the non-small cell lung cancer is EGFR mutant-type and has failed prior EGFR-TKI treatment.

In some embodiments, the non-small cell lung cancer is an EGFR wild-type squamous cell carcinoma.

In some embodiments, the non-small cell lung cancer is an EGFR wild-type non-squamous cell carcinoma.

In some embodiments, the non-small cell lung cancer is EGFR wild-type with a TPS of < 1%.

In some embodiments, the non-small cell lung cancer is EGFR wild type with a TPS of 1% to 49%.

In some embodiments, the non-small cell lung cancer is EGFR wild-type with a TPS of ≥ 50%.

In some embodiments, the non-small cell lung cancer is EGFR-mutant and has failed prior treatment with a first- or second-generation EGFR TKI.

In some embodiments, the non-small cell lung cancer is EGFR-mutant and has failed prior treatment with a third-generation EGFR TKI.

In some embodiments, the non-small cell lung cancer is EGFR-mutant and has central nervous system metastasis.

In some embodiments, the non-small cell lung cancer is EGFR-mutant and has no central nervous system metastasis.

In specific embodiments, the cellular proliferative disorder is selected from cancer, benign papillomatosis, benign neoplastic diseases and gestational trophoblastic diseases. In particular embodiments, the gestational trophoblastic disease is selected from the group consisting of hydatidiform moles, and gestational trophoblastic neoplasia (*e.g.,* invasive moles, choriocarcinomas, placental-site trophoblastic tumors, and epithelioid trophoblastic tumors).

In a particular embodiment, the cellular proliferative disorder being treated is cancer. Accordingly, in one embodiment, provided herein are methods for treating cancer in a patient, the method comprising administering to the patient a combination therapy that comprises (a) an anti-human PD-1 antibody (or antigen-binding fragment thereof); and (b) an Immunoconjugate of Formula (I), wherein the amounts administered are together effective to treat cancer.

In a specific embodiment, the amount administered is effective to treat cancer in the human patient. In another specific embodiment, the amount administered is effective to inhibit cancer cell replication or cancer cell metastasis in the patient.

In one embodiment, the cancer is metastatic. In another embodiment, the cancer is relapsed. In another embodiment, the cancer is refractory. In yet another embodiment, the cancer is relapsed and refractory.

In one embodiment, the patient has previously received treatment for cancer. In another embodiment, the patient has not previously received treatment for cancer.

In one embodiment, the patient has previously received systemic treatment for cancer. In another embodiment, the patient has not previously received systemic treatment for cancer.

In other embodiments, the cancer is present in an adult patient; in additional embodiments, the cancer is present in a pediatric patient.

The compositions, methods, kits, and uses provided herein are useful for the treatment of cancer. Cancers that may be treated using the compositions, methods, kits, and uses disclosed herein include, but are not limited to: (1) Cardiac: sarcoma (angiosarcoma, fibrosarcoma, rhabdomyosarcoma, liposarcoma), myxoma, rhabdomyoma, fibroma, lipoma and teratoma; (2) Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma, non-small cell; (3) Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma), colon, colorectal, rectal; (4) Genitourinary tract: kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma), and urothelial cancer; (5) Liver: hepatoma (hepatocellular carcinoma), cholangiocarcinoma, hepatoblastoma, angiosarcoma, hepatocellular adenoma, hemangioma; (6) Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; (7) Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiforme, oligodendroglioma, schwannoma, retinoblastoma, congenital tumors), spinal cord neurofibroma, meningioma, glioma, sarcoma); (8) Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma), breast; (9) Hematologic: blood (myeloid leukemia [acute and chronic], acute lymphoblastic leukemia, chronic lymphocytic leukemia, chronic myelomonocytic (CMML), myelocellular proliferative disorders, multiple myeloma, myelodysplastic syndrome), Hodgkin's disease, non-Hodgkin's lymphoma [malignant lymphoma]; (10) Skin: malignant melanoma, basal cell carcinoma, squamous cell carcinoma, Karposi's sarcoma, moles dysplastic nevi, lipoma, angioma, dermatofibroma, keloids, psoriasis; and (11) Adrenal glands: neuroblastoma. Examples of cancer that may be treated using the compounds, compositions and methods described herein include thyroid cancer, anaplastic thyroid carcinoma, epidermal cancer, head and neck cancer (e.g., squamous cell cancer of the head and neck), sarcoma, tetracarcinoma, hepatoma and multiple myeloma.

The term "cancerous cell" as used herein, includes a cell afflicted by any one of the above-identified conditions.

In a particular embodiment, the cancer being treated is selected from: non-small cell lung cancer, prostate cancer, ovarian cancer, cervical cancer, or urothelial cancer. In a particular embodiment, the cancer is selected from: non-small cell lung cancer, recurrent cervical cancer, metastatic cervical cancer, locally advanced urothelial cancer, metastatic urothelial cancer, platinum-sensitive recurrent ovarian cancer, or metastatic castration-resistant prostate cancer.

In one embodiment, provided herein is a method of treating unresectable or metastatic melanoma in a human patient. In some embodiments, the method comprises treating resected high-risk stage III melanoma.

In specific embodiments, the cancer is selected from lung cancers. In particular embodiments, the lung cancer is selected from the group consisting of non-small cell lung cancer, bronchial tumors, and pleuropulmonary blastomas.

In one embodiment, provided herein is a method of treating metastatic non-small cell lung cancer (NSCLC) in a human patient. In some embodiments, the NSCLC is non-squamous. In other embodiments, the NSCLC is squamous. In certain embodiments of the method for treating NSCLC, the patient's tumor has no EGFR or ALK genomic aberrations. In certain embodiments of the method for treating NSCLC, the patient's tumor has an EGFR or ALK genomic aberration and had disease progression on or after receiving treatment for the EGFR or ALK aberration(s) prior to receiving combination therapy described herein. In a specific embodiment, the NSCLC being treated is without EGFR mutation, and ALK fusion genes. In a specific embodiment, the NSCLC being treated has an EGFR actionable mutation, and the patient has failed prior EGFR-TKI treatment. In another specific embodiment, the non-small cell lung cancer has a 19 del mutation and/or has a 21 L858R mutation. In another specific embodiment, the non-small cell lung cancer is a non-small cell lung cancer with an EGFR actionable mutation, and the patient has failed prior EGFR-TKI treatment and has failed prior PD-1/PD-L1 antibody treatment and chemotherapy.

In specific embodiments, the cancer is selected from malignant mesothelioma. In particular embodiments, the cancer is selected from the group consisting of epithelial mesothelioma and sarcomatoids.

In one embodiment, provided herein is a method of treating refractory classical Hodgkin lymphoma (cHL) in a human patient. In certain embodiments, the patient has relapsed after 1, 2, 3 or more lines of therapy for cHL. In specific embodiments, the patient is an adult patient. In alternative embodiments the patient is a pediatric patient.

In one embodiment, provided herein is a method of urothelial cancer in a human patient. In certain embodiment, the urothelial cancer is locally advanced or metastatic urothelial cancer. In certain embodiments, the patient is not eligible for cisplatin-containing chemotherapy. In further embodiments, the patient has disease progression during or following platinum-containing chemotherapy or within 12 months of neoadjuvant or adjuvant treatment with platinum-containing chemotherapy. In specific embodiments, the patient's tumor expresses PD-L1 (CPS ≥10).

In one embodiment, provided herein is a method of treating unresectable or metastatic, micro satellite instability-high (MSI-H) or mismatch repair deficient solid tumors in a human patient. In specific embodiments, the patient had disease progression following prior anti-cancer treatment.

In one embodiment, provided herein is a method of treating unresectable or metastatic, micro satellite instability-high (MSI-H) or mismatch repair deficient colorectal cancer in a human patient. In specific embodiments, the patient had disease progression following prior treatment with a fluoropyrimidine, oxaliplatin, and irinotecan.

In one embodiment, provided herein is a method of treating recurrent locally advanced or metastatic gastric cancer or recurrent locally advanced or metastatic gastroesophageal junction adenocarcinoma in a human patient. In specific embodiments, the patient's tumor expresses PD-L1 [Combined Positive Score (CPS) ≥1]. In some embodiments, the patient has disease progression on or after two or more prior lines of therapy including fluoropyrimidine- and platinum-containing chemotherapy. In some embodiments, the patient has disease progression on or after two or more prior lines of therapy including HER2/neu-targeted therapy.

In specific embodiments, the cancer is selected from lymphomas. In particular embodiments, the cancer is selected from the group consisting of Hodgkin's lymphoma (*e.g.,* classical Hodgkin refractory lymphoma), non-Hodgkin's lymphomas, and cutaneous T-cell lymphomas.

In one embodiment, provided herein is a method of treating non-Hodgkin lymphoma (NHL) in a human patient. In one embodiment, the NHL is low-grade lymphoma. In another embodiment the NHL is high-grade lymphoma. In certain embodiments, the NHL is selected from primary mediastinal large B-cell lymphoma, diffuse large b-cell lymphoma (DLBCL), lymphoplasmacytic lymphoma, mycosis fungoides, small lymphocytic lymphoma, mediastinal large B-cell lymphoma, splenic marginal zone B-cell lymphoma, extranodal marginal zone B-cell lymphoma of mucosa-associated lymphoid tissue (malt), nodal marginal zone B-cell lymphoma, lymphoplasmacytic lymphoma, primary effusion lymphoma, Sezary syndrome, primary central nervous system lymphomas, mantle cell lymphoma, follicular lymphoma, marginal zone lymphoma, anaplastic large cell lymphoma (primary cutaneous type), anaplastic large cell lymphoma (systemic type), peripheral T-cell lymphoma, angioimmunoblastic T-cell lymphoma, adult T-cell lymphoma/leukemia, nasal type extranodal NK/T-cell lymphoma, enteropathy-associated T-cell lymphoma, gamma/delta hepatosplenic T-cell lymphoma, subcutaneous panniculitis-like T-cell and Burkitt lymphoma.

In one embodiment, provided herein is a method of treating cancer in a human patient comprising, wherein the patient has a tumor with a high mutational burden.

In specific embodiments, the cancer is selected from brain and spinal cancers. In particular embodiments, the brain and spinal cancer is selected from the group consisting of anaplastic astrocytomas, glioblastomas, astrocytomas, and estheosioneuroblastomas (also known as olfactory blastomas). In particular embodiments, the brain cancer is selected from the group consisting of astrocytic tumor (*e.g.,* pilocytic astrocytoma, subependymal giant-cell astrocytoma, diffuse astrocytoma, pleomorphic xanthoastrocytoma, anaplastic astrocytoma, astrocytoma, giant cell glioblastoma, glioblastoma, secondary glioblastoma, primary adult glioblastoma, and primary pediatric glioblastoma), oligodendroglial tumor (*e.g.,* oligodendroglioma, and anaplastic oligodendroglioma), oligoastrocytic tumor (*e.g.,* oligo astrocytoma, and anaplastic oligoastrocytoma), ependymoma (*e.g.,* myxopapillary ependymoma, and anaplastic ependymoma); medulloblastoma, primitive neuroectodermal tumor, schwannoma, meningioma, atypical meningioma, anaplastic meningioma, pituitary adenoma, brain stem glioma, cerebellar astrocytoma, cerebral astorcytoma/malignant glioma, visual pathway and hypothalmic glioma, and primary central nervous system lymphoma. In specific instances of these embodiments, the brain cancer is selected from the group consisting of glioma, glioblastoma multiforme, paraganglioma, and suprantentorial primordial neuroectodermal tumors (sPNET). In one embodiment, the brain or spinal cancer is a metastatic brain tumor or tumors.

In specific embodiments, the cancer is selected from cancers of the head and neck, including recurrent or metastatic head and neck squamous cell carcinoma (HNSCC), nasopharyngeal cancers, nasal cavity and paranasal sinus cancers, hypopharyngeal cancers, oral cavity cancers (*e.g.,* squamous cell carcinomas, lymphomas, and sarcomas), lip cancers, oropharyngeal cancers, salivary gland tumors, cancers of the larynx (*e.g.,* laryngeal squamous cell carcinomas, rhabdomyosarcomas), and cancers of the eye or ocular cancers. In particular embodiments, the ocular cancer is selected from the group consisting of intraocular melanoma and retinoblastoma. In one embodiment, provided herein is a method of treating recurrent or metastatic head and neck squamous cell cancer (HNSCC) in a human patient. In some embodiments, the patient was previously treated with platinum-containing chemotherapy. In certain embodiments, the patient had disease progression during or after platinum-containing chemotherapy.

In specific embodiments, the cancer is selected from leukemia and cancers of the blood. In particular embodiments, the cancer is selected from the group consisting of myeloproliferative neoplasms, myelodysplastic syndromes, myelodysplastic/ myeloproliferative neoplasms, acute myeloid leukemia (AML), myelodysplastic syndrome (MDS), chronic myelogenous leukemia (CML), myeloproliferative neoplasm (MPN), post-MPN AML, post-MDS AML, del(5q)-associated high risk MDS or AML, blast-phase chronic myelogenous leukemia, angioimmunoblastic lymphoma, acute lymphoblastic leukemia, Langerans cell histiocytosis, hairy cell leukemia, and plasma cell neoplasms including plasmacytomas and multiple myelomas. Leukemias referenced herein may be acute or chronic.

In specific embodiments, the cancer is selected from skin cancers. In particular embodiments, the skin cancer is selected from the group consisting of melanoma, squamous cell cancers, and basal cell cancers. In specific embodiments, the skin cancer is unresectable or metastatic melanoma.

In specific embodiments, the cancer is selected from cancers of the reproductive system. In particular embodiments, the cancer is selected from the group consisting of breast cancers, cervical cancers, vaginal cancers, ovarian cancers, endometrial cancers, prostate cancers, penile cancers, and testicular cancers. In specific instances of these embodiments, the cancer is a breast cancer selected from the group consisting of ductal carcinomas and phyllodes tumors. In specific instances of these embodiments, the breast cancer may be male breast cancer or female breast cancer. In some instances of these embodiments, the breast cancer is triple-negative breast cancer. In other instances, the breast cancer is ER+/HER2- breast cancer. In specific instances of these embodiments, the cancer is a cervical cancer selected from the group consisting of squamous cell] carcinomas and adenocarcinomas.

In specific embodiments, the cancer is selected from cancers of the gastrointestinal system. In particular embodiments, the cancer is selected from the group consisting of esophageal cancers, gastric cancers (also known as stomach cancers), gastrointestinal carcinoid tumors, pancreatic cancers, gall bladder cancers, colorectal cancers, and anal cancer. In instances of these embodiments, the cancer is selected from the group consisting of esophageal squamous cell carcinomas, esophageal adenocarcinomas, gastric adenocarcinomas, gastrointestinal carcinoid tumors, gastrointestinal stromal tumors, gastric lymphomas, gastrointestinal lymphomas, solid pseudopapillary tumors of the pancreas, pancreatoblastoma, islet cell tumors, pancreatic carcinomas including acinar cell carcinomas and ductal adenocarcinomas, gall bladder adenocarcinomas, colorectal adenocarcinomas, microsatellite stable colorectal cancer, advanced microsatellite stable colorectal cancer, metastatic microsatellite stable colorectal cancer and anal squamous cell carcinomas.

In specific embodiments, the cancer is selected from liver and bile duct cancers. In particular embodiments, the cancer is liver cancer (also known as hepatocellular carcinoma). In particular embodiments, the cancer is bile duct cancer (also known as cholangiocarcinoma); in instances of these embodiments, the bile duct cancer is selected from the group consisting of intrahepatic cholangiocarcinoma and extrahepatic cholangiocarcinoma.

In specific embodiments, the cancer is selected from kidney and bladder cancers. In particular embodiments, the cancer is a kidney cancer selected from the group consisting of renal cell cancer, Wilms tumors, and transitional cell cancers. In particular embodiments, the cancer is a bladder cancer selected from the group consisting of urothelial carcinoma (a transitional cell carcinoma), squamous cell carcinomas, and adenocarcinomas.

In specific embodiments, the cancer is selected from bone cancers. In particular embodiments, the bone cancer is selected from the group consisting of osteosarcoma, malignant fibrous histiocytoma of bone, Ewing sarcoma, chordoma (cancer of the bone along the spine).

In specific embodiments, the cancer is selected from sarcomas. In particular embodiments, the sarcoma is selected from the group consisting of central chondrosarcoma, central and periosteal chondroma, fibrosarcoma, clear cell sarcoma of tendon sheaths, and Kaposi's sarcoma.

In specific embodiments, the cancer is selected from glandular cancers. In particular embodiments, the cancer is selected from the group consisting of adrenocortical cancer (also known as adrenocortical carcinoma or adrenal cortical carcinoma), pheochromocytomas, paragangliomas, pituitary tumors, thymoma, and thymic carcinomas.

In specific embodiments, the cancer is selected from thyroid cancers. In particular embodiments, the thyroid cancer is selected from the group consisting of medullary thyroid carcinomas, papillary thyroid carcinomas, and follicular thyroid carcinomas.

In specific embodiments, the cancer is selected from germ cell tumors. In particular embodiments, the cancer is selected from the group consisting of malignant extracranial germ cell tumors and malignant extragonadal germ cell tumors. In specific instances of these embodiments, the malignant extragonadal germ cell tumors are selected from the group consisting of nonseminomas and seminomas.

In specific embodiments, the cancer is selected from heart tumors. In particular embodiments, the heart tumor is selected from the group consisting of malignant teratoma, lymphoma, rhabdomyosacroma, angiosarcoma, chondrosarcoma, infantile fibrosarcoma, and synovial sarcoma.

In embodiments, the cancer is a metastatic tumor, for example, liver metastases from colorectal cancer or pancreatic cancer; and brain metastases from lung or breast cancer.

In some embodiments, the cancer exhibits high PD-L1 expression [(Tumor Proportion Score (TPS) ≥50%)] and was not previously treated with platinum-containing chemotherapy. In alternative embodiments, the patient has a tumor with PD-L1 expression (TPS ≥1%), and was previously treated with platinum-containing chemotherapy. In specific embodiments, the patient had disease progression on or after receiving platinum-containing chemotherapy.

In certain embodiments the PD-L1 TPS is determined by an FDA-approved test.

In particular embodiments, the cancer is classified as stage III cancer or stage IV cancer. In some instances of these embodiments, the cancer is not surgically resectable.

### ADMINISTRATION AND DOSAGE

The present disclosure relates to methods of treating a cellular proliferative disorder, said method comprising administering to a patient in need thereof a combination therapy that comprises: (a) an anti-human PD-1 antibody (or antigen-binding fragment thereof), a platinum-containing chemotherapeutic agent, and/or an EGFR-tyrosine kinase inhibitor (EGFR-TKI); and (b) an Immunoconjugate of Formula (I), wherein the amounts administered are together effective to treat or prevent the cellular proliferative disorder.

The present disclosure relates to a method for treating a cell proliferative disorder, the method comprises administering to a patient in need thereof a combination therapy, the combination therapy comprising: (a) an anti-human PD-1 antibody (or antigen-binding fragment thereof); and (b) an immunoconjugate represented by Formula (I), wherein the amounts administered are effective together for treating or preventing the cell proliferative disorder.

The Immunoconjugates of Formula (I), and the anti-human PD-1 antibody (or antigen-binding fragment thereof), will each (with a pharmaceutically acceptable carrier or excipient(s)), typically be formulated into a dosage form adapted for administration to a patient by a desired route of administration. For example, dosage forms include those adapted for (1) oral administration, such as tablets, capsules, caplets, pills, troches, powders, syrups, elixirs, suspensions, solutions, emulsions, sachets, and cachets; and (2) parenteral administration, such as sterile solutions, suspensions, and powders for reconstitution. Suitable pharmaceutically acceptable carriers or excipients will vary depending upon the particular dosage form chosen. In addition, suitable pharmaceutically acceptable carriers or excipients may be chosen for a particular function that they may serve in the composition. In embodiments, the Immunoconjugate of Formula (I), and/or the anti-human PD-1 antibody (or antigen-binding fragment thereof) may be formulated into a dosage form that allows for systemic use, *i.e.,* distribution throughout the body of the patient; examples of such systemic administration include oral administration, intravenous administration, and subcutaneous administration In additional embodiments, the Immunoconjugate of Formula (I), the anti-human PD-1 antibody (or antigen-binding fragment thereof), the platinum-containing chemotherapeutic agent, and/or the EGFR-tyrosine kinase inhibitor (EGFR-TKI) may be formulated into a dosage form that allows for targeted or isolated use, *i.e.,* administration of the Immunoconjugate of Formula (I), and/or the anti-human PD-1 antibody (or antigen-binding fragment thereof) only to the portion of the patient's body to be treated; examples of such targetted administration include intratumoral injection.

In some embodiments, at least one of the therapeutic agents (the anti-PD-1 antibody, or binding fragment thereof, and the Immunoconjugate of Formula (I)) in the combination therapy is administered using the same dosage regimen (dose, frequency, and duration of treatment) that is typically employed when the agent is used as monotherapy for treating the same condition. In other embodiments, the patient receives a lower total amount of at least one of the therapeutic agents in the combination therapy than when the agent is used as monotherapy, *e.g*., smaller doses, less frequent doses, and/or shorter treatment duration.

In embodiments of the compositions, methods, kits, and uses disclosed herein, the anti-human PD-1 antibody (or antigen binding fragment thereof) is administered by intravenous infusion or subcutaneous injection, and the Immunoconjugate of Formula (I) is administered by intravenous infusion or subcutaneous injection. In specific embodiments, the human PD-1 antibody (or antigen binding fragment thereof) the Immunoconjugate of Formula (I) are each administered by intravenous infusion.

When administering a combination therapy of the present disclosure to a patient, the agents comprising the combination may be administered in any order such as, for example, sequentially, concurrently, together, simultaneously and the like. The amounts of the various agents in such combination therapy may be different amounts (different dosage amounts) or same amounts (same dosage amounts). Thus, for non-limiting illustration purposes, an Immunoconjugate of Formula (I), and an anti-human PD-1 antibody (or antigen-binding fragment thereof) may be present in fixed amounts (dosage amounts) in a single dosage unit.

In one embodiment, the Immunoconjugate of Formula (I) is administered during a time when the anti-human PD-1 antibody (or antigen-binding fragment thereof) exerts its prophylactic or therapeutic effect, or *vice versa.*

In another embodiment, the Immunoconjugate of Formula (I), and the anti-human PD-1 antibody (or antigen-binding fragment thereof) are administered in doses commonly employed when such agents are used as monotherapy for treating cancer.

In another embodiment, the Immunoconjugate of Formula (I), and the anti-human PD-1 antibody (or antigen-binding fragment thereof) are administered in doses lower than the doses commonly employed when such agents are used as monotherapy for treating cancer.

In still another embodiment, the Immunoconjugate of Formula (I), and the anti-human PD-1 antibody (or antigen-binding fragment thereof) act synergistically and are administered in doses lower than the doses commonly employed when such agents are used as monotherapy for treating cancer.

The Immunoconjugate of Formula (I), and the anti-human PD-1 antibody (or antigen-binding fragment thereof) can act additively or synergistically. A synergistic combination may allow the use of lower dosages of one or both of these agents, and/or less frequent administration of one or both of these agents. A lower dosage or less frequent administration of these agents may lower the toxicity of therapy without reducing the efficacy of therapy.

In one embodiment, the administration of Immunoconjugate of Formula (I), and the anti-human PD-1 antibody (or antigen-binding fragment thereof) may inhibit the resistance of cancer to either or both of these agents.

In some embodiments, the combination therapies of the present disclosure are administered for a period until the patient shows no symptoms of the diseases or disorders. In some embodiments, the combination therapies of the present disclosure are administered for a period until the patient is cured of the diseases or disorders. In some embodiments, the combination therapies of the present disclosure are administered for a period until the patient shows resistance to the therapy. In some embodiments, the combination therapies of the present disclosure are administered for a period until the patient shows side effects that would require discontinuation of the therapy.

In some embodiments, the patient is fasting for at least 1 hour, 2 hours, 3 hours, 4 hours, 6 hours, 8 hours, 12 hours before one or more components of a combination therapy is administered. In some embodiments, the patient needs to fast for at least 1 hour, 2 hours, 3 hours, 4 hours, 6 hours, 8 hours, 12 hours after one or more components of a combination therapy is administered.

A combination therapy of the present disclosure may be used prior to or following surgery to remove a tumor and may be used prior to, during, or after radiation treatment.

In some embodiments, a combination therapy of the present disclosure is administered to a patient who has not previously been treated with a biotherapeutic or chemotherapeutic agent, *i.e.,* is treatmentnaive. In other embodiments, the combination therapy is administered to a patient who failed to achieve a sustained response after prior therapy with the biotherapeutic or chemotherapeutic agent, *i.e.,* is treatment-experienced.

Products provided as therapeutic combinations may include a composition comprising an anti-human PD-1 antibody (or antigen-binding fragment thereof), and a composition comprising an Immunoconjugate of Formula (I) in separate form, *e.g.,* in the form of a kit or in any form designed to enable separate administration either concurrently or on separate dosing schedules.

In some embodiments, the anti-human PD-1 antibody or antigen-binding fragment thereof is administered to the patient at a dose of 50 mg to 500 mg, 50 mg to 450 mg, 50 mg to 400 mg, 50 mg to 350 mg, 50 mg to 300 mg, 50 mg to 250 mg, 50 mg to 200 mg, 50 mg to 150 mg, 50 mg to 100 mg, 100 mg to 500 mg, 100 mg to 450 mg, 100 mg to 400 mg, 100 mg to 350 mg, 100 mg to 300 mg, 100 mg to 250 mg, 100 mg to 200 mg, 100 mg to 150 mg, 150 mg to 500 mg mg, 150 mg to 450 mg, 150 mg to 400 mg, 150 mg to 350 mg, 150 mg to 300 mg, 150 mg to 250 mg, 150 mg to 200 mg, 200 mg to 500 mg, 200 mg to 450 mg, 200 mg to 400 mg, 200 mg to 350 mg, 200 mg to 300 mg, 200 mg to 250 mg, 250 mg to 500 mg, 250 mg to 450 mg, 250 mg to 400 mg, 250 mg to 350 mg, 250 mg to 300 mg, 300 mg to 500 mg, 300 mg to 450 mg, 300 mg to 400 mg, 300 mg to 350 mg, 350 mg to 500 mg, 200 mg to 400 mg, 350 mg to 450 mg, 350 mg to 400 mg, 400 mg to 500 mg, 400 mg to 450 mg, or 450 mg to 500 mg. In some embodiments, the anti-human PD-1 antibody or antigen-binding fragment thereof is administered to the patient at a dose of 200 mg to 400 mg.

In some embodiments, the anti-PD-1 antibody (e.g., anti-PD-1 monoclonal antibody) or antigen binding fragment thereof is administered subcutaneously or intravenously, on a weekly, biweekly, triweekly, every 4 weeks, every 5 weeks, every 6 weeks, monthly, bimonthly, or quarterly basis at about 10, about 20, about 50, about 80, about 100, about 200, about 300, about 400, about 500, about 1000 or about 2500 mg/patient.

In some specific methods, the dose of the anti-PD-1 antibody (e.g., anti-PD-1 monoclonal antibody) or antigen binding fragment thereof is from about 0.01 mg/kg to about 50 mg/kg, from about 0.05 mg/kg to about 25 mg/kg, from about 0.1 mg/kg to about 10 mg/kg, from about 0.2 mg/kg to about 9 mg/kg, from about 0.3 mg/kg to about 8 mg/kg, from about 0.4 mg/kg to about 7 mg/kg, from about 0.5 mg/kg to about 6 mg/kg, from about 0.6 mg/kg to about 5 mg/kg, from about 0.7 mg/kg to about 4 mg/kg, from about 0.8 mg/kg to about 3 mg/kg, from about 0.9 mg/kg to about 2 mg/kg, from about 1.0 mg/kg to about 1.5 mg/kg, from about 1.0 mg/kg to about 2.0 mg/kg, from about 1.0 mg/kg to about 3.0 mg/kg, or from about 2.0 mg/kg to about 4.0 mg/kg.

In some specific methods, the dose of the anti-PD-1 antibody (e.g., anti-PD-1 monoclonal antibody) or antigen binding fragment thereof is from about 10 mg to about 500 mg, from about 25 mg to about 500 mg, from about 50 mg to about 500 mg, from about 100 mg to about 500 mg, from about 200 mg to about 500 mg, from about 150 mg to about 250 mg, from about 175 mg to about 250 mg, from about 200 mg to about 250 mg, from about 150 mg to about 240 mg, from about 175 mg to about 240 mg, or from about 200 mg to about 240 mg. In some embodiments, the dose of the anti-PD-1 antibody (e.g., anti-PD-1 monoclonal antibody) or antigen binding fragment thereof is about 50 mg, about 75 mg, about 100 mg, about 125 mg, about 150 mg, about 175 mg, about 200 mg, about 225 mg, about 240 mg, about 250 mg, about 300 mg, about 400 mg, or about 500 mg.

In another embodiment, the PD-1 antagonist in the therapy is pembrolizumab, or a pembrolizumab variant, which is administered in a liquid medicament at a dose selected from the group consisting of 1 mg/kg Q2W, 2 mg/kg Q2W, 3 mg/kg Q2W, 5 mg/kg Q2W, 10 mg/kg Q2W, 1 mg/kg Q3W, 2 mg/kg Q3W, 3 mg/kg Q3W, 5 mg/kg Q3W, or 10 mg/kg Q3W. In other embodiments, the PD-1 antagonist in the therapy is pembrolizumab, or a pembrolizumab variant, which is administered in a liquid medicament at a flat dose such as 200 mg Q3W or 400 mg Q6W.

In some embodiments, the PD-1 antagonist in the therapy is pembrolizumab, which is administered at a dose of 400 mg every 6 weeks (Q6W).

In some embodiments of the methods, compositions, kits and uses described herein, the anti-human PD-1 antibody (e.g., anti-PD-1 monoclonal antibody) or antigen binding fragment thereof is pembrolizumab, and the human patient is administered about 200 mg, about 240 mg, about 400 mg, about 480 mg, or about 2 mg/kg pembrolizumab once every three or six weeks. In one embodiment, the human patient is administered about 200 mg pembrolizumab once every three weeks. In one embodiment, the human patient is administered about 240 mg pembrolizumab once every three weeks. In one embodiment, the human patient is administered 2 mg/kg pembrolizumab once every three weeks. In one embodiment, the human patient is administered 400 mg pembrolizumab once every six weeks.

In certain embodiments of the methods, compositions, kits and uses described herein, the anti-human PD-1 monoclonal antibody or antigen binding fragment thereof is pembrolizumab and the human patient is administered 200 mg pembrolizumab once every three weeks.

In certain embodiments of the methods, compositions, kits and uses described herein, the anti-human PD-1 monoclonal antibody or antigen binding fragment thereof is pembrolizumab and the human patient is administered 400 mg pembrolizumab once every six weeks.

In some embodiments of the methods, compositions, kits and uses described herein, the anti-human PD-1 monoclonal antibody or antigen binding fragment thereof is pembrolizumab, and the human patient is administered about 200 mg, about 240 mg, about 400 mg, about 480 mg, or about 2 mg/kg pembrolizumab once every six weeks. In one embodiment, the human patient is administered about 200 mg pembrolizumab once every six weeks. In one embodiment, the human patient is administered about 240 mg pembrolizumab once every six weeks. In one embodiment, the human patient is administered about 400 mg pembrolizumab once every six weeks. In one embodiment, the human patient is administered 480 mg pembrolizumab once every six weeks. In one embodiment, the human patient is administered 2 mg/kg pembrolizumab once every six weeks.

In some embodiments, pembrolizumab is provided as a liquid medicament that comprises 25 mg/ml pembrolizumab, 7% (w/v) sucrose, 0.02% (w/v) polysorbate 80 in 10 mM histidine buffer pH 5.5. In other embodiments, pembrolizumab is provided as a liquid medicament that comprises about 125 to about 200 mg/mL of pembrolizumab, or an antigen binding fragment thereof; about 10 mM histidine buffer; about 10 mM L-methionine, or a pharmaceutically acceptable salt thereof; about 7% (w/v) sucrose; and about 0.02 % (w/v) polysorbate 80.

In certain embodiments of the methods, compositions, kits and uses described herein, the anti-human PD-1 monoclonal antibody or antigen binding fragment thereof is pembrolizumab, and the human patient is administered about 200 mg pembrolizumab once every three weeks. In certain embodiments of the methods, compositions, kits and uses described herein, the anti-human PD-1 monoclonal antibody or antigen binding fragment thereof is pembrolizumab, and the human patient is administered about 400 mg pembrolizumab once every six weeks.

In some embodiments, the selected dose of pembrolizumab is administered by IV infusion. In one embodiment, the selected dose of pembrolizumab is administered by IV infusion over a time period of between 25 and 40 minutes, or about 30 minutes. In other embodiments, the selected dose of pembrolizumab is administered by subcutaneous injection.

In some embodiments, the selected dose of pembrolizumab is administered subcutaneously. In certain embodiments, the amount of pembrolizumab administered subcutaneously to the patient is from 320 mg to 420 mg, from 340 mg to 420 mg, from 345 mg to 415 mg, from 350 mg to 410 mg, from 355 mg to 405 mg, from 360 mg to 400 mg, from 365 mg to 395 mg, from 370 mg to 390 mg, from 375 mg to 385 mg, or from 379 mg to 381 mg. In one embodiment, pembrolizumab is administered by subcutaneous injection at a dose of about 280 mg to about 450 mg. In a further embodiment, pembrolizumab is administered by subcutaneous injection at a dose of about 300 mg to about 450 mg. In yet a further embodiment, pembrolizumab is administered subcutaneously at a dose of about 320 mg to about 450 mg.

In certain embodiments, pembrolizumab is administered subcutaneously to the patient, wherein the pembrolizumab is part of a composition and is present in the composition at a concentration of 130 mg/mL. In certain embodiments, pembrolizumab administered subcutaneously to the patient, wherein the pembrolizumab is part of a composition and is present in the composition at a concentration of 165 mg/mL. In certain embodiments, pembrolizumab is administered subcutaneously to the patient in two injections. In certain embodiments, the amount of pembrolizumab administered subcutaneously to the patient is 380 mg in one pre-filled syringe. In certain embodiments, the amount of pembrolizumab administered subcutaneously to the patient is 380 mg in two pre-filled syringes.

In one embodiment, the selected dose of pembrolizumab is administered by subcutaneous injection at a dose that is at least about 1.6 times higher than a 200 mg or a 2 mg/kg dose. In one embodiment, the subcutaneous dose is administered once every three weeks. In one embodiment, the subcutaneous dose is administered once every six weeks. In one embodiment, the bioavailability of the pembrolizumab subcutaneous dose is at least 63%. In one embodiment, the bioavailability of the pembrolizumab subcutaneous dose is at least 64%. In one embodiment, the bioavailability of the pembrolizumab subcutaneous dose is at least 66%.

In other embodiments of the methods, compositions, kits, and uses described herein, the anti-human PD-1 monoclonal antibody or antigen binding fragment thereof is nivolumab, the human patient is administered about 240 mg or about 3 mg/kg nivolumab, and nivolumab is administered once every two weeks. In one specific embodiment, the human patient is administered about 240 mg nivolumab once every two weeks. In one specific embodiment, the human patient is administered about 3 mg/kg nivolumab once every two weeks. In other embodiments of the methods, compositions, kits and uses described herein, the anti-human PD-1 monoclonal antibody or antigen binding fragment thereof is nivolumab, the human patient is administered about 480 mg nivolumab once every four weeks.

The immunoconjugates of Formula (I), and a pharmaceutically acceptable carrier or excipient(s) will typically be formulated into a dosage form adapted for administration to a patient by a desired route of administration. For example, dosage forms include sterile solutions, suspensions, and powders for reconstitution. Suitable pharmaceutically acceptable carriers or excipients will vary depending upon the particular dosage form chosen. In addition, suitable pharmaceutically acceptable carriers or excipients may be chosen for a particular function. In embodiments, the Immunoconjugate of Formula (I) may be formulated into a dosage form that allows for systemic use, i.e., distribution of Immunoconjugate of Formula (I) throughout the body of the patient; examples of such systemic administration include subcutaneous administration and intravenous administration. In additional embodiments, the Immunoconjugate of Formula (I) may be formulated into a dosage form that allows for targeted or isolated use, i.e., administration of the Immunoconjugate of Formula (I) only to the portion of the patient's body to be treated; examples of such targetted administration include intratumoral injection. In certain embodiments, the doses are provided intravenously or subcutaneously. In certain embodiments, the dose of the Immunoconjugate of Formula (I) is 1.0mg/kg to 6.0mg/kg, 1.0mg/kg to 5.5mg/kg, 1.0mg/kg to 5.0mg/kg, 1.0mg/kg to 4.5mg/kg, 1.0mg/kg to 4.0mg/kg, 1.0mg/kg to 3.5mg/kg, 1.0mg/kg to 3.0mg/kg, 1.0mg/kg to 2.5mg/kg, 1.0mg/kg to 2.0mg/kg, 1.0mg/kg to 1.5mg/kg, 1.5mg/kg to 6.0mg/kg, 1.5mg/kg to 5.5mg/kg, 1.5mg/kg to 5.0mg/kg, 1.5mg/kg to 4.5mg/kg, 1.5mg/kg to 4.0mg/kg, 1.5mg/kg to 3.5mg/kg, 1.5mg/kg to 3.0mg/kg, 1.5mg/kg to 2.5mg/kg, 1.5mg/kg to 2.0mg/kg, 2.0mg/kg to 6.0mg/kg, 2.0mg/kg to 5.5mg/kg, 2.0mg/kg to 5.0mg/kg, 2.0mg/kg to 4.5mg/kg, 2.0mg/kg to 4.0mg/kg, 2.0mg/kg to 3.5mg/kg, 2.0mg/kg to 3.0mg/kg, 2.0mg/kg to 2.5mg/kg, 2.5mg/kg to 6.0mg/kg, 2.5mg/kg to 5.5mg/kg, 2.5mg/kg to 5.0mg/kg, 2.5mg/kg to 4.5mg/kg, 2.5mg/kg to 4.0mg/kg, 2.5mg/kg to 3.5mg/kg, 2.5mg/kg to 3.0mg/kg, 3.0mg/kg to 6.0mg/kg, 3.0mg/kg to 5.5mg/kg, 3.0mg/kg to 5.0mg/kg, 3.0mg/kg to 4.5mg/kg, 3.0mg/kg to 4.0mg/kg, 3.0mg/kg to 3.5mg/kg, 3.5mg/kg to 6.0mg/kg, 3.5mg/kg to 5.5mg/kg, 3.5mg/kg to 5.0mg/kg, 3.5mg/kg to 4.5mg/kg, 3.5mg/kg to 4.0mg/kg, 4.0mg/kg to 6.0mg/kg, 4.0mg/kg to 5.5mg/kg, 4.0mg/kg to 5.0mg/kg, 4.0mg/kg to 4.5mg/kg, 4.5mg/kg to 6.0mg/kg, 4.5mg/kg to 5.5mg/kg, 4.5mg/kg to 5.0mg/kg, 5.0mg/kg to 6.0mg/kg, 5.0mg/kg to 5.5mg/kg, or 5.5mg/kg to 6.0mg/kg. In some embodiments, the dose of the Immunoconjugate of Formula (I) is 3.0 mg/kg to 5.0 mg/kg. In certain embodiments, a total dose for a treatment interval is generally at least 0.25 mg/kg body weight, more generally at least 0.25 mg/kg, 0.5 mg/kg, 0.75 mg/kg, 1.0 mg/kg, 1.25 mg/kg, 1.50 mg/kg, 1.75 mg/kg, 2.00 mg/kg, 2.25 mg/kg, 2.50 mg/kg, 2.75 mg/kg, 3.00 mg/kg, 3.25 mg/kg, 3.50 mg/kg, 3.75 mg/kg, 4.00 mg/kg, 4.25 mg/kg, 4.50 mg/kg, 4.75 mg/kg, 5.00 mg/kg, 5.25 mg/kg, 5.50 mg/kg, 5.75 mg/kg, 6.00 mg/kg, 6.25 mg/kg, 6.50 mg/kg, 6.75 mg/kg, and 7.00 mg/kg. In other embodiments, Immunoconjugates of Formula (I) are administered subcutaneously or intravenously, on a weekly, biweekly, "every 3 weeks," "every 4 weeks," monthly, "every 6 weeks," bimonthly, or quarterly basis at 2.00 mg/kg, 2.25 mg/kg, 2.50 mg/kg, 2.75 mg/kg, 3.00 mg/kg, 3.25 mg/kg, 3.75 mg/kg, 4.25 mg/kg, 4.50 mg/kg, 4.75 mg/kg, 5.00 mg/kg, 5.25 mg/kg, 5.50 mg/kg, 5.75 mg/kg, or 6.00 mg/kg. In specific embodiments, the Immunoconjugate of Formula (I) is administered at 4.50 mg/kg, 4.75 mg/kg, 5.00 mg/kg, 5.25 mg/kg, or 5.50 mg/kg, every 3 weeks. In specific embodiments, the Immunoconjugate of Formula (I) is administered at 4.50 mg/kg, 4.75 mg/kg, 5.00 mg/kg, 5.25 mg/kg, or 5.50 mg/kg, every 3 weeks, every 4 weeks or every 6 weeks. In another specific embodiments, the Immunoconjugate of Formua (I) is administered at 5.00 mg/kg every 3 weeks, every 4 weeks, or every 6 weeks.

In one embodiment, in the combination therapies of the present disclosure, an Immunoconjugate of Formula (I) is administered at a dose of 0.25 mg/kg to 10 mg/kg. In some embodiments, the Immunoconjugate of Formula (I) may be administered at a dose of 0.25 0.5, 0.75, 1, 1.25, 1.5, 1.75, 2, 2.25, 2.5, 2.75, 3, 3.25, 3.5, 3.75, 4, 4.25, 4.5, 4.75, 5, 5.5, 6, 6.5, 7, 8, 9, or 10 mg/kg as a single dose, or in any combination thereof for multiple doses. In certain embodiments, the Immunoconjugate of Formula (I) is administered at a dose of 4, 4.25, 4.5, 4.75, 5, 5.25, 5.5, 5.75, or 6 mg/kg. In other embodiments, the Immunoconjugate of Formula (I) is administered at a dose of 3.5, 3.75, 4.0, 4.25, 4.5, 4.75, 5, 5.25, or 5.5 mg/kg. In a specific embodiment, the Immunoconjugate of Formula (I) is administered at a dose of 5 mg/kg.

In some embodiments, the Immunoconjugate of Formula (I) is administered in repeated cycles of 1, 2, 3, 4, 5, 6, 7, 8, 9, 10, 11, 12, 13, 14, 15, or 16 weeks. In one specific embodiment, the Immunoconjugate of Formula (I) is administered in three-week cycles. In another specific embodiment, the Immunoconjugate of Formula (I) is administered in four-week cycles. In still another specific embodiment, the Immunoconjugate of Formula (I) is administered in six-week cycles. The treatment regimen may comprise 1, 2, 3, 4, 5, 6, 7, 8, 9, 10, 11, 12, 13, 14, 15, 16, 17, 18, 19, 20, or more cycles of administration (e.g., 3 or more cycles, or 4 or more cycles). In certain embodiments, the Immunoconjugate of Formula (I) is administered on one, two, three, four, five, six, or seven days of the cycle. The days of administration may be consecutive or may have one, two, three, four, five, or six days, one week, two weeks, three weeks, or four weeks, or any combination thereof, between them. In particular embodiments, the Immunoconjugate of Formula (I) is administered on Day 1 only of each cycle (e.g., a three-week cycle). In particular embodiments, the Immunoconjugate of Formula (I) is administered on Days 1 and 8 of each cycle (e.g., a three-week cycle). In particular embodiments, the Immunoconjugate of Formula (I) is administered on Days 1, 8, and 15 of each cycle (e.g., a four-week cycle or a six-week cycle). In a specific embodiment, the Immunoconjugate of Formula (I) is administered on the first day of a 3-week cycle. In another specific embodiment, the Immunoconjugate of Formula (I) is administered on the first day of a 4-week cycle. In another embodiment, the Immunoconjugate of Formula (I) is administered on days 1, 15, and 29 of a six-week cycle. In other embodiments, the Immunoconjugate of Formula (I) is administered at 3.50 mg/kg, 3.75 mg/kg, 4.0 mg/kg, 4.25 mg/kg, 4.50 mg/kg, 4.75 mg/kg, 5.00 mg/kg, 5.25 mg/kg, or 5.50 mg/kg, every 2 weeks, every 3 weeks, every 4 weeks, or every 6 weeks. In a specific embodiment, the Immunoconjugate of Formula (I) is administered at 4.00 mg/kg every 2 weeks. In another specific embodiment, the Immunoconjugate of Formula (I) is administered at 4.00 mg/kg every 3 weeks. In a specific embodiment, the Immunoconjugate of Formula (I) is administered at 5.00 mg/kg every 2 weeks. In another specific embodiment, the Immunoconjugate of Formula (I) is administered at 5.00 mg/kg every 3 weeks.

In some embodiments, the Immunoconjugate of Formula (I) and the anti-human PD-1 antibody or antigen-binding fragment thereof are each administered in a cycle of every week, every 1.5 weeks, every 2 weeks, every 3 weeks, every 4 weeks, every 5 weeks, every 6 weeks, monthly, every two months, or every quarter.

In some embodiments, the Immunoconjugate of Formula (I) is administered once every 2 weeks, every 3 weeks, every 4 weeks, every 5 weeks, or every 6 weeks at 1 mg/kg, 1.25 mg/kg, 1.5 mg/kg, 1.75 mg/kg, 2 mg/kg, 2.25 mg/kg, 2.5 mg/kg, 2.75 mg/kg, 3 mg/kg, 3.50 mg/kg, 3.75 mg/kg, 4.0 mg/kg, 4.25 mg/kg, 4.50 mg/kg, 4.75 mg/kg, 5.00 mg/kg, 5.25 mg/kg, or 5.50 mg/kg.

In some embodiments, the Immunoconjugate of Formula (I) is administered twice every 2 weeks, every 3 weeks, every 4 weeks, every 5 weeks, or every 6 weeks at 1 mg/kg, 1.25 mg/kg, 1.5 mg/kg, 1.75 mg/kg, 2 mg/kg, 2.25 mg/kg, 2.5 mg/kg, 2.75 mg/kg, 3 mg/kg, 3.50 mg/kg, 3.75 mg/kg, 4.0 mg/kg, 4.25 mg/kg, 4.50 mg/kg, 4.75 mg/kg, 5.00 mg/kg, 5.25 mg/kg, or 5.50 mg/kg.

In some embodiments, the Immunoconjugate of Formula (I) is administered three times every 2 weeks, every 3 weeks, every 4 weeks, every 5 weeks, or every 6 weeks at 1 mg/kg, 1.25 mg/kg, 1.5 mg/kg, 1.75 mg/kg, 2 mg/kg, 2.25 mg/kg, 2.5 mg/kg, 2.75 mg/kg, 3 mg/kg, 3.50 mg/kg, 3.75 mg/kg, 4.0 mg/kg, 4.25 mg/kg, 4.50 mg/kg, 4.75 mg/kg, 5.00 mg/kg, 5.25 mg/kg, or 5.50 mg/kg.

In some embodiments, the Immunoconjugate A is administered once every 2 weeks at 3.0 mg/kg.

In some embodiments, the Immunoconjugate A is administered twice every 2 weeks at 3.0 mg/kg.

In some embodiments, the Immunoconjugate A is administered three times every 2 weeks at 3.0 mg/kg.

In some embodiments, the Immunoconjugate A is administered once every 2 weeks at 4.0 mg/kg.

In some embodiments, the Immunoconjugate A is administered twice every 2 weeks at 4.0 mg/kg.

In some embodiments, the Immunoconjugate A is administered three times every 2 weeks at 4.0 mg/kg.

In some embodiments, the Immunoconjugate A is administered once every 3 weeks at 2.25 mg/kg.

In some embodiments, the Immunoconjugate A is administered twice every 3 weeks at 2.25 mg/kg.

In some embodiments, the Immunoconjugate A is administered three times every 3 weeks at 2.25 mg/kg.

In some embodiments, the Immunoconjugate of Formula (I) is administered once, twice, or three times every 2 weeks, every 3 weeks, every 4 weeks, every 5 weeks, or every 6 weeks at 1 mg/kg, 1.25 mg/kg, 1.5 mg/kg, 1.75 mg/kg, 2 mg/kg, 2.25 mg/kg, 2.5 mg/kg, 2.75 mg/kg, 3 mg/kg, 3.50 mg/kg, 3.75 mg/kg, 4.0 mg/kg, 4.25 mg/kg, 4.50 mg/kg, 4.75 mg/kg, 5.00 mg/kg, 5.25 mg/kg, or 5.50 mg/kg; after 1, 2, 3, 4, 5, or 6 cycles, the Immunoconjugate of Formula (I) is administered once, twice, or three times every 2 weeks, every 3 weeks, every 4 weeks, every 5 weeks or every 6 weeks at a dose of 1 mg/kg, 3 mg/kg, 3.50 mg/kg, 3.75 mg/kg, 4.0 mg/kg, 4.25 mg/kg, 4.50 mg/kg, 4.75 mg/kg, 5.00 mg/kg, 5.25 mg/kg, or 5.50 mg/kg; wherein one cycle consists of 6 weeks.

In some embodiments, the Immunoconjugate of Formula (I) is administered once every 2 weeks, once every 3 weeks, twice every 3 weeks, once every 4 weeks, once every 5 weeks or once every 6 weeks at a dose of 1 mg/kg, 1.25 mg/kg, 1.5 mg/kg, 1.75 mg/kg, 2 mg/kg, 2.25 mg/kg, 2.5 mg/kg, 2.75 mg/kg, 3 mg/kg, 3.50 mg/kg, 3.75 mg/kg, 4.0 mg/kg, 4.25 mg/kg, 4.50 mg/kg, 4.75 mg/kg, 5.00 mg/kg, 5.25 mg/kg or 5.50 mg/kg; after 1, 2, 3, 4, 5, or 6 cycles, the Immunoconjugate of Formula (I) is administered once every 2 weeks, once every 3 weeks, twice every 3 weeks, once every 4 weeks, once every 5 weeks or once every 6 weeks at a dose of 1 mg/kg, 1.25 mg/kg, 1.5 mg/kg, 1.75 mg/kg, 2 mg/kg, 2.25 mg/kg, 2.5 mg/kg, 2.75 mg/kg, 3 mg/kg, 3.50 mg/kg, 3.75 mg/kg, 4.0 mg/kg, 4.25 mg/kg, 4.50 mg/kg, 4.75 mg/kg, 5.00 mg/kg, 5.25 mg/kg or 5.50 mg/kg; wherein one cycle consists of 6 weeks.

In some embodiments, the anti-human PD-1 antibody or antigen-binding fragment thereof is administered at a dose of about 400 mg, and the Immunoconjugate of Formula (I) is administered at a dose of about 4 mg/kg.

In some embodiments, the Immunoconjugate A is administered once every 3 weeks at 2.25 mg/kg; after 1, 2, or 3 cycles, the Immunoconjugate A is administered one every 2 weeks at 4.0 mg/kg; wherein one cycle consists of 6 weeks.

In some embodiments, the Immunoconjugate A is administered twice every 3 weeks at 2.25 mg/kg; after 1, 2, or 3 cycles, the Immunoconjugate A is administered once every 2 weeks at 4.0 mg/kg; wherein one cycle consists of 6 weeks.

In some embodiments, the Immunoconjugate A is administered once every 2 weeks at 3.0 mg/kg; after 1, 2, or 3 cycles, the Immunoconjugate A is administered once every 2 weeks at 4.0 mg/kg; wherein one cycle consists of 6 weeks.

In some embodiments, the Immunoconjugate A is administered once every 3 weeks at 2.25 mg/kg; after 2 cycles, the Immunoconjugate A is administered once every 2 weeks at 4.0 mg/kg; wherein one cycle consists of 6 weeks.

In some embodiments, the Immunoconjugate A is administered twice every 3 weeks at 2.25 mg/kg; after 2 cycles, the Immunoconjugate A is administered once every 2 weeks at 4.0 mg/kg; wherein one cycle consists of 6 weeks.

In some embodiments, the Immunoconjugate A is administered once every 2 weeks at 3.0 mg/kg; after 2 cycles, the Immunoconjugate A is administered once every 2 weeks at 4.0 mg/kg; wherein one cycle consists of 6 weeks.

The Immunoconjugate of Formula (I) may be administered initially according to a dosage regimen described herein and subsequently according to a different dosage regimen described herein (*e.g.,* to increase or decrease the frequency of administration). In some embodiments, the Immunoconjugate of Formula (I) is administered weekly during the first 1, 2, 3, 4, 5, or 6, 7, 8, 9, 10, 11, or 12 weeks, then every 3 weeks thereafter. In certain embodiments, the Immunoconjugate of Formula (I) is administered weekly during the first 2, 3, 4, 5, or 6 weeks, and then every 3 weeks. In certain embodiments, the Immunoconjugate of Formula (I) is administered weekly during the first 1, 2, 3, 4, 5, or 6 weeks, and then every 4 weeks.

The Immunoconjugate of Formula (I) may be administered via parenteral administration. As used herein, "parenteral administration" of an immunoconjugate includes any route of administration characterized by physical breaching of a tissue of a patient and administration of the immunoconjugate through the breach in the tissue, thus generally resulting in the direct administration into the blood stream, into muscle, or into an internal organ. Parenteral administration thus includes, but is not limited to, administration of an immunoconjugate by
injection of the immunoconjugate, by application of the immunoconjugate through a surgical incision, by application of the immunoconjugate through a tissue-penetrating non-surgical wound, and the like. In particular, parenteral administration is contemplated to include, but is not limited to, subcutaneous, intraperitoneal, intramuscular, intrasternal, intravenous, intraarterial, intrathecal, intraventricular, intraurethral, intracranial, intratumoral, and intrasynovial injection or infusions; and kidney dialytic infusion techniques. In a specific embodiment, the Immunoconjugate of Formula (I) is administered intravenously. In another specific embodiment the Immunoconjugate of Formula (I) is administered subcutaneously. Regional perfusion is also contemplated. In some embodiments, the infusion may be administered by one route (e.g., intravenously) for initial doses and then be administered by another route for subsequent doses.

In certain embodiments, the Immunoconjugate of Formula (I) is administered by intravenous (IV) infusion. The infusion may take place over a period of about 0.1 to about 4 hours (e.g., about 5, 10, 15, 20, 25, 30, 35, 40, 45, 50, 55, 60, 65, 70, 75, 80, 85, 90, 120, or 180 minutes). In particular embodiments, the infusion time is 30 minutes. Infusion times may be extended as necessary to accommodate individual patient tolerance of treatment. Where the immunoconjugate is administered in more than one dose, in some embodiments, the infusion time for the first dose is longer than the infusion time for subsequent doses, or alternatively, the infusion time for the first dose is shorter than the infusion time for subsequent doses.

In one embodiment, provided is a method treating a cellular proliferative disorder, said method comprising administering to a patient in need thereof a combination therapy that comprises: (a) an anti-human PD-1 antibody (or antigen-binding fragment thereof); and (b) an Immunoconjugate of Formula (I); wherein the anti-human PD-1 antibody (or antigen-binding fragment thereof) and the Immunoconjugate of Formula (I) are each administered on day 1 of a three-week cycle ("Combination Regimen 1").

In a specific embodiment, for Combination Regimen 1, the anti-human PD-1 antibody (or antigen-binding fragment thereof) is administered at a dose of 200 mg per each 3-week cycle, and the Immunoconjugate of Formula (I) is administered at a dose of 5 mg/kg per each 3-week cycle. In another specific embodiment, for Combination Regimen 1, the anti-human PD-1 antibody (or antigen-binding fragment thereof) is administered at a dose of 200 mg per each 3-week cycle, and the Immunoconjugate of Formula (I) is administered at a dose of 4 mg/kg per each 3-week cycle.

In a specific embodiment, for Combination Regimen 1, the anti-human PD-1 antibody is pembrolizumab.

In a specific embodiment, for Combination Regimen 1, the Immunoconjugate of Formula (I) is Immunconjugate A.

In another specific embodiment, for Combination Regimen 1, the anti-human PD-1 antibody is pembrolizumab, and the Immunoconjugate of Formula (I) is Immunconjugate A.

In a specific embodiment, for Combination Regimen 1, the Immunoconjugate of Formula (I) is Immunconjugate A.

In another embodiment, Combination Regimen 1 is administered in from 1 to 4 cycles.

In one embodiment, Combination Regimen 1 is administered to a patient to treat cancer, wherein the cancer is non-small cell lung cancer.

In a specific embodiment, Combination Regimen 1 is administered to a patient to treat cancer, wherein the cancer is non-small cell lung cancer, and:
(a) the non-small cell lung cancer is without EGFR mutation, and ALK fusion genes; or
(b) the non-small cell lung cancer has an EGFR actionable mutation, and the patient has failed prior EGFR-TKI treatment.

In one embodiment, provided is a method treating a cellular proliferative disorder, said method comprising administering to a patient in need thereof a combination therapy that comprises: (a) an anti-human PD-1 antibody (or antigen-binding fragment thereof); and (b) an Immunoconjugate of Formula (I); wherein the anti-human PD-1 antibody (or antigen-binding fragment thereof) is administered on day 1, and the Immunoconjugate of Formula (I) is administered on days 1, 15, and 29 of a six-week cycle ("Combination Regimen 2")

In a specific embodiment, for Combination Regimen 2, the anti-human PD-1 antibody (or antigen-binding fragment thereof) is administered at a single dose of 400 mg on day 1 of each 6-week cycle, and the Immunoconjugate of Formula (I) is administered at a dose of 5 mg/kg on each of days 1, 15, and 29 of each 6-week cycle. In another specific embodiment, for Combination Regimen 2, the anti-human PD-1 antibody (or antigen-binding fragment thereof) is administered at a single dose of 400 mg on day 1 of each 6-week cycle, and the Immunoconjugate of Formula (I) is administered at a dose of 4 mg/kg on each of days 1, 15, and 29 of each 6-week cycle.

In a specific embodiment, for Combination Regimen 2, the anti-human PD-1 antibody is pembrolizumab.

In a specific embodiment, for Combination Regimen 2, the Immunoconjugate of Formula (I) is Immunconjugate A.

In another specific embodiment, for Combination Regimen 2, the anti-human PD-1 antibody is pembrolizumab, and the Immunoconjugate of Formula (I) is Immunconjugate A.

In another embodiment, Combination Regimen 2 is administered in from 1 to 4 cycles.

In one embodiment, Combination Regimen 2 is administered to a patient to treat cancer, wherein the cancer is non-small cell lung cancer.

In a specific embodiment, Combination Regimen 2 is administered to a patient to treat cancer, wherein the cancer is non-small cell lung cancer, and:
(a) the non-small cell lung cancer is without EGFR mutation, and ALK fusion genes; or
(b) the non-small cell lung cancer has an EGFR actionable mutation, and the patient has failed prior EGFR-TKI treatment.

In one embodiment, a method for treating non-small cell lung cancer is provided, the method comprises administering a combination therapy to a patient in need thereof, the combination therapy comprising: (a) an anti-human PD-1 antibody (or antigen-binding fragment thereof); and (b) an immunoconjugate represented by Formula (I); wherein the doses and frequencies of administration of the anti-human PD-1 antibody (or antigen-binding fragment thereof) and the Immunoconjugate of Formula (I) are as described in any one of the above items.

In one embodiment, the non-small cell lung cancer is as described in any one of the above items.

In one embodiment, the non-small cell lung cancer is EGFR wild-type, ALK fusion gene-negative, with PD-L1 TPS ≥ 1%.

In one embodiment, the Immunoconjugate of Formula (I) is administered at a frequency of once every 1 to 4 weeks, and the anti-human PD-1 antibody (or antigen-binding fragment thereof) is administered at a frequency of once every 1 to 10 weeks; preferably once every 2 to 8 weeks.

In one embodiment, the Immunoconjugate of Formula (I) is administered at a frequency of once every 1 to 4 weeks and at a dose of 1 to 10 mg/kg, and the anti-human PD-1 antibody (or antigen-binding fragment thereof) is administered at a frequency of once every 1 to 10 weeks (preferably once every 2 to 8 weeks) and at a dose of 100 to 800 mg/kg.

In one embodiment, the Immunoconjugate of Formula (I) is administered at a frequency of once every 2 weeks, and the anti-human PD-1 antibody (or antigen-binding fragment thereof) is administered at a frequency of once every 6 weeks.

In one embodiment, the Immunoconjugate of Formula (I) is administered at a frequency of once every 2 weeks and a dose of 4 mg/kg, and the anti-human PD-1 antibody (or antigen-binding fragment thereof) is administered at a frequency of once every 6 weeks and a dose of 400 mg/kg.

In one embodiment, a method for treating non-small cell lung cancer is provided, the method comprising administering a combination therapy to a patient in need thereof, the combination therapy comprising: (a) an anti-human PD-1 antibody (or antigen-binding fragment thereof); (b) an immunoconjugate represented by Formula (I); and (c) a platinum-containing chemotherapeutic agent; wherein the doses and frequencies of administration of the anti-human PD-1 antibody (or antigen-binding fragment thereof), the platinum-containing chemotherapeutic agent, and the Immunoconjugate of Formula (I) are as described above in any one of the above items.

In one embodiment, the non-small cell lung cancer is as described above in any one of the above items.

In one embodiment, the non-small cell lung cancer is EGFR wild-type and ALK fusion gene-negative.

In one embodiment, the Immunoconjugate of Formula (I) is administered at a frequency of once every 1 to 5 weeks, the anti-human PD-1 antibody (or antigen-binding fragment thereof) is administered at a frequency of once every 1 to 10 weeks (preferably once every 2 to 8 weeks), and the platinum-containing chemotherapeutic agent is administered at a frequency of once every 1 to 5 weeks.

In some embodiments, the platinum-containing chemotherapeutic agent is administered at a dose of about AUC 1 mg/mL/min to AUC 10 mg/mL/min or 10 mg/m² to 150 mg/m².

In some embodiments, the Immunoconjugate of Formula (I) and the platinum-containing chemotherapeutic agent are each administered in a cycle of every week, every 1.5 weeks, every 2 weeks, every 3 weeks, every 4 weeks, every 5 weeks, every 6 weeks, every month, every two months, or every quarter.

In some embodiments, the Immunoconjugate of Formula (I) and the platinum-containing chemotherapeutic agent are each administered in a 3-week cycle, with doses given on Day 1 and/or Day 8 of each 3-week cycle;
preferably, the Immunoconjugate of Formula (I) and the platinum-containing chemotherapeutic agent are each administered in a 3-week cycle, with doses given on Day 1 of each 3-week cycle;
preferably, the Immunoconjugate of Formula (I) and the platinum-containing chemotherapeutic agent are each administered in a 3-week cycle, wherein the Immunoconjugate of Formula (I) is administered on Day 1 and Day 8 of each 3-week cycle, and the platinum-containing chemotherapeutic agent is administered on Day 1 of each 3-week cycle.

In some embodiments, the Immunoconjugate of Formula (I) and the platinum-containing chemotherapeutic agent are each administered in a 6-week cycle, wherein the Immunoconjugate of Formula (I) is administered on Days 1, 8, 22 and/or 29 of each 6-week cycle, and the platinum-containing chemotherapeutic agent is administered on Days 1 and 22 of each 6-week cycle;
wherein the Immunoconjugate of Formula (I) and the platinum-containing chemotherapeutic agent are each administered in a 6-week cycle, wherein the Immunoconjugate of Formula (I) is administered on Days 1, 8, 22 and 29 of each 6-week cycle, and the platinum-containing chemotherapeutic agent is administered on Days 1 and 22 of each 6-week cycle;
wherein the Immunoconjugate of Formula (I) and the platinum-containing chemotherapeutic agent are each administered in a 6-week cycle, wherein the Immunoconjugate of Formula (I) is administered on Days 1 and 22 of each 6-week cycle, and the platinum-containing chemotherapeutic agent is administered on Days 1 and 22 of each 6-week cycle.

In some embodiments, the Immunoconjugate of Formula (I) and the platinum-containing chemotherapeutic agent are administered in 1, 2, 3 or 4 phases.

In some embodiments, the Immunoconjugate of Formula (I) and the platinum-containing chemotherapeutic agent are administered in 2 phases, wherein,
in the first phase, the Immunoconjugate of Formula (I) is administered in a 3-week cycle and on Day 1 of each 3-week cycle, and the platinum-containing chemotherapeutic agent is administered in a 3-week cycle and on Day 1 of each 3-week cycle;
in the second phase, the Immunoconjugate of Formula (I) is administered in a 2-week cycle and on Day 1 of each 2-week cycle.

In some embodiments, the Immunoconjugate of Formula (I) and the platinum-containing chemotherapeutic agent are administered in 2 phases stages, wherein,
in the first phase, the Immunoconjugate of Formula (I) is administered in a 3-week cycle and Day 1 and Day 8 of each 3-week cycle, and the platinum-containing chemotherapeutic agent is administered in a 3-week cycle and on Day 1 of each 3-week cycle;
in the second phase, the Immunoconjugate of Formula (I) is administered in a 2-week cycle and on Day 1 of each 2-week cycle.

In some embodiments, each phase lasts for 2 to 24 weeks, 4 to 24 weeks, 6 to 24 weeks, 8 to 24 weeks, 10 to 24 weeks, 12 to 24 weeks, 14 to 24 weeks, 16 to 24 weeks, 18 to 24 weeks, 20 to 24 weeks, or 22 to 24 weeks.

In some embodiments, the Immunoconjugate of Formula (I) is administered at a dose of about 4 mg/kg and carboplatin is administered at a dose of AUC 5 mg/mL/min, or,
the Immunoconjugate of Formula (I) is administered at a dose of about 5 mg/kg and carboplatin is administered at a dose of AUC 5 mg/mL/min.

In some embodiments, the Immunoconjugate of Formula (I) is administered at a dose of about 4 mg/kg and cisplatin is administered at a dose of 75 mg/m², or,
the Immunoconjugate of Formula (I) is administered at a dose of about 5 mg/kg and cisplatin is administered at a dose of 75 mg/m².

In some embodiments, in the first phase, the Immunoconjugate of Formula (I) is administered at a dose of about 3 mg/kg, and carboplatin is administered at a dose of AUC 5 mg/mL/min;
in the second phase, the Immunoconjugate of Formula (I) is administered at a dose of about 4 mg/kg.

In some embodiments, in the first phase, the Immunoconjugate of Formula (I) is administered at a dose of about 2.25 mg/kg, and carboplatin is administered at a dose of AUC 5 mg/mL/min;
in the second phase, the Immunoconjugate of Formula (I) is administered at a dose of about 4 mg/kg.

In some embodiments, the number of cycles is 1 to 4.

In some embodiments, the Immunoconjugate of Formula (I), the anti-human PD-1 antibody or antigen-binding fragment thereof, and the platinum-containing chemotherapeutic agent are each administered in a cycle of every week, every 1.5 weeks, every 2 weeks, every 3 weeks, every 4 weeks, every 5 weeks, every 6 weeks, every month, every two months, or every quarter.

In some embodiments, the Immunoconjugate of Formula (I), the anti-human PD-1 antibody or antigen-binding fragment thereof, and the platinum-containing chemotherapeutic agent are each administered in a 3-week cycle, and are each administered on Day 1 of each 3-week cycle.

In some embodiments, the Immunoconjugate of Formula (I), the anti-human PD-1 antibody or antigen-binding fragment thereof and the platinum-containing chemotherapeutic agent are each administered in a 6-week cycle, wherein the Immunoconjugate of Formula (I) is administered on Days 1, 8, 22 and/or 29 of each 6-week cycle, the anti-human PD-1 antibody or antigen-binding fragment thereof is administered on Day 1 of each 6-week cycle, and the platinum-containing chemotherapeutic agent is administered on Days 1 and 22 of each 6-week cycle;
preferably, the Immunoconjugate of Formula (I) is administered on Days 1, 8, 22 and 29 of each 6-week cycle, the anti-human PD-1 antibody or antigen-binding fragment thereof is administered on Day 1 of each 6-week cycle, and the platinum-containing chemotherapeutic agent is administered on Days 1 and 22 of each 6-week cycle;
preferably, the Immunoconjugate of Formula (I) is administered on Days 1 and 22 of each 6-week cycle, the anti-human PD-1 antibody or antigen-binding fragment thereof is administered on Day 1 of each 6-week cycle, and the platinum-containing chemotherapeutic agent is administered on Days 1 and 22 of each 6-week cycle.

In some embodiments, the Immunoconjugate of Formula (I), the anti-human PD-1 antibody or antigen-binding fragment thereof and the platinum-containing chemotherapeutic agent are administered in 1, 2, 3 or 4 phases.

In some embodiments, the Immunoconjugate of Formula (I), the anti-human PD-1 antibody or antigen-binding fragment thereof and the platinum-containing chemotherapeutic agent are administered in 2 phases, wherein,
in the first phase, the Immunoconjugate of Formula (I) is administered in a 3-week cycle and on Day 1 of each 3-week cycle, the anti-human PD-1 antibody or antigen-binding fragment thereof is administered in a 6-week cycle and on Day 1 of each 6-week cycle, and the platinum-containing chemotherapeutic agent is administered in a 3-week cycle and on Day 1 of each 3-week cycle;
in the second phase, the Immunoconjugate of Formula (I) is administered in a 2-week cycle and on Day 1 of each 2-week cycle, and the anti-human PD-1 antibody or antigen-binding fragment thereof is administered in a 6-week cycle and on Day 1 of each 6-week cycle.

In some embodiments, the Immunoconjugate of Formula (I), the anti-human PD-1 antibody or antigen-binding fragment thereof and the platinum-containing chemotherapeutic agent are administered in 2 phases, wherein,
in the first phase, the Immunoconjugate of Formula (I) is administered in a 3-week cycle and on Days 1 and 8 of each 3-week cycle, the anti-human PD-1 antibody or antigen-binding fragment thereof is administered in a 6-week cycle and on Day 1 of each 6-week cycle, and the platinum-containing chemotherapeutic agent is administered in a 3-week cycle and on Day 1 of each 3-week cycle;
in the second phase, the Immunoconjugate of Formula (I) is administered in a 2-week cycle and on Day 1 of each 2-week cycle, and the anti-human PD-1 antibody or antigen-binding fragment thereof is administered in a 6-week cycle and on Day 1 of each 6-week cycle.

In some embodiments, each phase lasts for 2 to 24 weeks, 4 to 24 weeks, 6 to 24 weeks, 8 to 24 weeks, 10 to 24 weeks, 12 to 24 weeks, 14 to 24 weeks, 16 to 24 weeks, 18 to 24 weeks, 20 to 24 weeks, or 22 to 24 weeks.

In some embodiments, the Immunoconjugate of Formula (I) is administered at a dose of about 4 mg/kg, the anti-human PD-1 antibody or antigen-binding fragment thereof is administered at a dose of about 200 mg, and carboplatin is administered at a dose of AUC 5 mg/mL/min, or,
the Immunoconjugate of Formula (I) is administered at a dose of about 5 mg/kg, the anti-human PD-1 antibody or antigen-binding fragment thereof is administered at a dose of about 200 mg, and carboplatin is administered at a dose of AUC 5 mg/mL/min.

In some embodiments, the Immunoconjugate of Formula (I) is administered at a dose of about 4 mg/kg, the anti-human PD-1 antibody or antigen-binding fragment thereof is administered at a dose of about 400 mg, and carboplatin is administered at a dose of AUC 5 mg/mL/min, or,
the Immunoconjugate of Formula (I) is administered at a dose of about 5 mg/kg, the anti-human PD-1 antibody or antigen-binding fragment thereof is administered at a dose of about 400 mg, and carboplatin is administered at a dose of AUC 5 mg/mL/min.

In some embodiments, in the first phase, the Immunoconjugate of Formula (I) is administered at a dose of about 3 mg/kg, the anti-human PD-1 antibody or antigen-binding fragment thereof is administered at a dose of about 400 mg, and carboplatin is administered at a dose of AUC 5 mg/mL/min;
in the second phase, the Immunoconjugate of Formula (I) is administered at a dose of about 4 mg/kg, and the anti-human PD-1 antibody or antigen-binding fragment thereof is administered at a dose of about 400 mg.

In some embodiments, in the first phase, the Immunoconjugate of Formula (I) is administered at a dose of about 2.25 mg/kg, the anti-human PD-1 antibody or antigen-binding fragment thereof is administered at a dose of about 400 mg, and carboplatin is administered at a dose of AUC 5 mg/mL/min;
in the second phase, the Immunoconjugate of Formula (I) is administered at a dose of about 4 mg/kg, and the anti-human PD-1 antibody or antigen-binding fragment thereof is administered at a dose of about 400 mg.

In some embodiments, the number of cycles is 1 to 4.

In some embodiments, the Immunoconjugate of Formula (I) is administered at a frequency of once every 1 to 5 weeks and a dose of 1 to 10 mg/kg, the anti-human PD-1 antibody (or antigen-binding fragment thereof) is administered at a frequency of once every 1 to 10 weeks (preferably once every 2 to 8 weeks) and a dose of 100 to 800 mg, and the platinum-containing chemotherapeutic agent is administered at a frequency of once every 1 to 5 weeks and a dose with a bioavailability of 1 to 10 mg/ml/min.

In some embodiments, the Immunoconjugate of Formula (I) is administered at a frequency of once every 1 to 5 weeks and a dose of 1 to 10 mg/kg, the anti-human PD-1 antibody (or antigen-binding fragment thereof) is administered at a frequency of once every 1 to 10 weeks (preferably once every 2 to 8 weeks) and a dose of 100 to 800 mg, and the platinum-containing chemotherapeutic agent is administered at a frequency of once every 1 to 5 weeks and a dose of AUC 1 to 10 mg/ml/min; after 1 to 5 cycles, the Immunoconjugate of Formula (I) is administered at a frequency of once every 1 to 5 weeks and a dose of 1 to 10 mg/kg, and the anti-human PD-1 antibody (or antigen-binding fragment thereof) is administered at a frequency of once every 1 to 10 weeks (preferably once every 2 to 8 weeks) and a dose of 100 to 800 mg; wherein one cycle consists of 6 weeks.

In some embodiments, the Immunoconjugate of Formula (I) is administered at a frequency of once every 3 weeks, the anti-human PD-1 antibody (or antigen-binding fragment thereof) is administered at a frequency of once every 6 weeks, and the platinum-containing chemotherapeutic agent is administered at a frequency of once every 3 weeks.

In some embodiments, the Immunoconjugate of Formula (I) is administered at a frequency of once every 3 weeks and a dose of 4 mg/kg, the anti-human PD-1 antibody (or antigen-binding fragment thereof) is administered at a frequency of once every 6 weeks and a dose of 400 mg/kg, and the platinum-containing chemotherapeutic agent is administered at a frequency of once every 3 weeks and a dose of AUC 5 mg/ml/min.

In some embodiments, the Immunoconjugate of Formula (I) is administered at a frequency of once every 3 weeks and a dose of 4 mg/kg, and the anti-human PD-1 antibody (or antigen-binding fragment thereof) is administered at a frequency of once every 6 weeks and a dose of 400 mg/kg, and the platinum-containing chemotherapeutic agent is administered at a frequency of once every 3 weeks and a dose of AUC 5 mg/ml/min; after 2 cycles, the anti-human PD-1 antibody (or antigen-binding fragment thereof) is administered at a frequency of once every 6 weeks and a dose of 400 mg, and the Immunoconjugate of Formula (I) is administered at a frequency of once every 2 weeks and a dose of 4 mg/kg; wherein one cycle consists of 6 weeks.

In some embodiments, a method for treating non-small cell lung cancer is provided, the method comprises administering a combination therapy to a patient in need thereof, the combination therapy comprises: (a) an immunoconjugate represented by Formula (I); and (b) a platinum-containing chemotherapeutic agent; wherein the doses and frequencies of administration of the platinum-containing chemotherapeutic agent and the Immunoconjugate of Formula (I) are as described in any one of the above items.

In some embodiments, the non-small cell lung cancer is as described in any one of the above items.

In some embodiments, the non-small cell lung cancer is a non-small cell lung cancer with EGFR sensitive mutation and failed prior EGFR-TKI treatment.

In some embodiments, the Immunoconjugate of Formula (I) is administered at a frequency of once every 1 to 5 weeks, and the platinum-containing chemotherapeutic agent is administered at a frequency of once every 1 to 5 weeks.

In some embodiments, the Immunoconjugate of Formula (I) is administered at a frequency of once every 1 to 5 weeks and a dose of 1 to 10 mg/kg, and the platinum-containing chemotherapeutic agent is administered at a frequency of once every 1 to 5 weeks and a dose of AUC 1 to 10 mg/ml/min.

In some embodiments, the Immunoconjugate of Formula (I) is administered at a frequency of once every 1 to 5 weeks and a dose of 1 to 10 mg/kg, and the platinum-containing chemotherapeutic agent is administered at a frequency of once every 1 to 5 weeks and a dose of AUC 1 to 10 mg/ml/min; after 1 to 5 cycles, the Immunoconjugate of Formula (I) is administered at a frequency of once every 1 to 5 weeks and a dose of 1 to 10 mg/kg; wherein one cycle consists of 6 weeks.

In some embodiments, the Immunoconjugate of Formula (I) is administered at a frequency of once every 3 weeks, and the platinum-containing chemotherapeutic agent is administered at a frequency of once every 3 weeks.

In some embodiments, the Immunoconjugate of Formula (I) is administered at a frequency of twice every 3 weeks and a dose of 2.25 mg/kg, and the platinum-containing chemotherapeutic agent is administered at a frequency of once every 3 weeks and a dose of AUC 5 mg/ml/min.

In some embodiments, the Immunoconjugate of Formula (I) is administered at a frequency of once every 3 weeks and a dose of 3 mg/kg, and the platinum-containing chemotherapeutic agent is administered at a frequency of once every 3 weeks and a dose of AUC 5 mg/ml/min.

In some embodiments, the Immunoconjugate of Formula (I) is administered at a frequency of twice every 3 weeks and a dose of 2.25 mg/kg, and the platinum-containing chemotherapeutic agent is administered at a frequency of once every 3 weeks and a dose of AUC 5 mg/ml/min; after 2 cycles, the Immunoconjugate of Formula (I) is administered at a frequency of once every 2 weeks and a dose of 4 mg/kg; wherein one cycle consists of 6 weeks.

In some embodiments, the Immunoconjugate of Formula (I) is administered at a frequency of once every 3 weeks and a dose of 3 mg/kg, and the platinum-containing chemotherapeutic agent is administered at a frequency of once every 3 weeks and a dose of AUC 5 mg/ml/min; after 2 cycles, the Immunoconjugate of Formula (I) is administered at a frequency of once every 2 weeks and a dose of 4 mg/kg; wherein one cycle consists of 6 weeks.

In some embodiments, a method for treating non-small cell lung cancer is provided, the method comprises administering a combination therapy to a patient in need thereof, the combination therapy comprising: (a) an immunoconjugate represented by Formula (I); and (b) an EGFR-tyrosine kinase inhibitor; wherein the frequencies and doses of administration of the Immunoconjugate of Formula (I) and the EGFR-tyrosine kinase inhibitor are as described in any one of the above items.

In some embodiments, the non-small cell lung cancer is as described in any one of the above items.

In some embodiments, the non-small cell lung cancer is a non-small cell lung cancer with an EGFR mutation.

In some embodiments, the non-small cell lung cancer is a non-small cell lung cancer with a sensitive mutation of EGFR 19del or 21L858R.

In some embodiments, the non-small cell lung cancer is a non-small cell lung cancer with a sensitive mutation of EGFR 19del or 21L858R and has not received previous systemic treatment.

In some embodiments, the Immunoconjugate of Formula (I) is administered at a frequency of once every 1 to 5 weeks, and the EGFR-tyrosine kinase inhibitor is administered at a frequency of 3 times every day, twice every day, once every day, once every 2 days, or once every 3 days.

In some embodiments, the Immunoconjugate of Formula (I) is administered at a frequency of once every 1 to 5 weeks and a dose of 1 to 10 mg/kg, and the EGFR-tyrosine kinase inhibitor is administered at a frequency of 3 times every day, twice every day, once every day, once every 2 days, or once every 3 days and a dose of 30 to 150 mg.

In some embodiments, the Immunoconjugate of Formula (I) is administered at a frequency of once every 2 weeks, and the EGFR-tyrosine kinase inhibitor is administered at a frequency of once every day.

In some embodiments, the Immunoconjugate of Formula (I) is administered at a frequency of once every 2 weeks and a dose of 3 mg/kg, and the EGFR-tyrosine kinase inhibitor is administered at a frequency of once every day and a dose of 80 mg.

In some embodiments, the Immunoconjugate of Formula (I) is administered at a frequency of once every 2 weeks and a dose of 4 mg/kg, and the EGFR-tyrosine kinase inhibitor is administered at a frequency of once every day and a dose of 80 mg.

In some embodiments, the Immunoconjugate of Formula (I) is administered in a cycle of every week, every 1.5 weeks, every 2 weeks, every 3 weeks, every 4 weeks, every 5 weeks, every 6 weeks, every month, every two months or every quarter, and the EGFR-tyrosine kinase inhibitor is administered in a cycle of every day, every 2 days, every 3 days, every 4 days, every 5 days, every 6 days or every week.

In some embodiments, the Immunoconjugate of Formula (I) is administered in a 2-week cycle, and each is administered on Day 1 of each 2-week cycle, and the EGFR-tyrosine kinase inhibitor is administered every day.

In some embodiments:
(i) on Day 1 of a 2-week cycle, the Immunoconjugate of Formula (I) is administered at a dose of about 3 mg/kg;
(ii) osimertinib is administered at a dose of about 80 mg every day;
(iv) the number of cycles is 1 to 4.

In some embodiments:
(i) on Day 1 of a 2-week cycle, the Immunoconjugate of Formula (I) is administered at a dose of about 4 mg/kg;
(ii) osimertinib is administered at a dose of about 80 mg every day;
(iv) the number of cycles is 1 to 4.

In some embodiments:
(i) on Day 1 of a 2-week cycle, the Immunoconjugate of Formula (I) is administered at a dose of about 5 mg/kg;
(ii) osimertinib is administered at a dose of about 80 mg every day;
(iv) the number of cycles is 1 to 4.

In some embodiments, at least one of the therapeutic agents (the anti-PD-1 antibody, or binding fragment thereof, and the Immunoconjugate of Formula (I)) in the combination therapy is administered using the same dosage regimen (dose, frequency, and duration of treatment) that is typically employed when the agent is used as monotherapy for treating the same condition. In other embodiments, the patient receives a lower total amount of at least one of the therapeutic agents in the combination therapy than when the agent is used as monotherapy, *e.g*., smaller doses, less frequent doses, and/or shorter treatment duration.

### Additional Therapeutic Agents

The therapeutic combinations, and combination therapies disclosed herein may be used in combination with one or more other additional therapeutic agents, including but not limited to, additional anti-cancer agents, wherein said additional anti-cancer agents are used in the prevention, treatment, control, amelioration, or reduction of risk of a particular disease or condition (e.g., cellular proliferative disorders). In one embodiment, a therapeutic combination disclosed herein is combined with one or more additional anti-cancer agents for use in the prevention, treatment, control amelioration, or reduction of risk of cancer. Such additional therepeutic agents may be administered, by a route and in an amount commonly used therefor, contemporaneously or sequentially with a combination therapy of the present disclosure.

Accordingly, in one aspect, the present disclosure provides pharmaceutical compositions comprising: (a) the Immunoconjugate of Formula (I); (b) an anti-human PD-1 antibody (or antigen-binding fragment thereof); and (c) an additional therapeutic agent, or pharmaceutically acceptable salt thereof, and (d) a pharmaceutically acceptable carrier. In one embodiment, the amounts of (a), (b), and (c) present in the pharmaceutical composition are together effective to treat cancer.

In one embodiment, provided herein is a pharmaceutical composition comprising:
(a) an anti-human PD-1 antibody or antigen binding fragment thereof;
(b) a pharmaceutically acceptable carrier; and
(c) a plurality of immunoconjugates of Formula (I): 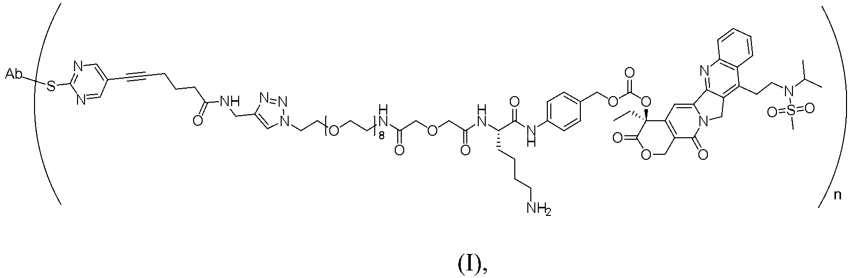
wherein:
Ab is an antibody that binds to Trop-2; and
n is a decimal from 0 to 10, and represents the average number of linker/payload moieties joined to each antibody for the plurality of Immunoconjugates of Formula (I);
wherein the amounts of (a) and (c) present in the composition are together effective to treat cancer.

In one embodment, for the pharmaceutical composition, the antibody is sacituzumab

In another embodiment, for the pharmaceutical composition, the anti-human PD-1 antibody or antigen binding fragment thereof is pembrolizumab.

Also provided herein are combination therapies comprising administering to a patient: a pharmaceutical composition comprising: (a) the Immunoconjugate of Formula (I); (b) an anti-human PD-1 antibody (or antigen-binding fragment thereof); and (c) an additional therapeutic agent, or pharmaceutically acceptable salt thereof. In one embodiment, the amounts administered of (a), (b), and (c) are together effective to treat cancer.

When co-administered with an additional therapeutic agent, the additional therapeutic agent can be administered in repeated cycles of 1, 2, 3, 4, 5, 6, 7, 8, 9, 10, 11, 12, 13, 14, 15, or 16 weeks. In one specific embodiment, the additional therapeutic agent is administered in three-week cycles. In another specific embodiment, the the additional therapeutic agent is administered in four-week cycles. In still another specific embodiment, the additional therapeutic agent is administered in six-week cycles. In certain embodiments, the the additional therapeutic agent is administered on one, two, three, four, five, six, or seven days of the cycle. The days of administration may be consecutive or may have one, two, three, four, five, or six days, one week, two weeks, three weeks, or four weeks, or any combination thereof, between them. In particular embodiments, the additional therapeutic agent is administered on Day 1 only of each cycle (e.g., a two-week cycle, a three-week cycle, or a 4-week cycle). In particular embodiments, the additional therapeutic agent is administered on Day 1 of a three-week cycle. In another specific embodiment, the additional therapeutic agent is administered on the first day of a 4-week cycle. In particular embodiments, the additional therapeutic agent is administered on Days 1, 8, and 15 of a three-week cycle, a four-week cycle or a six-week cycle.

The additional therapeutic agent(s) may be one or more agents selected from the group consisting of STING agonists, poly ADP ribose polymerase (PARP) inhibitors, mitogen-activated protein kinase (MEK) inhibitors, cyclin-dependent kinase (CDK) inhibitors, indoleamine 2,3-dioxygenase (IDO) inhibitors, tryptophan 2,3-dioxygenase (TDO) selective inhibitors, anti-viral compounds, antigens, adjuvants, anti-cancer agents, CTLA-4, LAG-3 and PD-1 pathway antagonists, lipids, liposomes, peptides, cytotoxic agents, chemotherapeutic agents, immunomodulatory cell lines, checkpoint inhibitors, vascular endothelial growth factor (VEGF) receptor inhibitors, topoisomerase II inhibitors, smoothen inhibitors, alkylating agents, anti-tumor antibiotics, anti-metabolites, retinoids, and immunomodulatory agents including but not limited to anti-cancer vaccines. It will be understood the descriptions of the above additional therepeutic agents may be overlapping. It will also be understood that the treatment combinations are subject to optimization, and it is understood that the best combination to use of the anti-human PD-1 antibody (or antigen-binding fragment thereof), the Immunoconjugate of Formula (I), and one or more additional therepeutic agents will be determined based on the individual patient needs.

When the therapeutic combination disclosed herein is used contemporaneously with one or more additional therapeutic agents, the anti-human PD-1 antibody (or antigen-binding fragment thereof) and/or the Immunoconjugate of Formula (I) may be administered either simultaneously with, or before or after, one or more additional therapeutic agent(s). Either of the anti-human PD-1 antibody (or antigen-binding fragment thereof) and/or the Immunoconjugate of Formula (I) may be administered separately, by the same or different route of administration, or together in the same pharmaceutical composition as the other agent(s).

The weight ratio of the anti-human PD-1 antibody (or antigen-binding fragment thereof) to the Immunoconjugate of Formula (I) may be varied and will depend upon the therapeutically effective dose of each agent. Generally, a therapeutically effective dose of each will be used. Combinations including at least one anti-human PD-1 antibody (or antigen-binding fragment thereof), an Immunoconjugate of Formula (I), and one or more additional therapeutic agents will generally include a therapeutically effective dose of each therapeutic agent. In such combinations, the anti-human PD-1 antibody (or antigen-binding fragment thereof) and/or the Immunoconjugate of Formula (I) disclosed herein, and additional therapeutic agents may be administered separately or in conjunction. In addition, the administration of one element may be prior to, concurrent with, or subsequent to the administration of additional therapeutic agent(s). In one embodiment, the combinations including at least one anti-human PD-1 antibody (or antigen-binding fragment thereof), an Immunoconjugate of Formula (I), and one or more additional therapeutic agents will comprise a dose of each component, such the the amounts administered are together effective to treat cancer.

In one embodiment, this disclosure provides an anti-human PD-1 antibody (or antigen-binding fragment thereof) and/or an Immunoconjugate of Formula (I), and at least one additional therapeutic agent as a combined preparation for simultaneous, separate or sequential use in therapy. In one embodiment, the therapy is the treatment of a cellular proliferative disorder, such as cancer.

The disclosure also provides the use of an Immunoconjugate of Formula (I) for treating a cellular proliferative disorder, where the patient has previously (*e.g*., within 24 hours) been treated with an anti-human PD-1 antibody (or antigen-binding fragment thereof). In one embodiment, the Immunoconjugate of Formula (I) is adminstered within 30 minutes after the administration of the anti-human PD-1 antibody (or antigen-binding fragment thereof) is complete. The disclosure also provides the use of an anti-human PD-1 antibody (or antigen-binding fragment thereof) for treating a cellular proliferative disorder, where the patient has previously (*e.g*., within 24 hours) been treated with an Immunoconjugate of Formula (I). In one embodiment, the anti-human PD-1 antibody (or antigen-binding fragment thereof) is adminstered within 30 minutes after the administration of the Immunoconjugate of Formula (I) is complete.

Anti-viral compounds that may be used in combination with the therapeutic combinations disclosed herein include hepatitis B virus (HBV) inhibitors, hepatitis C virus (HCV) protease inhibitors, HCV polymerase inhibitors, HCV NS4A inhibitors, HCV NS5A inhibitors, HCV NS5b inhibitors, and human immunodeficiency virus (HIV) inhibitors.

Antigens and adjuvants that may be used in combination with the therapeutic combinations disclosed herein include B7 costimulatory molecule, interleukin-2, interferon-γ, GM-CSF, CTLA-4 antagonists, OX-40/OX-40 ligand, CD40/CD40 ligand, sargramostim, levamisol, vaccinia virus, Bacille Calmette-Guerin (BCG), liposomes, alum, Freund's complete or incomplete adjuvant, detoxified endotoxins, mineral oils, surface active substances such as lipolecithin, pluronic polyols, polyanions, peptides, and oil or hydrocarbon emulsions. Adjuvants, such as aluminum hydroxide or aluminum phosphate, can be added to increase the ability of the vaccine to trigger, enhance, or prolong an immune response. Additional materials, such as cytokines, chemokines, and bacterial nucleic acid sequences, like CpG, a toll-like receptor (TLR) 9 agonist as well as additional agonists for TLR 2, TLR 4, TLR 5, TLR 7, TLR 8, TLR7/8, TLR9, and TLR 8/9 including lipoprotein, lipopolysaccharide (LPS), monophosphoryllipid A, lipoteichoic acid, imiquimod, resiquimod, and in addition retinoic acid-inducible gene I (RIG-I) agonists such as poly I:C, used separately or in combination are also potential adjuvants.

Chemotherapeutic agents that may be used in combination with the therapeutic combinations disclosed herein include abiraterone acetate, altretamine, anhydrovinblastine, aroplatin, asparaginase (also known as L-asparaginase, and Erwinia L-asparaginase), auristatin, bexarotene, bicalutamide, BMS 184476, 2,3,4,5,6-pentafluoro-N-(3-fluoro-4-methoxyphenyl)benzene sulfonamide, bleomycin, N,N-dimethyl-L-valyl-L-valyl-N-methyl-L-valyl-L-prolyl- 1-Lproline-t-butylamide, cachectin, cemadotin, chlorambucil, cyclophosphamide, 3',4'-didehydro-4'deoxy-8'-norvin-caleukoblastine, dinaciclib, docetaxol, doxetaxel, cyclophosphamide, carboplatin, carmustine, cisplatin, cryptophycin, cyclophosphamide, cytarabine, dacarbazine (DTIC), dactinomycin, daunorubicin, decitabine dolastatin, doxorubicin (adriamycin), etoposide, 5-fluorouracil, finasteride, flutamide, hydroxyurea and hydroxyurea andtaxanes, ifosfamide, liarozole, lonidamine, lomustine, MDV3100, mechlorethamine (nitrogen mustard), melphalan, mivobulin isethionate, rhizoxin, sertenef, streptozocin, mitomycin, methotrexate, taxanes, nilutamide, olaparib, onapristone, oxaliplatin, paclitaxel, prednimustine, procarbazine, RPR109881, selumetinib, stramustine phosphate, tamoxifen, tasonermin, taxol, tretinoin, vinblastine, vincristine, vindesine sulfate, and vinflunine, and pharmaceutically acceptable salts thereof.

Examples of vascular endothelial growth factor (VEGF) receptor inhibitors include, but are not limited to, bevacizumab (sold under the trademark AVASTIN by Genentech/Roche), axitinib (described in PCT International Patent Publication No. WO01/002369), Brivanib Alaninate ((S)-((R)-1-(4-(4-Fluoro-2-methyl-1H-indol-5-yloxy)-5-methylpyrrolo[2,1-f][1,2,4]triazin-6-yloxy)propan-2-yl)2-aminopropanoate, also known as BMS-582664), motesanib (N-(2,3-dihydro-3,3-dimethyl-1H-indol-6-yl)-2-[(4-pyridinylmethyl)amino]-3-pyridinecarboxamide. and described in International Patent Publication No. WO02/068470), pasireotide (also known as SO 230, and described in International Patent Publication No. WO02/010192), and sorafenib.

Examples of topoisomerase II inhibitors, include but are not limited to, etoposide, and teniposide.

Examples of alkylating agents, include but are not limited to, 5-azacytidine, decitabine, temozolomide, dactinomycin (also known as actinomycin-D, melphalan, altretamine, carmustine, bendamustine, busulfan, platinum-based chemotherapeutic agents (e.g., aroplatin, cisplatin, carboplatin, and oxaliplatin), lomustine, chlorambucil, cyclophosphamide, dacarbazine, altretamine, ifosfamide, procarbazine, mechlorethamine, streptozocin, thiotepa, and pharmaceutically acceptable salts thereof. In one embodiment, the additional thereapeutic agent is a platinum-based chemotherapeutic agent. In a specific embodiment, the additional therapeutic agent is cisplatin. In another specific embodiment, the additional therapeutic agent is carboplatin. In specific embodiments, when co-administered with carboplatin, the dose of carboplatin is from about AUC 1 mg/mL/min to AUC 10 mg/mL/min, administered intravenously. In a specific embodiment, when co-administered with carboplatin, the dose of carboplatin is AUC 5 mg/mL/min, administered intravenously. In other embodiments, when co-administered with cisplatin, the dose of cisplatin is from about 10 mg/m² to 150 mg/m², administered intravenously. In a specific embodiment, when co-administered with cisplatin, the dose of cisplatin is 75 mg/m², administered intravenously.

Examples of anti-tumor antibiotics include, but are not limited to, doxorubicin, bleomycin, daunorubicin, daunorubicin liposomal (daunorubicin citrate liposome), mitoxantrone, epirubicin, idarubicin, and mitomycin C.

Examples of anti-metabolites include, but are not limited to, claribine, 5-fluorouracil, 6-thioguanine, pemetrexed (sold under the tradename ALIMTA^{®}), cytarabine (also known as arabinosylcytosine (Ara-C)), cytarabine liposomal (also known as Liposomal Ara-C, sold under the tradename DEPOCYT^{™}), decitabine (sold under the tradename DACOGEN^{®}), hydroxyurea and fludarabine, floxuridine, cladribine (also known as 2-chlorodeoxyadenosine (2-CdA), methotrexate (also known as amethopterin, methotrexate sodium (MTX)), and pentostatin.

Examples of retinoids include, but are not limited to, alitretinoin, tretinoin, isotretinoin, and bexarotene.

In one embodiment, provided is a method treating a cellular proliferative disorder, said method comprising administering to a patient in need thereof a combination therapy that comprises: (a) an anti-human PD-1 antibody (or antigen-binding fragment thereof); (b) an Immunoconjugate of Formula (I); and (c) a platinum-based chemotherapeutic agent, wherein the anti-human PD-1 antibody (or antigen-binding fragment thereof), the Immunoconjugate of Formula (I), and the platinum-based chemotherapeutic agent are each administered on day 1 of a three-week cycle. ("Combination Regimen 3")

In a specific embodiment, for Combination Regimen 3, the anti-human PD-1 antibody (or antigen-binding fragment thereof) is administered at a dose of 200 mg per each 3-week cycle, and the Immunoconjugate of Formula (I) is administered at a dose of 5 mg/kg per each 3-week cycle.

In a specific embodiment, for Combination Regimen 3, the anti-human PD-1 antibody is pembrolizumab.

In a specific embodiment, for Combination Regimen 3, the Immunoconjugate of Formula (I) is Immunconjugate A.

In another specific embodiment, for Combination Regimen 3, the anti-human PD-1 antibody is pembrolizumab, and the Immunoconjugate of Formula (I) is Immunconjugate A.

In another specific embodiment, for Combination Regimen 3, platinum-based chemotherapeutic agent is cisplatin or carboplatin.

In another specific embodiment, for Combination Regimen 3, the anti-human PD-1 antibody is pembrolizumab, the Immunoconjugate of Formula (I) is Immunconjugate A, and the platinum-based chemotherapeutic agent is carboplatin.

In a specific embodiment, for Combination Regimen 3, the anti-human PD-1 antibody is pembrolizumab, the Immunoconjugate of Formula (I) is Immunconjugate A, and the platinum-based chemotherapeutic agent is cisplatin.

In another embodiment, Combination Regimen 3 is administered in from 1 to 4 cycles.

In one embodiment, provided is a method treating a cellular proliferative disorder, said method comprising administering to a patient in need thereof a combination therapy that comprises: (a) an anti-human PD-1 antibody (or antigen-binding fragment thereof); (b) an Immunoconjugate of Formula (I); and (c) a platinum-based chemotherapeutic agent, wherein the anti-human PD-1 antibody (or antigen-binding fragment thereof) is administered on day 1; the Immunoconjugate of Formula (I) is administered on days 1, 15, and 29; and the platinum-based chemotherapeutic agent is administered on days 1 and 22 of a six-week cycle ("Combination Regimen 4")

In a specific embodiment, for Combination Regimen 4 the anti-human PD-1 antibody (or antigen-binding fragment thereof) is administered at a dose of 400 mg per each 6-week cycle, and the Immunoconjugate of Formula (I) is administered at a dose of 5 mg/kg at each of days 1, 15, and 29 of each 6-week cycle.

In a specific embodiment, for Combination Regimen 4, the anti-human PD-1 antibody is pembrolizumab.

In a specific embodiment, for Combination Regimen 4, the Immunoconjugate of Formula (I) is Immunconjugate A.

In another specific embodiment, for Combination Regimen 4, the anti-human PD-1 antibody is pembrolizumab, and the Immunoconjugate of Formula (I) is Immunconjugate A.

In another specific embodiment, for Combination Regimen 4, the platinum-based chemotherapeutic agent is carboplatin or cisplatin.

In another specific embodiment, for Combination Regimen 4, the anti-human PD-1 antibody is pembrolizumab, the Immunoconjugate of Formula (I) is Immunconjugate A, and the platinum-based chemotherapeutic agent is carboplatin, which is administered at a dose of AUC 5 mg/mL/min for each dose administered.

In certain embodiments, for Combination Regimen 4, the anti-human PD-1 antibody is pembrolizumab, the Immunoconjugate of Formula (I) is Immunconjugate A, and the platinum-based chemotherapeutic agent is cisplatin, which is administered at a dose of 75 mg/m² for each dose administered.

In a specific embodiment, Combination Regimen 3 is administered as follows:
(i) the Immunoconjugate of Formula (I) is administered at a dose of about 5 mg/kg on day 1 of a 3-week cycle;
(ii) the anti-human PD-1 antibody or antigen binding fragment thereof is administered at a dose of about 200 mg on day 1 of a 3-week cycle;
(iii) carboplatin is administered at a dose of about AUC 5 mg/ml/min on day 1 of a 3-week cycle; and
(iv) the number of cycles is from 1 to 4.

In another specific embodiment, Combination Regimen 3 is administered as follows:
(i) the Immunoconjugate of Formula (I) is administered at a dose of about 5 mg/kg on day 1 of a 3-week cycle;
(ii) the anti-human PD-1 antibody or antigen binding fragment thereof is administered at a dose of about 200 mg on day 1 of a 3-week cycle;
(iii) cisplatin is administered at a dose of about 75 mg/m² on day 1 of a 3-week cycle; and
(iv) the number of cycles is from 1 to 4.

In one embodiment, Combination Regimen 3 is administered to a patient to treat cancer, wherein the cancer is non-small cell lung cancer.

In a specific embodiment, Combination Regimen 3 is administered to a patient to treat cancer, wherein the cancer is non-small cell lung cancer, and:
(a) the non-small cell lung cancer is without EGFR mutation, and ALK fusion genes; or
(b) the non-small cell lung cancer has an EGFR actionable mutation, and the patient has failed prior EGFR-TKI treatment.

In a specific embodiment, Combination Regimen 4 is administered as follows:
(i) the Immunoconjugate of Formula (I) is administered at a dose of about 5 mg/kg on days 1, 15, and 29 of a 6-week cycle;
(ii) the anti-human PD-1 antibody or antigen binding fragment thereof is administered at a dose of about 400 mg on day 1 of a 6-week cycle;
(iii) carboplatin is administered at a dose of about AUC 5 mg/ml/min on days 1 and 22 of a 6-week cycle; and
(iv) the number of cycles is from 1 to 4.

In another specific embodiment, Combination Regimen 4 is administered as follows:
(i) the Immunoconjugate of Formula (I) is administered at a dose of about 5 mg/kg on days 1, 15, and 29 of a 6-week cycle;
(ii) the anti-human PD-1 antibody or antigen binding fragment thereof is administered at a dose of about 400 mg on day 1 of a 6-week cycle;
(iii) cisplatin is administered at a dose of about 75 mg/m² on days 1 and 22 of a 6-week cycle; and
(iv) the number of cycles is from 1 to 4.

In one embodiment, Combination Regimen 4 is administered to a patient to treat cancer, wherein the cancer is non-small cell lung cancer.

In a specific embodiment, Combination Regimen 4 is administered to a patient to treat cancer, wherein the cancer is non-small cell lung cancer, and:
(a) the non-small cell lung cancer is without EGFR mutation, and ALK fusion genes; or
(b) the non-small cell lung cancer has an EGFR actionable mutation, and the patient has failed prior EGFR-TKI treatment.

### ADDITIONAL EMBODIMENTS

Additional embodiments of the disclosure include the compositions, combinations, uses and methods set forth in above, wherein it is to be understood that each embodiment may be combined with one or more other embodiments, to the extent that such a combination is consistent with the description of the embodiments. It is further to be understood that the embodiments provided above are understood to include all embodiments, including such embodiments as result from combinations of embodiments.

### Kits

The present disclosure also provides articles of manufacture, e.g., kits, comprising one or more containers (e.g., single-use or multi-use containers) containing a pharmaceutical composition of an Immunoconjugate of Formula (I) described herein at a dose described herein, a pharmaceutical composition of an anti-human PD-1 antibody (or antigen-binding fragment thereof) described herein at a dose described herein, optionally an additional therapeutic agent, and instructions for use according to a treatment regimen described herein. The Immunoconjugate of Formula (I), anti-human PD-1 antibody (or antigen-binding fragment thereof), and additional therapeutic agent can be packaged together or separately in suitable packing such as a vial or ampule made from non-reactive glass or plastic.

In one embodiment, the kit comprises means for separately retaining said compositions, such as a container, divided bottle, or divided foil packet. A kit of this disclosure may be used for administration of different dosage forms, for example, oral and parenteral, for administration of the separate compositions at different dosage intervals, or for titration of the separate compositions against one another. To assist with compliance, a kit of the disclosure typically comprises directions for administration.

In one embodiment, the present disclosure provides a kit comprising:
(a) an anti-human PD-1 antibody or antigen binding fragment thereof; and
(b) an Immunoconjugate of Formula (I): 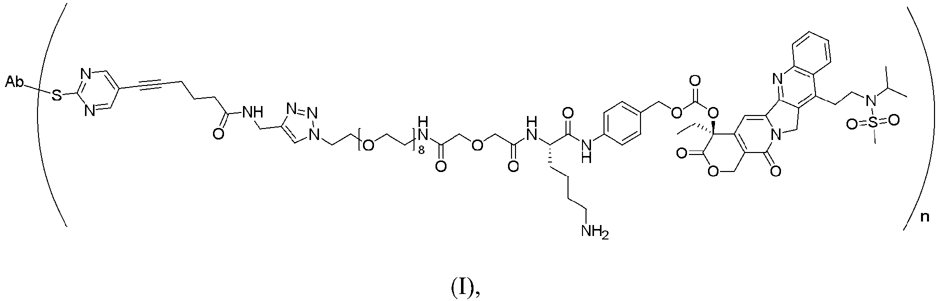 wherein:
   Ab is an antibody that binds to Trop-2; and
   n is an integer from 1 to 10;
   wherein the amounts of (a) and (b) present in the kit are together effective to treat cancer.

In one embodiment, for a kit of the present disclosure, the anti-human PD-1 antibody or antigen binding fragment contained in the kit is pembrolizumab.

In another embodiment, for a kit of the present disclosure, the Immunoconjugate of Formula (I) contained in the kit is Immunoconjugate A.

### GENERAL METHODS

Standard methods in molecular biology are described Sambrook, Fritsch and Maniatis (1982 & 1989 2nd Edition, 2001 3rd Edition) Molecular Cloning, A Laboratory Manual, Cold Spring Harbor Laboratory Press, Cold Spring Harbor, NY; Sambrook and Russell (2001) Molecular Cloning, 3rd ed., Cold Spring Harbor Laboratory Press, Cold Spring Harbor, NY; Wu (1993) Recombinant DNA, Vol. 217, Academic Press, San Diego, CA). Standard methods also appear in Ausbel, et al. (2001) Current Protocols in Molecular Biology, Vols.1-4, John Wiley and Sons, Inc. New York, NY, which describes cloning in bacterial cells and DNA mutagenesis (Vol. 1), cloning in mammalian cells and yeast (Vol. 2), glycoconjugates and protein expression (Vol. 3), and bioinformatics (Vol. 4).

Methods for protein purification including immunoprecipitation, chromatography, electrophoresis, centrifugation, and crystallization are described (Coligan, et al. (2000) Current Protocols in Protein Science, Vol. 1, John Wiley and Sons, Inc., New York). Chemical analysis, chemical modification, post-translational modification, production of fusion proteins, glycosylation of proteins are described *(see, e.g.,* Coligan, et al. (2000) Current Protocols in Protein Science, Vol. 2, John Wiley and Sons, Inc., New York; Ausubel, et al. (2001) Current Protocols in Molecular Biology, Vol. 3, John Wiley and Sons, Inc., NY, NY, pp. 16.0.5-16.22.17; Sigma-Aldrich, Co. (2001) Products for Life Science Research, St. Louis, MO; pp. 45-89; Amersham Pharmacia Biotech (2001) BioDirectory, Piscataway, N.J., pp. 384-391). Production, purification, and fragmentation of polyclonal and monoclonal antibodies are described (Coligan, et al. (2001) Current Protocols in Immunology, Vol. 1, John Wiley and Sons, Inc., New York; Harlow and Lane (1999) Using Antibodies, Cold Spring Harbor Laboratory Press, Cold Spring Harbor, NY; Harlow and Lane, *supra*). Standard techniques for characterizing ligand/receptor interactions are available *(see, e.g.,* Coligan, et al. (2001) Current Protocols in Immunology, Vol. 4, John Wiley, Inc., New York).

Monoclonal, polyclonal, and humanized antibodies can be prepared (*see, e.g.,* Sheperd and Dean (eds.) (2000) Monoclonal Antibodies, Oxford Univ. Press, New York, NY; Kontermann and Dubel (eds.) (2001) Antibody Engineering, Springer-Verlag, New York; Harlow and Lane (1988) Antibodies A Laboratory Manual, Cold Spring Harbor Laboratory Press, Cold Spring Harbor, NY, pp. 139-243; Carpenter, et al. (2000) J. Immunol. 165:6205; He, et al. (1998) J. Immunol. 160:1029; Tang et al. (1999) J. Biol. Chem. 274:27371-27378; Baca et al. (1997) J. Biol. Chem. 272:10678-10684; Chothia et al. (1989) Nature 342:877-883; Foote and Winter (1992) J. Mol. Biol. 224:487-499; U.S. Pat. No. 6,329,511).

An alternative to humanization is to use human antibody libraries displayed on phage or human antibody libraries in transgenic mice (Vaughan et al. (1996) Nature Biotechnol. 14:309-314; Barbas (1995) Nature Medicine 1:837-839; Mendez et al. (1997) Nature Genetics 15:146-156; Hoogenboom and Chames (2000) Immunol. Today 21:371-377; Barbas et al. (2001) Phage Display: A Laboratory Manual, Cold Spring Harbor Laboratory Press, Cold Spring Harbor, New York; Kay et al. (1996) Phage Display of Peptides and Proteins: A Laboratory Manual, Academic Press, San Diego, CA; de Bruin et al. (1999) Nature Biotechnol. 17:397-399).

Purification of antigen is not necessary for the generation of antibodies. Animals can be immunized with cells bearing the antigen of interest. Splenocytes can then be isolated from the immunized animals, and the splenocytes can fused with a myeloma cell line to produce a hybridoma *(see, e.g.,* Meyaard et al. (1997) Immunity 7:283-290; Wright et al. (2000) Immunity 13:233-242; Preston *et al., supra*; Kaithamana et al. (1999) J. Immunol. 63:5157-5164).

Methods for flow cytometry, including fluorescence activated cell sorting (FACS), are available *(see, e.g.,* Owens, et al. (1994) Flow Cytometry Principles for Clinical Laboratory Practice, John Wiley and Sons, Hoboken, NJ; Givan (2001) Flow Cytometry, 2nd ed.; Wiley-Liss, Hoboken, NJ; Shapiro (2003) Practical Flow Cytometry, John Wiley and Sons, Hoboken, NJ). Fluorescent reagents suitable for modifying nucleic acids, including nucleic acid primers and probes, polypeptides, and antibodies, for use, *e.g.,* as diagnostic reagents, are available (Molecular Probesy (2003) Catalogue, Molecular Probes, Inc., Eugene, OR; Sigma-Aldrich (2003) Catalogue, St. Louis, MO).

Standard methods of histology of the immune system are described *(see, e.g.,* Muller-Harmelink (ed.) (1986) Human Thymus: Histopathology and Pathology, Springer Verlag, New York, NY; Hiatt, et al. (2000) Color Atlas of Histology, Lippincott, Williams, and Wilkins, Phila, PA; Louis, et al. (2002) Basic Histology: Text and Atlas, McGraw-Hill, New York, NY).

Software packages and databases for determining, *e.g*., antigenic fragments, leader sequences, protein folding, functional domains, glycosylation sites, and sequence alignments, are available (*see, e.g*., GenBank, Vector NTI^{®} Suite (Informax, Inc., Bethesda, MD); GCG Wisconsin Package (Accelrys, Inc., San Diego, CA); DeCypher^{®} (TimeLogic Corp., Crystal Bay, Nevada); Menne, et al. (2000) Bioinformatics 16: 741-742; Menne, et al. (2000) Bioinformatics Applications Note 16:741-742; Wren, et al. (2002) Comput. Methods Programs Biomed. 68:177-181; von Heijne (1983) Eur. J. Biochem. 133:17-21; von Heijne (1986) Nucleic Acids Res. 14:4683-4690).

### EXAMPLES

The following examples illustrate representative embodiments of the present disclosure and are not meant to be limiting in any way.

### EXAMPLE 1

### Preparation of Immunoconjugate A

Immunoconjugate A can be made using the methodology described in US Patent Publication No. US Patent Publication No. 20200347075, wherein Immunoconjugate A is referred to as "BT001021".

### EXAMPLE 2

### Study of Immunoconjugate A and Pembrolizumab as

### Combination Therapy in Subjects with NSCLC

This study will consist of 3 periods, including the screening period, treatment period, and survival follow-up period, as follows:
- The screening period is up to 28 days prior to the first dose of study intervention.
- During the treatment period, subjects will receive Immunoconjugate A as monotherapy or in combination with osimertinib in 28-day cycle, and Immunoconjugate A in combination with pembrolizumab ± chemotherapy or Immunoconjugate A in combination with chemotherapy in 21- or 42-day cycle until any of the study intervention discontinuation criteria is met. Any subject who discontinues study intervention is required to complete the End of Treatment (EOT) visit within 30 days of the last dose of study intervention or before starting a new anticancer treatment, whichever occurs first, and the EOT visit should be close to 30 days after the last dose of study intervention.
- After the end of treatment, each subject will be followed for 30 days following
   cessation of all study intervention for adverse event monitoring regardless of the initiation of new anticancer treatment. Serious adverse event will be followed for 90 days following cessation of study intervention, or 30 days following cessation of study intervention if the subject initiates new anticancer therapy, whichever occurs first.
- Subjects will continue survival follow-up visits every 3 months (±14 days) after the last dose of study intervention by telephone for survival information and new anticancer treatment, until subject's decision to withdraw from the study, loss to follow-up, death, or end of the study, whichever occurs first. Subjects who discontinue the study intervention due to reasons other than disease progression will continue tumor assessment until disease progression, initiation of new anticancer treatment, subject's decision to withdraw from the study, loss to follow-up, or death, whichever occurs first.

It is estimated that the study will last approximately 3 years from the time the first subject (or his/her legally authorized representative) provides documented informed consent until the last subject's last visit.

### Intervention Groups and Duration:

A total of 6 cohorts are planned for the Immunoconjugate A/pembrolizumab combination study. Cohort 1 and 2 will be evaluated first. Dosing schedules of Cohort 3, 4, 5 and 6 will be determined based on the preliminary safety, efficacy, and PK data of Cohort 1 and 2.

The starting dose for the safety run-in period of Cohort 1 and 2 will be determined based on the Immunoconjugate A + pembrolizumab combination study in selected advanced solid tumors. It is estimated that safety run-in period of Cohort 1 and 2 in the current study will start at Immunoconjugate A 5 mg/kg Q2W or Q3W in combination with pembrolizumab. However, if this dose is not tolerated in Study Immunoconjugate A, then the next lower dose from that study (4 mg/kg Q2W or Q3W Immunoconjugate A), based on PK, efficacy and safety data, will be evaluated.

**Cohort 1 and 2:** enroll subjects with NSCLC in need of first line therapy; without actionable EGFR mutation, and ALK fusion genes; PD-L1 TPS ≥ 1%. Eligible subjects will be randomized 1:1 to either Cohort 1 or 2 to receive study treatment as below (Note: During the expansion period, subjects are planned to be randomized at 1:1 ratio to either of the cohorts, stratified by squamous cell carcinoma vs. non-squamous cell carcinoma and PD-L1 TPS 1%-49% vs. TPS ≥50%):
- **Cohort 1:** Immunoconjugate A (5 mg/kg, Q3W) + pembrolizumab (200 mg, Q3W), every 3 weeks as a cycle.
- **Cohort 2:** Immunoconjugate A (5 mg/kg, Q2W) + pembrolizumab (400 mg, Q6W), every 6 weeks as a cycle.

**Cohort 3 and 4:** enroll subjects with NSCLC in need of first line therapy; without actionable EGFR mutation, and ALK fusion genes. Eligible subjects will be randomized 1:1 to either Cohort 3 or 4 to receive study treatment below (Note: During the expansion period, subjects are planned to be randomized at 1:1 ratio to either of the cohorts, stratified by squamous cell carcinoma vs. non-squamous cell carcinoma and PD-L1 TPS < 1% vs. TPS 1%-49% vs. TPS ≥50%):
- **Cohort 3:** Immunoconjugate A + pembrolizumab + carboplatin (AUC 5 mg/mL/min, Q3W), every 3 weeks as a cycle for 4 cycles, or every 6 weeks as a cycle for 2 cycles, followed by Immunoconjugate A + pembrolizumab.
- **Cohort 4:** Immunoconjugate A + pembrolizumab + cisplatin (75 mg/m², Q3W), every 3 weeks as a cycle for 4 cycles, or every 6 weeks as a cycle for 2 cycles, followed by Immunoconjugate A + pembrolizumab.

Note: The frequency and dosage of Immunoconjugate A and pembrolizumab in Cohort 3 and 4 will be determined based on Cohort 1 and 2 data. It is expected that the safety run-in period of Cohort 3 and 4 will start at Immunoconjugate A 4 mg/kg Q2W or Q3W (one dose level lower than that deemed to be tolerated in Cohort 1 and 2) in combination with pembrolizumab plus carboplatin or cisplatin, if the dose 5 mg/kg Q2W or Q3W is tolerated in Cohort 1 and 2.

**Cohort 5 and 6:** enroll NSCLC subjects with EGFR actionable mutation who failed prior EGFR-TKI treatment. Eligible subjects will be randomized 1:1 to either Cohort 5 or 6, stratified by previously experienced first or second-generation EGFR-TKI only vs. third generation EGFR-TKI and PD-L1 TPS < 1% vs. TPS 1%-49% vs. TPS≥ 50%. The dose of Immunoconjugate A and platinum used in Cohort 5 and 6 will be based on Cohort 3 and 4 data.
- **Cohort 5:** Immunoconjugate A + pembrolizumab + carboplatin or cisplatin, every 3 weeks as a cycle for 4 cycles, or every 6 weeks as a cycle for 2 cycles, followed by Immunoconjugate A + pembrolizumab.
- **Cohort 6:** Immunoconjugate A + carboplatin or cisplatin, every 3 weeks as a cycle for 4 cycles, or every 6 weeks as a cycle for 2 cycles, followed by Immunoconjugate A.

**Immunoconjugate A:** Immunoconjugate A will be administered intravenously on Day 1 of each cycle with every 3 weeks as a cycle (Cohort 1; and may further be applied to Cohort 3, 4, 5, and 6 if determined by SRC) or Day 1, 15, and 29 of each cycle with every 6 weeks as a cycle (Cohort 2; and may further be applied to Cohort 3, 4, 5, and 6). The first infusion duration for each subject will be 90 (±15) minutes and infusion-related adverse effects (AEs) will be monitored. In the absence of infusion-related AEs, the subsequent infusion duration may be adjusted to 60 (+15) minutes. In the event of infusion-related AEs or to ensure the infusion safety of the subject, the investigator may adjust the infusion duration to 90 (±15) minutes.

**Pembrolizumab:** 200 mg pembrolizumab will be administered intravenously over 30 minutes on Day 1 of each cycle with every 3 weeks as a cycle (Cohort 1; and may further be applied to Cohort 3, 4, 5, and 6), or 400 mg pembrolizumab will be administered intravenously over 30 minutes on Day 1 of each cycle with every 6 weeks as a cycle (Cohort 2; and may further be applied to Cohort 3, 4, 5, and 6).

**Carboplatin:** AUC 5 mg/mL/min carboplatin will be administered intravenously on Day 1 of each cycle with every 3 weeks as a cycle; or Day 1 and 22 of each cycle with every 6 weeks as a cycle. Cohort 3 carboplatin cycle calculation will be in line with that for Immunoconjugate A and pembrolizumab in the determined Cohort (either Cohort 1 or 2). Regardless of the cycle calculation, carboplatin will be given every 3 weeks, in total 4 doses for 12 weeks.

**Cisplatin:** 75 mg/M² cisplatin will be administered intravenously on Day 1 of each cycle with every 3 weeks as a cycle; or Day 1 and 22 of each cycle with every 6 weeks as a cycle. Cohort 3 cisplatin cycle calculation will be in line with that for Immunoconjugate A and pembrolizumab in the determined Cohort (either Cohort 1 or 2). Regardless of the cycle calculation, cisplatin will be given every 3 weeks, in total 4 doses for 12 weeks.

### Co-administration sequence:

Study intervention will be administered sequentially when pembrolizumab, Immunoconjugate A and chemotherapy are given on the same day. Pembrolizumab is administered as intravenous infusion firstly, followed by intravenous infusion of Immunoconjugate A with an interval of at least 30 minutes, followed by intravenous infusion of carboplatin or cisplatin when combined with chemotherapy with an interval of at least 30 minutes (the maximum interval between Immunoconjugate A and chemotherapy is only permitted +1 day).

Study intervention will be administered sequentially when Immunoconjugate A and chemotherapy are given on the same day. Immunoconjugate A is administered as intravenous infusion firstly, followed by intravenous infusion of chemotherapy with an interval of at least 30 minutes.

Subjects will be permitted to continue the study treatment beyond initial RECIST 1.1 defined disease progression if Investigator assesses that the subject can still receive clinical benefit. Clinical benefit may be concluded provided that there is no occurrence of intolerable toxicity or worsened symptoms due to progressive disease (PD) as assessed by the investigator based on radiographic manifestations and clinical conditions. Subjects will be re-consented with an informed consent form (ICF) describing any reasonably foreseeable risks or discomforts.

### Dosage and Administration

**Infusion Duration:** Immunoconjugate A will be administered to each subject within 90 (±15) minutes at the first infusion, and the infusion-related AEs will be monitored. In the absence of infusion-related AEs, the subsequent infusion duration may be adjusted to 60 (+15) minutes. In the event of infusion-related AEs or to ensure the infusion safety of the study drug, the investigator may adjust the infusion duration to 90 (±15) minutes.

**Pembrolizumab** infusion will be administered over 30 minutes.

**Immunoconjugate** A will be administered following the pembrolizumab infusion with an interval of at least 30 minutes when pembrolizumab and Immunoconjugate A are given on the same day. When Immunoconjugate A is used in combination with pembrolizumab and chemotherapy, firstly, pembrolizumab will be administered via intravenous infusion, followed by intravenous infusion of Immunoconjugate A after at least 30 minutes interval, followed by intravenous infusion of carboplatin or cisplatin after at least 30 minutes interval (the maximum interval between Immunoconjugate A and chemotherapy is only permitted +1 day).

When Immunoconjugate A and chemotherapy (cisplatin or carboplatin) are given on the same day, Immunoconjugate A is administered via intravenous infusion firstly, followed by intravenous infusion of chemotherapy after an interval of at least 30 minutes.

**Adverse Reaction Monitoring:** subjects will be monitored for adverse reactions for at least 90 minutes following the completion of all infusions on Cycle 1, Day 1. If no infusion-related AEs occur, subjects will be monitored for adverse reactions for at least 60 minutes after completing subsequent infusions of study interventions, unless close monitoring is required as clinically indicated.

**Premedication for Immunoconjugate A infusion-related reaction:** Premedication for Immunoconjugate A will be permitted following pembrolizumab infusion completion and within 30 to 60 minutes prior to Immunoconjugate A infusion. Subjects are recommended to be premedicated with diphenhydramine (or equivalent dose of antihistamine) and acetaminophen (or equivalent dose of analgesic) ± other pretreatment regimens recommended by the Investigator per institutional standards or local guidelines.

The study consists of two parts. Part 1 is safety run-in period, for dose level evaluation; and Part 2 is expansion period, for further exploring the efficacy and safety based on the safe and tolerable dose range determined in Part 1.

### End of Study Definition

The study will be ended when the following conditions are met:
All the subjects have completed or discontinued from the study intervention and the Sponsor considers that the survival benefit data obtained from this study are mature.

The primary analysis has been completed and the Sponsor has initiated a separate extension study to ensure that subjects on study treatment and continue to benefit from study drugs can continue to receive treatment.

### Study Population

Prospective approval of protocol deviations from recruitment and enrollment criteria (also known as protocol waivers or exemptions) is not permitted. The study population is subjects with advanced or metastatic non-small cell lung cancer. Subjects must be able to provide written informed consent and meet all inclusion criteria and none of the exclusion criteria.

### Inclusion Criteria

Subjects are eligible to be included in the study only if all of the following criteria apply:
1. Subjects must be at least 18 years of age on day of signing informed consent, regardless of gender;
2. Subjects with histologically or cytologically confirmed NSCLC, locally advanced (stage IIIB/IIIC) or metastatic (Stage IV) NSCLC not amenable to receive definitive surgery and/or radiotherapy (with or without concurrent chemotherapy), according to TNM staging of lung cancer published by the International Union Against Cancer and American Joint Committee on Cancer, 8th edition;
3. Subjects for cohort 1, 2, 3 and 4 with non-squamous or mixed histology NSCLC should be confirmed by tumor histology to be EGFR wild-type and ALK fusion gene negative; without known alterations of driver genes such as ROS1, NTRK, and BRAF; without actionable genomic alterations for other approved targetable therapies. For subjects with squamous NSCLC, if the prior EGFR and ALK status is unknown, the corresponding tests are not required prior to enrollment in this study and the gene status will be considered negative.
Subjects for cohort 5 and 6, should be confirmed by tumor histology, cytology or blood sample to harbor EGFR actionable genomic mutation;
4. The study will include the following 6 cohorts:
a) **Cohort 1 and Cohort 2:** locally advanced or metastatic NSCLC subjects without actionable EGFR mutations and ALK fusion genes, without known ROS1, NTRK, BRAF gene alterations or other actionable gene mutation kinases with approved therapies, meeting all of the following criteria: i) no prior systemic treatment; ii) no prior treatment with PD-1/PD-L1 antibodies; iii) PD-L1 TPS ≥ 1% as determined by PD-L1 IHC 22C3 pharmDx assay by central test. Subjects who have received prior adjuvant, neoadjuvant therapy or definitive chemoradiotherapy are permitted to be enrolled if the disease progression occurred > 6 months after completing treatment;
b) **Cohort 3 and cohort 4:** locally advanced or metastatic NSCLC subjects without actionable EGFR mutations and ALK fusion genes, known ROS1, NTRK, BRAF gene alterations, or other actionable gene mutation kinases with approved therapies, who have not received prior systemic treatment including prior treatment with PD-1/PD-L1 antibodies. Subjects who had received prior adjuvant, neoadjuvant therapy or definitive chemoradiotherapy are permitted to be enrolled if the disease progression occurred > 6 months after completing treatment;
c) **Cohort 5 and Cohort 6:** subjects with EGFR actionable mutation including exon 19 deletion mutation or exon 21 point mutation (L858R) who failed prior EGFR-TKI treatment, meeting all of the following criteria: i) failed prior first or second generation EGFR-TKIs with confirmation of negative T790M mutation in exon 20 by tumor histology after treatment failure; or failed third generation EGFR-TKI treatment regardless of T790M mutation status; ii) no prior chemotherapy or PD-1/PD-L1 antibody treatment for locally advanced or metastatic NSCLC; Subjects who had received prior adjuvant, neoadjuvant therapy or definitive chemoradiotherapy are permitted to be enrolled if the disease progression occurred > 6 months after completing treatment;

5. Subjects are able to provide tumor blocks or slides before the first dose of study intervention (for archival tumor tissue: formalin-fixed paraffin-embedded tissue blocks or approximately 10 unstained slides for cohort 1, 2, 3, 4 and 5 or 5 unstained slides for cohort 6).
a) If less than 10 unstained slides (cohort 1, 2, 3, 4 and 6) or 5 unstained slides (cohort 5) are provided, the decision to enroll the subject may be made on a case-by-case basis after discussion with the Medical Monitor.
b) Subjects who are unable to provide archival tumor blocks or slides are required to agree to provide a fresh biopsy prior to receiving study intervention;

6. Subject must have at least one radiographically measurable lesion as per Response Evaluation Criteria in Solid Tumors (RECIST) 1.1 criteria; brain metastatic lesion(s) will not be selected as target lesion;
7. Subject has an ECOG performance status of either 0 or 1 as assessed within 7 days before initiation of study intervention;
8. Life expectancy at least 3 months for the subject;
9. Adequate organ function as indicated by the following laboratory values (without receiving blood transfusion, recombinant human thrombopoietin or colony stimulating factor treatment within 2 weeks before screening) (Table 3-4):

**Table 3-4 Adequate Organ Function Laboratory Values**

| **System** | **Laboratory Value** |
|---|---|
| **Hematological** | |
| Absolute neutrophil count (ANC) | ≥ 1.5x10⁹/L |
| Platelets | ≥ 100x10⁹/L |
| Hemoglobin | ≥ 9 g/dL |

| **Renal** | |
|---|---|
| Calculated creatinine clearance (CrCl)^{a} (GFR by Chronic Kidney Disease Epidemiology | ≥ 60 mL/min for subjects to be enrolled in Cohort 3, 4, 5, and 6. |
| Collaboration [CKD-EPI] or Modification of Diet in Renal Disease (MDRD) formulas can also be used in place of CrCl) | ≥ 60 mL/min for other subjects. |

| **Hepatic** | |
|---|---|
| Total bilirubin | ≤ 1.5 x ULN |
| AST (SGOT), ALT (SGPT) and ALP | ≤ 2.5 x ULN; |
| | AST and ALT ≤ 5 x ULN for subjects with liver metastases; |
| | ALP≤ 5 x ULN for subjects with liver and/or bone metastases |
| Serum albumin | ≥ 30 g/L |

| **Coagulation** | |
|---|---|
| International Normalized Ratio (INR), Activated | ≤ 1.5 x ULN |
| Partial Thromboplastin Time (aPTT) and Prothrombin Time (PT) | |
| ALT (SGPT) =alanine aminotransferase (serum glutamic pyruvic transaminase); AST (SGOT) =aspartate aminotransferase (serum glutamic oxaloacetic transaminase); ALP= alkaline phosphatase; GFR=glomerular filtration rate; ULN=upper limit of normal | |
| ^{a}Creatinine Clearance should be calculated using the Cockcroft-Gault Method. | |

10. Left ventricular ejection fraction (LVEF)≥ 50% by echocardiogram (ECHO) or multiple gated acquisition (MUGA) scan;
11. Subjects must have recovered from all toxicities led by prior treatment (recover to < Grade 2 based on CTCAE 5.0 criteria, or to levels defined in the eligibility criteria) with the exception of toxicities not considered a safety risk (e.g. alopecia, vitiligo, and other asymptomatic laboratory abnormalities);
12. Contraceptive methods used by male and female subjects must comply with contraceptive methods of local regulations for clinical study subjects;
Note: the reliability of abstinence in male and/or female eligibility needs to be assessed based on the duration of the clinical study and the preferred and usual lifestyle of the subject. Periodic abstinence (e.g., calendar, ovulation, sympto-thermal, and post-ovulation methods) and withdrawal are not acceptable methods of contraception.
a. Male subjects:
   - Male subjects must agree to use highly effective contraception from signing the informed consent form (ICF) until at least 6 months after the last dose of study intervention, and must not donate sperm during this period.
b. Female subjects:
   - Female subjects eligible to participate in the study should be non-pregnant, not breastfeeding, and meet at least 1 of the following conditions:
      ∘ Not a woman of childbearing potential (WOCBP)
         or
      ∘ A WOCBP who agrees to followcontraceptive guidelines from signing the ICF until at least 6 months after the last dose of study intervention; and

13. Subjects should voluntarily participate in the study, sign the ICF, and will be able to comply with the protocol-specified visits and relevant procedures.

### Exclusion Criteria

Subjects who meet any of the following criteria may not be enrolled in the study:

### Medical Conditions

1. Subjects with mixed SCLC histopathological features;
2. Subjects with a known history of prior malignancy unless the subject has undergone potentially curative therapy with no evidence of that disease recurrence for 3 years since initiation of that therapy;
   Note: The time requirement for no evidence of disease for 3 years does not apply to the NSCLC tumor under investigation by the study. The time requirement also does not apply to subjects who underwent successful definitive resection of basal cell carcinoma of the skin, superficial bladder cancer, squamous cell carcinoma of the skin, in situ cervical cancer, or other in situ cancers;
3. Subjects with known meningeal metastases, brainstem metastases, spinal cord metastases and/or compression, or active CNS metastases. Subjects with locally treated brain metastases may participate provided they are clinically stable for at least 4 weeks, and without requirement of steroid treatment for at least 14 days prior to first dose of study treatment; Stable brain metastases by this definition should be established prior to the first dose of study medication. For those subjects in whom CNS metastases are firstly discovered at the time of screening, the treating Investigator should consider delay of study treatment to document stability of CNS metastases with repeat imaging at least 4 weeks later (in which case, repeat of all screening activity may be required);
4. Subjects with ≥ Grade 2 peripheral neuropathy who are to receive platinum treatment in this study;
5. Subjects with history of esophagogastric varices, severe ulcers, gastrointestinal perforation, gastrointestinal obstruction, intra-abdominal abscess, or acute gastro-intestinal bleeding within 6 months prior to the first dose of study intervention;
6. Subjects who had arteriovenous thromboembolic events, such as cerebrovascular accident (including transient ischemic attack), deep vein thrombosis (except for venous thrombosis caused by venous catheterization in prior chemotherapy that have resolved as judged by the Investigator) and pulmonary embolism within 6 months prior to the first dose of study intervention;
7. Subjects with active inflammatory bowel disease or previous clear history of inflammatory bowel disease (e.g., Crohn's disease, ulcerative colitis, or chronic diarrhea);
8. Subjects who suffer from cardiovascular diseases of clinical significance, such as:
   a) Subjects who had serious or uncontrolled cardiac disease or clinical symptoms requiring treatment within 6 months prior to the first dose of the study intervention, including: congestive heart failure classified as Class III to IV by New York Heart Association (NYHA), unstable angina that cannot be controlled by medication, serious arrhythmia requiring medical treatment (except for atrial fibrillation or paroxysmal supraventricular tachycardia), myocardial infarction;
   b) Subjects with previous history of myocarditis, or cardiomyopathy; and
   c) Subjects with QTc interval > 480 ms at baseline;
9. Subjects with a history of interstitial lung disease (ILD)/non-infectious pneumonitis that required steroids, or has current ILD/pneumonitis, or where suspected ILD/pneumonitis cannot be ruled out by imaging at screening;
10. Subjects with uncontrolled systemic disease as judged by the Investigator:
   a) Subjects with uncontrolled hypertension under medication intervention (systolic blood pressure > 160 mmHg and/or diastolic blood pressure > 100 mmHg), history of unstable hypertension, or history of poorly compliant anti-hypertension treatment;
   b) Subjects with uncontrolled diabetes under medication intervention (fasting glucose ≥ 10 mmol/L and/or HbA1c ≥ 8%);
   c) Subjects with pleural effusion, pericardial effusion, or ascites that is clinically symptomatic or requires repeated drainage;
11. Subjects with active autoimmune disease that required systemic treatment in the past 2 years (e.g., use of disease symptom relieving agents, corticosteroids or immunosuppressive drugs). Hormone replacement therapy (e.g., thyroxine, insulin, or physiologic corticosteroid replacement therapy for adrenal or pituitary insufficiency) is not considered a form of systemic treatment and is permitted;
12. Subjects with active hepatitis B or hepatitis C.
   Note: for subjects with hepatitis B surface antigen (HBsAg) positive and/or hepatitis B core antibody (HBcAb) positive, HBV-DNA test is required; HBV-DNA ≥ 500 IU/mL or above the lower limit of detection (if the lower limit is higher than 500 IU/mL) is considered as active hepatitis B. Active hepatitis C is defiend as hepatitis C antibody positive and HCV-RNA above the lower limit of detection;
13. Subjects have known human immunodeficiency virus (HIV) infection that is not well controlled. This includes HIV-infected participants with a history of Kaposi's sarcoma and/or Multicentric Castleman's Disease. HIV-infected participants must have well controlled HIV on Antiretroviral therapy (ART) meeting all of the criteria below:
   a) Participants on ART must have a CD4+ T cell count >350 cells/mm3 at the time of screening;
   b) Participants on ART must have achieved and maintained virologic suppression defined as confirmed HIV RNA level below 50 or the LLOQ (below the limit of detection) using the locally available assay at the time of screening and for at least 12 weeks before screening; and
   c) Participants on ART must have been on a stable regimen, without changes in drugs or dose modification, for at least 4 weeks before study entry (Day 1).
14. Subjects with known active tuberculosis:
15. Subjects who have received any chemotherapy, radiotherapy, immunotherapy, or biologic therapy within 4 weeks before the first dose of study intervention; received small molecule TKIs, anti-tumor hormone therapy, systemic immune stimulators (including but not limited to interferon, and IL-2), or traditional Chinese medicine preparations approved for anti-tumor indications within 2 weeks prior to the first dose of study intervention; palliative radiation to known metastatic sites within 2 weeks prior to the first dose of study intervention;
   Note: Subjects who have previously received prior anti-PD-1, anti-PD-L1 antibodies are permitted to enroll into the corresponding cohort of the study if they meet the following conditions:
      - Must not have experienced a toxicity that led to permanent discontinuation of prior immunotherapy (IMT).
      - All AEs while receiving prior IMT must have resolved to ≤ Grade 1 or baseline prior to screening for this study.
      - Must not have experienced a ≥ Grade 3 irAE or neurologic or ocular AE of any grade while receiving prior IMT.
   Note: Subjects with endocrine AE of any grade are permitted to enroll if they are stably maintained on appropriate replacement therapy and are asymptomatic.
      - Must not have required the use of additional immunosuppression other than corticosteroids for the management of an AE, not have experienced recurrence of an AE if re-challenged, and not currently require maintenance doses of > 10 mg prednisone or equivalent per day;
16. Subjects who have received other clinical investigational drugs or major surgery within 4 weeks prior to the first dose of study intervention;
17. Subjects who have received radiation therapy to lung that is more than 30 Gy within 6 months prior to the first dose of study intervention;
18. Subjects who required the systemic use of strong inhibitors or inducers of cytochrome P450 3A4 (CYP3A4) within 2 weeks or 5 half-life periods prior to the first dose of study intervention and during the study;
19. Subjects who have received continuous high-dose systemic corticosteroids within 2 weeks prior to the first dose of study intervention (low-dose corticosteroids, such as ≤ 10 mg/day prednisone or equivalent, are permitted if the dose is stable for at least 4 weeks), or other immunosuppressive therapy;
20. Subjects who have received a live or live-attenuated vaccine within 4 weeks prior to the first dose of study intervention, or are scheduled to receive live vaccines during the study;
21. Subjects with serious infections within 4 weeks prior to the first dose of study intervention (including but not limited to comorbidity, sepsis or severe pneumonia that require hospitalization) or concomitant infections requiring systemic antibiotic treatment within 2 weeks prior to the first dose of study intervention;
22. Subjects with known history of allogeneic organ transplantation and allogeneic hematopoietic stem cell transplantation;
23. Known allergy or hypersensitivity to pembrolizumab or Immunoconjugate A, or the excipients of pembrolizumab or Immunoconjugate A.
24. Is pregnant or breastfeeding, or expecting to conceive or father children within the projected duration of the study;
25. Subjects whose condition deteriorated rapidly, such as severe changes in performance status, during the screening process prior to the first dose of study intervention; and
26. Subjects with other circumstances that, in the opinion of the Investigator, are not appropriate for participation in this study.

### EXAMPLE 3

### Study on combination of Immunoconjugate A and various drugs for treatment of NSCLC

The patient groups and dosing regimens of various cohorts in this study are as follows:
Cohort 1: EGFR wild-type and ALK fusion gene-negative first-line NSCLC subjects were enrolled, PD-L1 TPS ≥ 1%. Immunoconjugate A + pembrolizumab were administered to the subject; the dose of Immunoconjugate A was 4 mg/kg based on the patient's body weight, once every 2 weeks; the dose of pembrolizumab was 400 mg, once every 6 weeks.

Cohort 3: EGFR wild-type first-line NSCLC subjects were enrolled. In the first phase, Immunoconjugate A + pembrolizumab + carboplatin were administered to the subjects. The dose of Immunoconjugate A was 2.25 mg/kg based on the patient's body weight, twice every 3 weeks, on Days 1 and 8 of every 3 weeks; the dose of pembrolizumab was 400 mg, once every 6 weeks; carboplatin was administered once every 3 weeks at a dose to achieve AUC 5 mg/ml/min; the duration of the first phase was 6 to 24 weeks (e.g., 6, 12, 18 or 24 weeks); after the first phase, the subjects entered the second phase, Immunoconjugate A + pembrolizumab. Immunoconjugate A were administered to the subjects at a dose of 4 mg/kg based on the patient's body weight, once every 2 weeks; pembrolizumab was administered at a dose of 400 mg, once every 6 weeks. Some subjects in Cohort 3 had squamous cell carcinoma and some had non-squamous cell carcinoma; Cohort 3 included subjects with PD-L1 TPS < 1%, subjects with TPS 1% to 49%, and subjects with TPS ≥ 50%.

Cohort 4: EGFR wild-type first-line NSCLC subjects were enrolled. In the first phase, Immunoconjugate A + pembrolizumab + carboplatin were administered to the subject. The dose of Immunoconjugate A was 3 mg/kg based on the patient's body weight, once every 3 weeks; the dose of pembrolizumab was 400 mg, once every 6 weeks; carboplatin was administered once every 3 weeks at a dose to achieve AUC 5 mg/ml/min. The duration of the first phase was 6 to 24 weeks (e.g., 6, 12, 18 or 24 weeks). After the first phase, the subjects entered the second phase, Immunoconjugate A + pembrolizumab were administered. The dose of Immunoconjugate A was 4 mg/kg based on the patient's body weight, once every 2 weeks; the dose of pembrolizumab was 400 mg, once every 6 weeks. Some subjects in Cohort 4 had squamous cell carcinoma and some had non-squamous cell carcinoma; Cohort 4 included subjects with PD-L1 TPS < 1%, subjects with TPS 1% to 49%, and subjects with TPS ≥ 50%.

Cohort 5: NSCLC subjects with EGFR mutation who had failed prior EGFR-TKI treatment were enrolled. In the first phase, Immunoconjugate A + carboplatin were administered to the subject. The dose of Immunoconjugate A was 2.25 mg/kg based on the patient's body weight, twice every 3 weeks; carboplatin was administered once every 3 weeks at a dose to achieve AUC 5 mg/ml/min; and the duration of the first phase was 6 to 24 weeks (e.g., 6, 12, 18 or 24 weeks). After the first phase, the subjects entered the second phase, Immunoconjugate A monotherapy was administered. The dose of Immunoconjugate A was 4 mg/kg based on the patient's body weight, once every 2 weeks. Cohort 5 included subjects who were previously treated with only first- or second-generation EGFR TKIs, as well as subjects who were treated with third-generation EGFR TKIs. Cohort 5 included subjects with central nervous system metastases, as well as subjects without central nervous system metastases.

Cohort 6: NSCLC subjects with EGFR mutations who had failed prior EGFR-TKI treatment were enrolled. In the first phase, Immunoconjugate A + carboplatin were administered to the subject. The dose of Immunoconjugate A was 3 mg/kg based on the patient's body weight, once every 3 weeks; carboplatin was administered once every 3 weeks at a dose to achieve AUC 5 mg/ml/min; and the duration of the first phase was 6 to 24 weeks (e.g., 6, 12, 18 or 24 weeks). After the first phase, the subjects entered the second phase, Immunoconjugate A monotherapy was administered to the subject. The dose of Immunoconjugate A was 4 mg/kg based on the patient's body weight, once every 2 weeks. Cohort 6 included subjects who were previously treated with only first- or second-generation EGFR TKIs, as well as subjects who were treated with third-generation EGFR TKIs. Cohort 6 included subjects with central nervous system metastases, as well as subjects without central nervous system metastases.

Cohort 7: NSCLC subjects with EGFR mutations were enrolled. Immunoconjugate A + Osimertinib were administered to the subject. The dose of Immunoconjugate A was 4 mg/kg based on patient body weight, once every 2 weeks; the dose of osimertinib was 80 mg, once daily. Cohort 7 included subjects with 19 del mutation and/or 21 L858R mutation; Cohort 7 included subjects with central nervous system metastases, as well as subjects without central nervous system metastases.

Cohort 7-1: NSCLC subjects with EGFR mutations were enrolled. Immunoconjugate A + Osimertinib were administered to the subject. The dose of Immunoconjugate A was 3 mg/kg based on patient body weight, once every 2 weeks; the dose of osimertinib was 80 mg, once daily. Cohort 7-1 included subjects with 19 del mutation and/or 21 L858R mutation; Cohort 7-1 included subjects with central nervous system metastases, as well as subjects without central nervous system metastases.

The inclusion criteria for this study were:
1. Subjects must be at least 18 years of age on day of signing informed consent, regardless of gender;
2. Subjects with histologically or cytologically confirmed NSCLC, locally advanced or metastatic (Stage IV) NSCLC not amenable to receive radical surgery and/or radical radiotherapy;
3. Histologically confirmed EGFR wild-type or EGFR-activating mutations confirmed by tumor histology, cytology, or hematological samples;
4. a) EGFR wild-type and ALK fusion gene-negative, with locally advanced or metastatic NSCLC without prior systemic therapy; c) EGFR-sensitive mutations, refractory to prior EGFR-TKI therapy, and progression following both anti-PD-1/PD-L1 immunotherapy and platinum-based chemotherapy; d) EGFR-sensitive mutations, either untreated with prior systemic therapy or having progressed on prior EGFR-TKI therapy;
5. Patients must provide tumor tissue blocks or sections prior to the first dose of the study treatment;
6. At least one radiologically measurable lesion according to the Response Evaluation Criteria in Solid Tumors (RECIST) v1.1;
7. Eastern Cooperative Oncology Group (ECOG) performance status score of 0 or 1 within 7 days before the first dose of the study treatment;
8. Expected survival of ≥ 3 months;
9. Adequate organ function;
10. Patients must had recovered from all toxicities caused by prior therapies.
11. Contraceptive measures used by male and female subjects must comply with local regulatory requirements for contraception in clinical trial participants.
12. Subjects voluntarily participated in the study, signed the informed consent form, and were able to comply with the visits and procedures specified in the protocol.

The exclusion criteria of this study were:
1. Histopathological type included small cell carcinoma components;
2. Subjects with known history of malignant tumors;
3. Subjects with known meningeal metastases, brainstem metastases, spinal cord metastases and/or compression, or active CNS metastases;
4. Subjects who experienced peripheral neuropathy with grade of ≥ 2 during prior platinum-based therapy;
5. History of esophageal and gastric varices, severe ulcers, gastrointestinal perforation, gastrointestinal obstruction, intra-abdominal abscess, or acute gastrointestinal bleeding within 6 months before the first dose of study treatment;
6. Arterial or venous thrombotic events within 6 months before the first dose of study treatment;
7. Active or previously confirmed inflammatory bowel disease;
8. Subjects with clinically significant cardiovascular diseases;
9. History of interstitial lung disease (ILD) or non-infectious pneumonitis requiring steroid treatment;
10. Uncontrolled systemic diseases as judged by the investigators, including uncontrolled hypertension, diabetes, and effusions requiring repeated drainage;
11. Active autoimmune diseases requiring systemic treatment in the past 2 years;
12. Diagnosis of active hepatitis B or C;
13. Subjects with known poorly controlled human immunodeficiency virus (HIV) infection;
14. Known active tuberculosis;
15. Received other investigational drugs within 4 weeks before the first dose of study treatment or experienced major surgery within 4 weeks;
16. Received radiation therapy to lung lesions with a total dose exceeding 30 Gy within 6 months before the first dose of study treatment;
17. Systemic use of strong inhibitors or inducers of cytochrome P450 3A4 enzyme (CYP3A4) was required within 2 weeks or 5 half-lives before the first dose of study treatment or during the study period;
18. Continuously received high-dose systemic corticosteroid therapy within 2 weeks before the first dose of study treatment;
19. Received live or attenuated vaccines within 4 weeks before the first dose of study treatment;
20. Experienced severe infections within 4 weeks before the first dose of study treatment;
21. Known history of allogeneic organ transplantation or allogeneic hematopoietic stem cell transplantation;
22. Subjects with known allergies or hypersensitivity to pembrolizumab or Conjugate A, or any excipients of pembrolizumab or conjugate A.

While preferred embodiments of the present disclosure have been shown and described herein, it will be obvious to those skilled in the art that such embodiments are provided by way of example only. Numerous variations, changes, and substitutions will now occur to those skilled in the art without departing from the present disclosure. It should be understood that various alternatives to the embodiments of the present disclosure described herein may be employed in practicing the methods and uses disclosed herein.

## Claims

1. A method of treating a cancer in a patient, comprising administering to the patient:
(a) an anti-human PD-1 antibody or antigen binding fragment thereof, a platinum-containing chemotherapeutic agent, and/or an EGFR-tyrosine kinase inhibitor; and
(b) an Immunoconjugate of Formula (I): 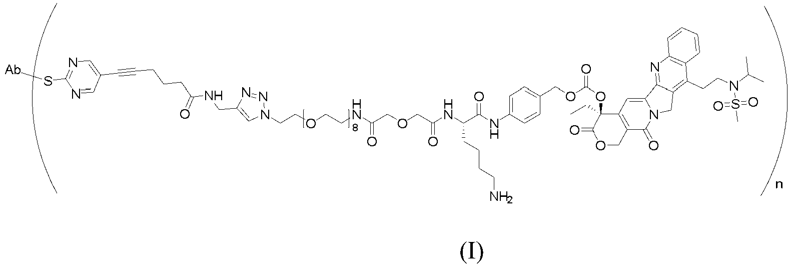 wherein:
Ab is an antibody that binds to Trop-2; and
n is an integer from 1 to 10,
wherein the amounts of (a) and (b) administered are together effective to treat cancer.

2. The method of claim 1, comprising administering to the patient:
(a) the anti-human PD-1 antibody or antigen-binding fragment thereof; and
(b) the Immunoconjugate of Formula (I).

3. The method of claim 1 or 2, wherein n is selected from 6-8.

4. The method of any one of claims 1 to 3, wherein the antibody capable of binding to Trop-2 comprises:
(i) a heavy chain variable region comprising an HCDR1 comprising the amino acid sequence of SEQ ID NO: 39, an HCDR2 comprising the amino acid sequence of SEQ ID NO: 40; and an HCDR3 comprising the amino acid sequence of SEQ ID NO: 41, and
(ii) a light chain variable region comprising an LCDR1 comprising the amino acid sequence of SEQ ID NO: 42; an LCDR2 comprising the amino acid sequence of SEQ ID NO: 43; and an LCDR3 comprising the amino acid sequence of SEQ ID NO: 44.

5. The method of any one of claims 1-4, wherein the antibody capable of binding to Trop-2 comprises:
(i) a heavy chain variable region comprising the amino acid sequence of SEQ ID NO: 37, and
(ii) a light chain variable region comprising the amino acid sequence of SEQ ID NO: 38.

6. The method of any one of claims 1-5, wherein the antibody capable of binding to Trop-2 comprises:
(i) a heavy chain comprising the amino acid sequence of SEQ ID NO: 35, and
(ii) a light chain comprising the amino acid sequence of SEQ ID NO: 36.

7. The method of any one of claims 1-6, wherein the antibody capable of binding to Trop-2 is sacituzumab.

8. The method of any one of claims 1-7, wherein the Immunoconjugate of Formula (I) is administered to the patient at a dose from 1.0 mg/kg to 6.0 mg/kg.

9. The method of any one of claims 1-8 wherein the anti-human PD-1 antibody or antigen binding fragment thereof is selected from nivolumab, cemiplimab, dostarlimab, pidilizumab, tislelizumab and pembrolizumab.

10. The method of any one of claims 1 to 9, wherein the anti-human PD-1 antibody or antigen binding fragment thereof is pembrolizumab.

11. The method of any one of claims 1-10, wherein the anti-human PD-1 antibody or antigen binding fragment thereof is administered to the patient at a dose of 50 mg to 500 mg.

12. The method of any one of claims 1 to 11, wherein the Immunoconjugate of Formula (I) and the anti-human PD-1 antibody or antigen-binding fragment thereof are each administered in a cycle of every week, every 1.5 weeks, every 2 weeks, every 3 weeks, every 4 weeks, every 5 weeks, every 6 weeks, every month, every two months, or every quarter.

13. The method of any one of claims 1-12, wherein the Immunoconjugate of Formula (I) and the anti-human PD-1 antibody or antigen binding fragment thereof are each administered in three-week cycles, and each are administered on Day 1 of each three-week cycle.

14. The method of any one of claims 1-12, wherein the Immunoconjugate of Formula (I) and the anti-human PD-1 antibody or antigen binding fragment thereof are each administered in six-week cycles, whrerein the anti-human PD-1 antibody or antigen binding fragment thereof is administered on Day 1 of each six-week cycle, and the Immunoconjugate of Formula (I) is administered on Days 1, 15, and 29 of each six-week cycle.

15. The method of claim 13, wherein the anti-human PD-1 antibody or antigen binding fragment thereof is administered at a dose of about 200 mg, and the Immunoconjugate of Formula (I) is administered at a dose of about 5 mg/kg.

16. The method of claim 14, wherein the anti-human PD-1 antibody or antigen-binding fragment thereof is administered at a dose of about 400 mg, and the Immunoconjugate of Formula (I) is administered at a dose of about 4 mg/kg.

17. The method of claim 14, wherein the the anti-human PD-1 antibody or antigen binding fragment thereof is administered at a dose of about 400 mg, and the Immunoconjugate of Formula (I) is administered at a dose of about 5 mg/kg.

18. The method of any one of claims 11-17, wherein the number of cycles is from 1-4.

19. The method of any one of claims 1 to 18, wherein the platinum-containing chemotherapeutic agent is carboplatin or cisplatin; preferably carboplatin.

20. The method of any one of claims 1 to 19, wherein the platinum-containing chemotherapeutic agent is administered at a dose of about AUC 1 mg/mL/min to AUC 10 mg/mL/min, or 10 mg/m² to 150 mg/m².

21. The method of any one of claims 1 to 20, wherein the Immunoconjugate of Formula (I) and the platinum-containing chemotherapeutic agent are each administered in a cycle of every week, every 1.5 weeks, every 2 weeks, every 3 weeks, every 4 weeks, every 5 weeks, every 6 weeks, every month, every two months or every quarter.

22. The method of any one of claims 1 to 21, wherein the Immunoconjugate of Formula (I) and the platinum-containing chemotherapeutic agent are each administered in a 3-week cycle, and are each administered on Days 1 and/or 8 of each 3-week cycle;
preferably, the Immunoconjugate of Formula (I) and the platinum-containing chemotherapeutic agent are each administered in a 3-week cycle, and are each administered on Day 1 of each 3-week cycle;
preferably, the Immunoconjugate of Formula (I) and the platinum-containing chemotherapeutic agent are each administered in a 3-week cycle, and the Immunoconjugate of Formula (I) is administered on Days 1 and 8 of each 3-week cycle, and the platinum-containing chemotherapeutic agent is administered on Day 1 of each 3-week cycle.

23. The method of any one of claims 1 to 21, wherein the Immunoconjugate of Formula (I) and the platinum-containing chemotherapeutic agent are each administered in a 6-week cycle, wherein the Immunoconjugate of Formula (I) is administered on Days 1, 8, 22 and/or 29 of each 6-week cycle, and the platinum-containing chemotherapeutic agent is administered on Days 1 and 22 of each 6-week cycle;
wherein the Immunoconjugate of Formula (I) and the platinum-containing chemotherapeutic agent are each administered in a 6-week cycle, wherein the Immunoconjugate of Formula (I) is administered on Days 1, 8, 22 and 29 of each 6-week cycle, and the platinum-containing chemotherapeutic agent is administered on Days 1 and 22 of each 6-week cycle;
wherein the Immunoconjugate of Formula (I) and the platinum-containing chemotherapeutic agent are each administered in a 6-week cycle, wherein the Immunoconjugate of Formula (I) is administered on Days 1 and 22 of each 6-week cycle, and the platinum-containing chemotherapeutic agent is administered on Days 1 and 22 of each 6-week cycle.

24. The method of any one of claims 1 to 21, wherein the Immunoconjugate of Formula (I) and the platinum-containing chemotherapeutic agent are administered in 1, 2, 3 or 4 phases.

25. The method of claim 24, wherein the Immunoconjugate of Formula (I) and the platinum-containing chemotherapeutic agent are administered in 2 phases, wherein,
in the first phase, the Immunoconjugate of Formula (I) is administered in a 3-week cycle and on Day 1 of each 3-week cycle, and the platinum-containing chemotherapeutic agent is administered in a 3-week cycle and on Day 1 of each 3-week cycle;
in the second phase, the Immunoconjugate of Formula (I) is administered in a 2-week cycle and on Day 1 of each 2-week cycle.

26. The method of claim 24, wherein the Immunoconjugate of Formula (I) and the platinum-containing chemotherapeutic agent are administered in two phases, wherein:
in the first phase, the Immunoconjugate of Formula (I) is administered in a 3-week cycle and on Days 1 and 8 of each 3-week cycle, and the platinum-containing chemotherapeutic agent is administered in a 3-week cycle and on Day 1 of each 3-week cycle;
in the second phase, the Immunoconjugate of Formula (I) is administered in a 2-week cycle and on Day 1 of each 2-week cycle.

27. The method of any one of claims 24 to 26, wherein each phase lasts for 2 to 24 weeks, 4 to 24 weeks, 6 to 24 weeks, 8 to 24 weeks, 10 to 24 weeks, 12 to 24 weeks, 14 to 24 weeks, 16 to 24 weeks, 18 to 24 weeks, 20 to 24 weeks or 22 to 24 weeks.

28. The method of claim 22 or 23, wherein the Immunoconjugate of Formula (I) is administered at a dose of about 4 mg/kg, and carboplatin is administered at a dose of AUC 5 mg/mL/min, or,
the Immunoconjugate of Formula (I) is administered at a dose of about 5 mg/kg, and carboplatin is administered at a dose of AUC 5 mg/mL/min.

29. The method of claim 22 or 23, wherein the Immunoconjugate of Formula (I) is administered at a dose of about 4 mg/kg, and cisplatin is administered at a dose of 75 mg/m², or,
the Immunoconjugate of Formula (I) is administered at a dose of about 5 mg/kg, and cisplatin is administered at a dose of 75 mg/m².

30. The method of claim 25, wherein,
in the first phase, the Immunoconjugate of Formula (I) is administered at a dose of about 3 mg/kg, and carboplatin is administered at a dose of AUC 5 mg/mL/min;
in the second phase, the Immunoconjugate of Formula (I) is administered at a dose of about 4 mg/kg.

31. The method of claim 26, wherein,
in the first phase, the Immunoconjugate of Formula (I) is administered at a dose of about 2.25 mg/kg, and carboplatin is administered at a dose of AUC 5 mg/mL/min;
in the second phase, the Immunoconjugate of Formula (I) is administered at a dose of about 4 mg/kg.

32. The method of any one of claims 21 to 31, wherein the number of cycles is 1 to 4.

33. The method of any one of claims 1 to 20, wherein the Immunoconjugate of Formula (I), the anti-human PD-1 antibody or antigen-binding fragment thereof, and the platinum-containing chemotherapeutic agent are each administered in a cycle of every week, every 1.5 weeks, every 2 weeks, every 3 weeks, every 4 weeks, every 5 weeks, every 6 weeks, every month, every two months, or every quarter.

34. The method of claim 33, wherein the Immunoconjugate of Formula (I), the anti-human PD-1 antibody or antigen-binding fragment thereof, and the platinum-containing chemotherapeutic agent are each administered in a 3-week cycle and on Day 1 of each 3-week cycle.

35. The method of any one of claims 1 to 21, 33, wherein the Immunoconjugate of Formula (I), the anti-human PD-1 antibody or antigen-binding fragment thereof and the platinum-containing chemotherapeutic agent are each administered in a 6-week cycle, wherein the Immunoconjugate of Formula (I) is administered on Days 1, 8, 22 and/or 29 of each 6-week cycle, the anti-human PD-1 antibody or antigen-binding fragment thereof is administered on Day 1 of each 6-week cycle, and the platinum-containing chemotherapeutic agent is administered on Days 1 and 22 of each 6-week cycle;
preferably, the Immunoconjugate of Formula (I) is administered on Days 1, 8, 22 and 29 of each 6-week cycle, the anti-human PD-1 antibody or antigen-binding fragment thereof is administered on Day 1 of each 6-week cycle, and the platinum-containing chemotherapeutic agent is administered on Days 1 and 22 of each 6-week cycle;
preferably, the Immunoconjugate of Formula (I) is administered on Days 1 and 22 of each 6-week cycle, the anti-human PD-1 antibody or antigen-binding fragment thereof is administered on Day 1 of each 6-week cycle, and the platinum-containing chemotherapeutic agent is administered on Days 1 and 22 of each 6-week cycle.

36. The method of any one of claims 1 to 21, 33 to 35, wherein the Immunoconjugate of Formula (I), the anti-human PD-1 antibody or antigen-binding fragment thereof and the platinum-containing chemotherapeutic agent are administered in 1, 2, 3 or 4 phases.

37. The method of claim 36, wherein the Immunoconjugate of Formula (I), the anti-human PD-1 antibody or antigen-binding fragment thereof and the platinum-containing chemotherapeutic agent are administered in 2 phases, wherein:
in the first phase, the Immunoconjugate of Formula (I) is administered in a 3-week cycle and on Day 1 of each 3-week cycle, the anti-human PD-1 antibody or antigen-binding fragment thereof is administered in a 6-week cycle and on Day 1 of each 6-week cycle, and the platinum-containing chemotherapeutic agent is administered in a 3-week cycle and on Day 1 of each 3-week cycle;
in the second phase, the Immunoconjugate of Formula (I) is administered in a 2-week cycle and on Day 1 of each 2-week cycle, and the anti-human PD-1 antibody or antigen-binding fragment thereof is administered in a 6-week cycle and Day 1 of each 6-week cycle.

38. The method of claim 36, wherein the Immunoconjugate of Formula (I), the anti-human PD-1 antibody or antigen-binding fragment thereof and the platinum-containing chemotherapeutic agent are administered in 2 phases, wherein:
in the first phase, the Immunoconjugate of Formula (I) is administered in a 3-week cycle and on Days 1 and 8 of each 3-week cycle, the anti-human PD-1 antibody or antigen-binding fragment thereof is administered in a 6-week cycle and on Day 1 of each 6-week cycle, and the platinum-containing chemotherapeutic agent is administered in a 3-week cycle and on Day 1 of each 3-week cycle;
in the second phase, the Immunoconjugate of Formula (I) is administered in a 2-week cycle and on Day 1 of each 2-week cycle, and the anti-human PD-1 antibody or antigen-binding fragment thereof is administered in a 6-week cycle and on Day 1 of each 6-week cycle.

39. The method of any one of claims 36 to 38, wherein each phase lasts for 2 to 24 weeks, 4 to 24 weeks, 6 to 24 weeks, 8 to 24 weeks, 10 to 24 weeks, 12 to 24 weeks, 14 to 24 weeks, 16 to 24 weeks, 18 to 24 weeks, 20 to 24 weeks, or 22 to 24 weeks.

40. The method of claim 34, wherein the Immunoconjugate of Formula (I) is administered at a dose of about 4 mg/kg, the anti-human PD-1 antibody or antigen-binding fragment thereof is administered at a dose of about 200 mg, and carboplatin is administered at a dose of AUC 5 mg/mL/min, or,
the Immunoconjugate of Formula (I) is administered at a dose of about 5 mg/kg, the anti-human PD-1 antibody or antigen-binding fragment thereof is administered at a dose of about 200 mg, and carboplatin is administered at a dose of AUC 5 mg/mL/min.

41. The method of claim 35, wherein the Immunoconjugate of Formula (I) is administered at a dose of about 4 mg/kg, the anti-human PD-1 antibody or antigen-binding fragment thereof is administered at a dose of about 400 mg, and carboplatin is administered at a dose of AUC 5 mg/mL/min, or,
the Immunoconjugate of Formula (I) is administered at a dose of about 5 mg/kg, the anti-human PD-1 antibody or antigen-binding fragment thereof is administered at a dose of about 400 mg, and carboplatin is administered at a dose of AUC 5 mg/mL/min.

42. The method of claim 37, wherein,
in the first phase, the Immunoconjugate of Formula (I) is administered at a dose of about 3 mg/kg, the anti-human PD-1 antibody or antigen-binding fragment thereof is administered at a dose of about 400 mg, and carboplatin is administered at a dose of AUC 5 mg/mL/min;
in the second phase, the Immunoconjugate of Formula (I) is administered at a dose of about 4 mg/kg, and the anti-human PD-1 antibody or antigen-binding fragment thereof is administered at a dose of about 400 mg.

43. The method of claim 38, wherein,
in the first phase, the Immunoconjugate of Formula (I) is administered at a dose of about 2.25 mg/kg, the anti-human PD-1 antibody or antigen-binding fragment thereof is administered at a dose of about 400 mg, and carboplatin is administered at a dose of AUC 5 mg/mL/min;
in the second phase, the Immunoconjugate of Formula (I) is administered at a dose of about 4 mg/kg, and the anti-human PD-1 antibody or antigen-binding fragment thereof is administered at a dose of about 400 mg.

44. The method of any one of claims 33 to 43, wherein the number of cycles is 1 to 4.

45. The method of claim 34, wherein:
(i) the Immunoconjugate of Formula (I) is administered at a dose of about 5 mg/kg on day 1 of a 3-week cycle;
(ii) the anti-human PD-1 antibody or antigen binding fragment thereof is administered at a dose of about 200 mg on day 1 of a 3-week cycle;
(iii) carboplatin is administered at a dose of about AUC 5 mg/ml/min on day 1 of a 3-week cycle; and
(iv) the number of cycles is from 1 to 4.

46. The method of claim 34, wherein:
(i) the Immunoconjugate of Formula (I) is administered at a dose of about 5 mg/kg on day 1 of a 3-week cycle;
(ii) the anti-human PD-1 antibody or antigen binding fragment thereof is administered at a dose of about 200 mg on day 1 of a 3-week cycle;
(iii) cisplatin is administered at a dose of about 75 mg/m² on day 1 of a 3-week cycle; and
(iv) the number of cycles is from 1 to 4.

47. The method of claim 35, wherein:
(i) the Immunoconjugate of Formula (I) is administered at a dose of about 5 mg/kg on days 1, 15, and 29 of a 6-week cycle;
(ii) the anti-human PD-1 antibody or antigen binding fragment thereof is administered at a dose of about 400 mg on day 1 of a 6-week cycle;
(iii) carboplatin is administered at a dose of about AUC 5 mg/ml/min on days 1 and 22 of a 6-week cycle; and
(iv) the number of cycles is from 1 to 4.

48. The method of claim 35, wherein:
(i) the Immunoconjugate of Formula (I) is administered at a dose of about 5 mg/kg on days 1, 15, and 29 of a 6-week cycle;
(ii) the anti-human PD-1 antibody or antigen binding fragment thereof is administered at a dose of about 400 mg on day 1 of a 6-week cycle;
(iii) cisplatin is administered at a dose of about 75 mg/m² on days 1 and 22 of a 6-week cycle; and
(iv) the number of cycles is from 1 to 4.

49. The method of any one of claims 1 to 48, comprising administering to the patient:
(a) the EGFR-tyrosine kinase inhibitor; and
(b) the Immunoconjugate of Formula (I);
wherein the EGFR-tyrosine kinase inhibitor is osimertinib.

50. The method of claim 49, wherein the Immunoconjugate of Formula (I) is administered in a cycle of every week, every 1.5 weeks, every 2 weeks, every 3 weeks, every 4 weeks, every 5 weeks, every 6 weeks, every month, every two months or every quarter, and the EGFR-tyrosine kinase inhibitor is administered in a cycle of every day, every 2 days, every 3 days, every 4 days, every 5 days, every 6 days or every week.

51. The method of claim 49 or 50, wherein the Immunoconjugate of Formula (I) is administered in a 2-week cycle, and each is administered on Day 1 of each 2-week cycle, and the EGFR-tyrosine kinase inhibitor is administered daily.

52. The method of claim 51, wherein:
(i) the Immunoconjugate of Formula (I) is administered at a dose of about 3 mg/kg on Day 1 of each 2-week cycle;
(ii) osimertinib is administered daily at a dose of about 80 mg;
(iv) the number of cycles is 1 to 4.

53. The method of claim 51, wherein:
(i) the Immunoconjugate of Formula (I) is administered at a dose of about 4 mg/kg on Day 1 of each 2-week cycle;
(ii) osimertinib is administered at a dose of about 80 mg daily;
(iv) the number of cycles is 1 to 4.

54. The method of claim 51, wherein:
(i) the Immunoconjugate of Formula (I) is administered at a dose of about 5 mg/kg on Day 1 of each 2-week cycle;
(ii) osimertinib is administered at a dose of about 80 mg daily;
(iv) the number of cycles is 1 to 4.

55. The method of any one of claims 1 to 54, wherein the disease being treated is non-small cell lung cancer.

56. The method of claim 55, wherein:
(a) the non-small cell lung cancer is without EGFR mutation, and ALK fusion genes; or
(b) the non-small cell lung cancer has an EGFR actionable mutation, and the patient has failed prior EGFR-TKI treatment.

57. The method of any one of claims 1 to 18, wherein: the Immunoconjugate of Formula (I) is administered at a frequency of once every 2 weeks, once every 3 weeks, twice every 3 weeks, once every 4 weeks, once every 5 weeks, or once every 6 weeks and a dose of 1 to 10 mg/kg, and the anti-human PD-1 antibody (or antigen-binding fragment thereof) is administered at a frequency of once every 1 to 10 weeks (preferably 2 to 8 weeks) and a dose of 100 to 800 mg;
preferably, the disease is EGFR wild-type and ALK fusion gene-negative non-small cell lung cancer.

58. The method of any one of claims 33 to 48, wherein: the Immunoconjugate of Formula (I) is administered at a frequency of once every 2 weeks, once every 3 weeks, twice every 3 weeks, once every 4 weeks, once every 5 weeks, or once every 6 weeks and a dose of 1-10 mg/kg, the anti-human PD-1 antibody (or antigen-binding fragment thereof) is administered at a frequency of once every 1 to 10 weeks (preferably 2 to 8 weeks) and a dose of 100 to 800 mg, and the platinum-containing chemotherapeutic agent is administered at a frequency of once every 1 to 5 weeks and a dose of AUC 1 to 10 mg/ml/min; after 1 to 5 cycles, the Immunoconjugate of Formula (I) is administered at a frequency of once every 1 to 5 weeks and a dose of 1 to 10 mg/kg, the anti-human PD-1 antibody (or antigen-binding fragment thereof) is administered at a frequency of once every 1 to 10 weeks (preferably 2 to 8 weeks) and a dose of 100 to 800 mg; wherein one cycle consists of 6 weeks;
preferably, the disease is EGFR wild-type and ALK fusion gene-negative non-small cell lung cancer.

59. The method of any one of claims 19 to 32, wherein: the Immunoconjugate of Formula (I) is administered at a frequency of once every 2 weeks, once every 3 weeks, twice every 3 weeks, once every 4 weeks, once every 5 weeks, or once every 6 weeks and a dose of 1 to 10 mg/kg, and the platinum-containing chemotherapy agent is administered at a frequency of once every 1 to 5 weeks and a dose of AUC 1 to 10 mg/ml/min; after 1 to 5 cycles, the Immunoconjugate of Formula (I) is administered at a frequency of once every 1 to 5 weeks and a dose of 1 to 10 mg/kg; wherein once cycle consists of 6 weeks;
preferably, the disease is non-small cell lung cancer with EGFR sensitive mutation and previous EGFR-TKI treatment failure.

60. The method of any one of claims 49 to 54, wherein: the Immunoconjugate of Formula (I) is administered at a frequency of once every 2 weeks, once every 3 weeks, twice every 3 weeks, once every 4 weeks, once every 5 weeks, or once every 6 weeks and a dose of 1 to 10 mg/kg, and the EGFR-tyrosine kinase inhibitor is administered 3 times every day, twice every day, once every day, once every 2 days, or once every 3 days at a dosage of 30 to 150 mg;
preferably, the disease is EGFR-mutated non-small cell lung cancer.

61. A pharmaceutical composition comprising:
(a) an anti-human PD-1 antibody or antigen binding fragment thereof, a platinum-containing chemotherapeutic agent, and/or an EGFR-tyrosine kinase inhibitor;
(b) a pharmaceutically acceptable carrier; and
(c) a plurality of immunoconjugates of Formula (I): 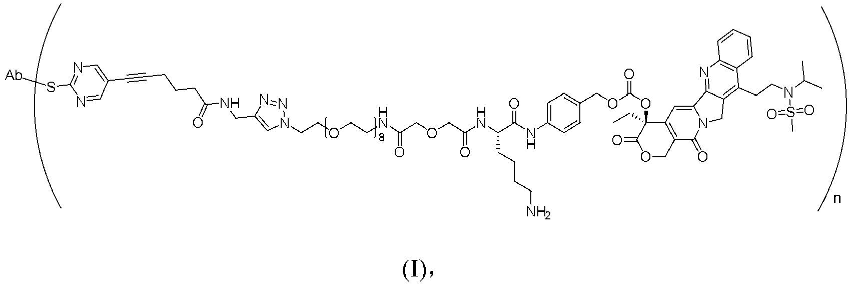 wherein:
Ab is an antibody that binds to Trop-2; and
*n̅* is an integer or decimal from 0 to 10, and represents the average number of linker/payload moieties joined to each antibody for the plurality of immunoconjugates of Formula (I);
wherein the amounts of (a) and (c) present in the composition are together effective to treat cancer.

62. The pharmaceutical composition of claim 61, wherein:
(1) the anti-human PD-1 antibody or antigen-binding fragment thereof is pembrolizumab;
(2) the platinum-containing chemotherapeutic agent is cisplatin or carboplatin;
(3) the EGFR-tyrosine kinase inhibitor is osimertinib; and/or
(4) the Immunoconjugate of Formula (I) is Immunoconjugate A.

63. A pharmaceutical composition, comprising:
(a) an anti-human PD-1 antibody or antigen-binding fragment thereof;
(b) a pharmaceutically acceptable carrier; and
(c) a plurality of immunoconjugates represented by Formula (I): 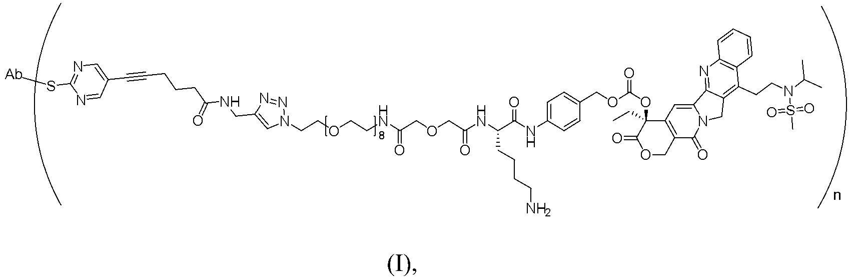 wherein:
Ab represents an antibody capable of binding to Trop-2; and
*n̅* is a decimal from 0 to 10, and represents the average of the number n of linker/payload moieties joint to each antibody used in the plurality of immunoconjugates represented by Formula (I);
wherein the amounts of (a) and (c) present in the pharmaceutical composition are together effective to treat a cell proliferative disorder such as cancer.

64. The pharmaceutical composition of claim 63, wherein the antibody capable of binding to TROP-2 is sacituzumab.

65. The pharmaceutical composition of claim 63 or 64, wherein the anti-human PD-1 antibody or antigen binding fragment thereof is pembrolizumab.

66. A kit comprising:
(a) an amount of an anti-human PD-1 antibody or antigen binding fragment thereof, and/or a platinum-containing chemotherapeutic agent, and/or an EGFR-tyrosine kinase inhibitor; and
(b) an amount of an Immunoconjugate of Formula (I): 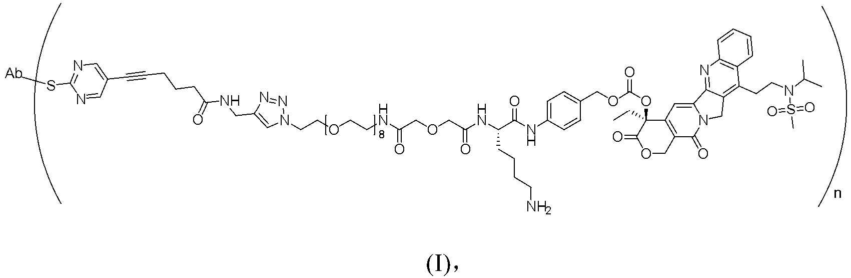 wherein:
Ab is an antibody that binds to Trop-2; and
n is an integer from 1 to 10;
wherein the amounts of (a) and (b) present in the kit are together effective to treat cancer.

67. A kit, comprising:
(a) an amount of an anti-human PD-1 antibody or antigen-binding fragment thereof; and
(b) an amount of an immunoconjugate represented by Formula (I): 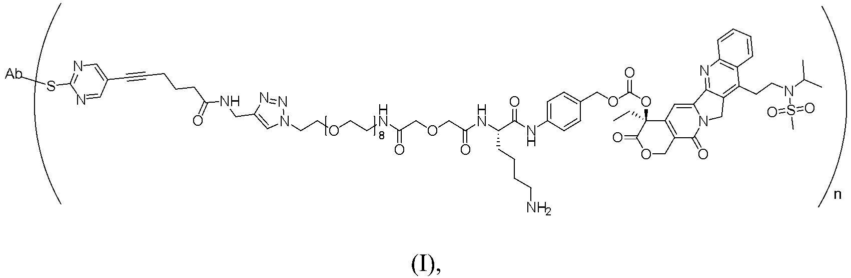 wherein:
Ab represents an antibody capable of binding to Trop-2; and
n is an integer from 0 to 10;
wherein the amounts of (a) and (c) in the kit together are effective for treating a cell proliferative disorder such as cancer.

68. The kit of claim 66 or 67, further comprising instructions for administering the Immunoconjugate of Formula (I), and the anti-human PD-1 antibody or antigen binding fragment thereof to a human patient.

69. The kit of any one of claims 66 to 68, wherein the anti-human PD-1 antibody or antigen binding fragment thereof is pembrolizumab.

70. The kit of any one of claims 66 to 69, wherein the Immunoconjugate of Formula (I) is Immunoconjugate A.

71. The kit of claim 66, wherein,
the EGFR-tyrosine kinase inhibitor is osimertinib; and/or the Immunoconjugate of Formula (I) is Immunoconjugate A.

72. A pharmaceutical combination, for use in treating a cell proliferative disorder such as cancer in a human patient, the pharmaceutical combination comprises:
(a) an Immunoconjugate of Formula (I); and
(b) an anti-human PD-1 antibody or antigen-binding fragment thereof, a platinum-containing chemotherapeutic agent, and/or an EGFR-tyrosine kinase inhibitor;
wherein the Immunoconjugate of Formula (I), the anti-human PD-1 antibody or antigen-binding fragment thereof, the platinum-containing chemotherapeutic agent, and/or the EGFR-tyrosine kinase inhibitor are as described in any one of claims 1 to 71.

73. Use of a pharmaceutical combination in the manufacture of a medicament for treating a cell proliferative disorder such as cancer in a human patient, the pharmaceutical combination comprises:
(a) an Immunoconjugate of Formula (I); and
(b) an anti-human PD-1 antibody or antigen-binding fragment thereof, a platinum-containing chemotherapeutic agent, and/or an EGFR-tyrosine kinase inhibitor;
wherein the Immunoconjugate of Formula (I), and the anti-human PD-1 antibody or antigen-binding fragment thereof are as described in any one of claims 1 to 72.
